(19)

Europäisches
Patentamt
European
Patent Office
Office européen
des brevets

(11) **EP 2 937 413 A1**

(12) **EUROPEAN PATENT APPLICATION**
published in accordance with Art. 153(4) EPC

(43) Date of publication:
**28.10.2015 Bulletin 2015/44**

(21) Application number: **13865967.7**

(22) Date of filing: **19.12.2013**

(51) Int Cl.:
***C12M 1/34*** *(2006.01)*

(86) International application number:
**PCT/JP2013/084084**

(87) International publication number:
**WO 2014/098182 (26.06.2014 Gazette 2014/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **19.12.2012 JP 2012276957**

(71) Applicant: **National University Corporation
Tokyo Medical and Dental University
Bunkyo-ku
Tokyo 113-8510 (JP)**

(72) Inventors:
• **YASUDA Kenji**
**Tokyo 113-8510 (JP)**
• **KANEKO Tomoyuki**
**Tokyo 113-8510 (JP)**
• **NOMURA Fumimasa**
**Tokyo 113-8510 (JP)**

(74) Representative: **EIP
Fairfax House
15 Fulwood Place
London WC1V 6HU (GB)**

(54) **METHOD AND DEVICE FOR EXAMINING MYOCARDIAL TOXICITY AND EVALUATING
CARDIOMYOCYTES**

(57)    Regarding cardiomyocytes and fibroblasts, it is meaningful to develop a device or system whereby, upon the transmission of pulsation from an adjacent cardiomyocyte or fibroblast, cell potential and cell morphology can be accurately measured on a single cell basis and the toxicity of a drug on cardiomyocytes can be examined on the basis of accurately measured cell potential and cell morphology of a single cell. In the present invention, a mass of cardiomyocytes is disposed on a transparent substrate and the qualities of the cardiomyocytes are evaluated depending on the response of the cardiomyocytes to a forced pulsation stimulus that is applied to the pulsating cardiomyocytes. A mass of cardiomyocytes, said mass being disposed on the transparent substrate, is exposed to a flow of a drug-containing liquid so as to allow the drug to act on cells configuring a network. The level of myocardial toxicity of the drug is evaluated by measuring the fluctuations that are obtained by comparing adjacent pulsating cardiomyocytes in the network.

**EP 2 937 413 A1**

**Description**

TECHNICAL FIELD

The present invention relates to a method and an apparatus for testing myocardial toxicity and evaluating myocardial cells.

BACKGROUND ART

Bio-assays have been widely used to observe changes in the state of cells, the responsiveness of the cells to agents, and the like. In general, cultured cells have been often used in conventional bioassays. In such systems, assays are performed using a plurality of cells, and an average of the values of a cell population has been measured as if it represented the characteristics of a single cell.

However, in fact, it is rare that there are cells whose cell cycle is synchronized in the cell population, and each cell synthesizes proteins in a different manner. Therefore, fluctuation is always the problem when analyzing the results of the response of the cells to a stimulus.

In other words, since the fluctuations of responses of the reaction mechanism of a cell itself are universally present, one can always only obtain an average of the responses. To solve this problem, there have been developed methodologies, such as synchronized culturing. However, to use a group of cells which are in the same stage, one must always continue to supply such cells, and therefore this feature has become an obstacle to broad-based application of the bioassay.

In addition, in reality it has been difficult to decide on the fluctuation because there are two types of stimulation (signals) to cells: one is given by the amount of a signal substance, nutrition, dissolved gas contained in the solution surrounding the cell, and the other is given by the physical contact and cell-to-cell interaction with other cells.

Difficulties in the physical contact and the cell-to-cell interaction problems of the cells can be resolved to some extent by performing bioassays on a cell mass such as tissue fragments. However, in such cases, unlike cultured cells, it is not always possible to obtain a cell mass with a homogeneous feature. Therefore, there is a problem that the resulting data can vary, and that the information is buried in the population.

To enable measurement using an information processing model in which each cell in the cell population is a minimum structural unit, the inventors of the present application have proposed a microarray for aggregated cells (bioassay chip) comprising a plurality of cell culture compartments for confining a cell inside a specific spatial arrangement; a groove or a tunnel linking between adjacent compartments, wherein a cell cannot pass through the groove or the tunnel; and a plurality of electrode patterns for measuring a change in electric potential of the cell arranged in the groove or the tunnel or the cell culture compartment as shown in JP 2006-94703 (Patent Document 1).

In addition, a method for electrocardiogram analysis has been proposed for the evaluation of the electrocardiogram obtained by reflecting complex cardiac functions by utilizing a method typically used for measuring non-linear dynamics. For example, a Poincare plotting method has been the most commonly used for the analysis of electrocardiogram (Non-Patent Document 1). A point in the plot refers to information of two adjacent pulsation data, in which, for example, a rate of pulsation at one time point is indicated on the X axis and a rate of pulsation at a previous time point is indicated on the Y axis. Thus, the fluctuation in the cardiac pulsation is estimated by quantifying the distribution of the points on the graph. Other methods for measuring the fluctuation of the cardiac pulsation include a correlation dimension method, a nonlinear predictability method (Non-Patent Document 2), an approximate entropy method (Non-Patent Document 3), and the like.

In addition, as for evaluation of cardiac toxicity, there are issues relating to an evaluation of side effects of a drug in terms of the contractile force of heart muscle cells, i.e., how a stroke volume of blood can be changed in response to administration of the drug. However, for this issue, *in vivo* measurements are currently a major approach, and a cell-based *in vitro* screening system has not been established so far.

[Background Art Documents]

[Patent Document]

[Patent Document 1] Japanese Laid-open Patent Publication No. 2006-94703

[Non-Patent Document]

[Non-Patent Document 1] Brennan M, Palaniswami M, Kamen P. Do existing measures of Poincare plot geometry reflect non-linear features of heart rate variability? Biomedical Engineering, IEEE Transactions on, Proc. IEEE Transactions on Biomedical Engineering, 2001, 48 , 1342-1347

[Non-Patent Document 2] Kanters JK, Holstein-Rathlou NH, Agner E (1994) "Lack of evidence for low-dimensional chaos in heart rate variability" Journal of Cardiovascular Electrophysiology 5 (7): 591-601.PMID 7987529.

[Non-Patent Document 3] Storella RJ, Wood HW, Mills KM et al (1994) "Approximate entropy and point correlation dimension of heart rate variability in healthy subjects" Integrative Physiological & Behavioral Science 33 (4): 315-20. PMID 10333974.

## SUMMARY OF THE INVENTION

### PROBLEM TO BE SOLVED BY THE INVENTION

In conventional bioassays, cells were treated as a tissue fragment or as a single cell as in cultured cells. As mentioned in the above background art section, when the number of cells is excessive, information collected is averaged, and there is a problem that responses to agents cannot be obtained accurately. When the cells are used as a single cell, the cell is used in a separated independent state instead of cells in natural multi-cellular tissues. Consequently, the effect of the interaction between cells is not exhibited. Therefore, there is still a problem in obtaining an accurate agent response, that is, a bioassay data.

There is a need for the development of an apparatus or a system that enables accurate measurement of the membrane potential or the cell morphology in a unit of a single cell as a measure of propagation of pulsation from mutually adjacent fibroblasts or cardiomyocytes, and the accurate measurement of the membrane potential or the cell morphology in a unit of a single cell as a measure of the toxicity of agents on cardiac muscle cells.

Use in regenerative medicine or agent screening requires that the functional aspects of cardiomyocytes which are differentiated from human stem cells including human iPS cells or human ES cells must be evaluated quantitatively to ascertain whether the qualitative features of the cardiomyocytes are the same as cardiomyocytes in the human heart cells.

### MEANS FOR SOLVING THE PROBLEM

In order to measure electrophysiological properties of cardiomyocytes and evaluate myocardial toxicity thereof, the present invention provides an apparatus as described below. In particular, the present invention provides an apparatus for evaluating myocardial toxicity of a drug as a measure of fluctuation of transmission rate and transmission time (time required for transmission) of excitation conduction of cardiomyocyte network.

[1] A myocardial toxicity evaluation apparatus, comprising:

a substrate;
a plurality of stably pulsating subject cardiomyocytes or a cell population comprising the subject cardiomyocytes arranged on the substrate;
a wall formed on the substrate to surround the periphery of the cardiomyocytes and the cell population and to fill a cell culture medium;
at least two measurement electrodes on each of which a single cell of the cell population or a local portion of the cell population is placed in a cardiomyocyte network consisting of the plurality of cardiomyocytes or the cell population comprising the plurality of cardiomyocytes;
a potential measuring means configured to allow measurements of cellular potential of the cardiomyocytes that are placed on the microelectrodes continuously over time using lead wires which are respectively connected to each of the measurement electrodes ;
a computing means configured to calculate transmission time or a transmission rate of excitation conduction transmitting between the at least two measurement electrodes using data measured by the potential measuring means, wherein the transmission time or the transmission rate of the excitation conduction is calculated through a comparison of time for an occurrence of depolarization due to an initial excitation conduction of the cellular potential between two adjacent measurement electrodes.

[2] The myocardial toxicity evaluation apparatus according to [1] above, which is characterized by the use of a sodium spike as a measurement index for the depolarization excited by the initial excitation conduction of the cellular potential.

[3] The myocardial toxicity evaluation apparatus according to [1] or [2] above, wherein the computing means performs a comparison computing of the transmission time or the transmission rate of the excitation conduction between adjacent two time points ($Delay_n$, $Delay_{n+1}$).

[4] The myocardial toxicity evaluation apparatus according to any one of [1] to [3] above, wherein the computing means calculates fluctuation of the transmission time or the transmission rate of the excitation conduction measured between the adjacent at least two electrodes over time, wherein the apparatus is used for an evaluation of myocardial toxicity of a drug using the fluctuation as a means of the evaluation.

[5] The myocardial toxicity evaluation apparatus according to any one of [1] to [4] above, wherein the number for continuous measurements of the cellular potentials of the cardiomyocytes over time is at least 30.

[6] The myocardial toxicity evaluation apparatus according to any one of [1] to [5] above, wherein the at least two measurement electrodes for the comparison measurements are arranged linearly, and the cardiomyocyte network is arranged in coordination with the arrangement of the electrodes.

[7] The myocardial toxicity evaluation apparatus according to [6] above, wherein an area surrounded by the wall on the substrate forms a culturing chamber which conforms in its shape to the arrangement of the at least two measurement electrodes for the comparison measurements.

[8] The myocardial toxicity evaluation apparatus according to any one of [1] to [7] above, wherein said apparatus further comprises a stimulation electrode for enforced pulsation of the cardiomyocytes, wherein the stimulation electrode is arranged within the area surrounded by the wall configured to fill the area with the cell culture medium.

[9] The myocardial toxicity evaluation apparatus according to [8] above, wherein the at least two measurement electrodes and the stimulation electrode are arranged linearly, the stimulation electrodes are arranged at end nodes, and the cardiomyocyte network is arranged in coordination with the arrangement of the electrodes.

[10] The myocardial toxicity evaluation apparatus according to any one of [1] to [9] above, wherein the cell population further comprises non-cardiomyocyte such as fibroblasts.

[11] The myocardial toxicity evaluation apparatus according to [10] above, wherein the cardiomyocyte population forms a network of cardiomyocytes and fibroblasts, wherein the rate at which the fibroblasts are mixed with the cardiomyocytes is in conformity to that of a heart.

[12] The myocardial toxicity evaluation apparatus according to [11] above, wherein the fibroblasts make up $40 \pm 10\%$ to $60 \pm 10\%$ of the cells in the cell population which forms a human heart model.

[13] The myocardial toxicity evaluation apparatus according to any one of [1] to [12] above, wherein the wall formed on the substrate to surround the periphery of the cardiomyocytes or the cell population is formed from an agarose gel.

[14] The myocardial toxicity evaluation apparatus according to any one of [1] to [13] above, wherein a plurality of cell housings and channels connecting the plurality of cell housings in a fluid communicable manner are arranged in the area surrounded by the wall formed on the substrate to surround the periphery of the cardiomyocytes and the cell population, wherein each of the cell housings is sized to receive a single cell and each of the measurement electrodes is arranged in each of the cell housings.

[15] The myocardial toxicity evaluation apparatus according to any one of [1] to [14] above, wherein the plurality of the cardiomyocytes consists of at least four or at least eight cardiomyocytes.

The present invention also provides a myocardial toxicity evaluation apparatus, a cardiomyocyte network chip, a method for preparation of a cardiomyocyte network chip, a method for producing a cell culturing chip and a cell culturing chip as follows:

(1) A myocardial toxicity evaluation apparatus, comprising:

a substrate;
a plurality of stably pulsating subject cardiomyocytes or a cell population comprising the subject cardiomyocytes arranged on the substrate;
a cell population holding area which is formed on the substrate and holds the cell population and a cell culture medium;
at least two measurement electrodes on which a single cell or a local portion of the cell population is placed in a cardiomyocyte network consisting of the plurality of cardiomyocytes or the cell population comprising the cardiomyocytes;
a potential measuring means configured to measure cellular potential of the cardiomyocytes that are placed on the measurement electrodes continuously over time using lead wires which are respectively connected to each of the measurement electrodes;
a computing means configured to calculate a transmission time or a transmission rate of excitation conduction transmitting between the at least two measurement electrodes using data measured by the potential measuring means, wherein the transmission time or the transmission rate of the excitation conduction is calculated through a comparison of time for an occurrence of depolarization due to an initial excitation conduction of cellular potential between two adjacent measurement electrodes.

(2) The myocardial toxicity evaluation apparatus according to (1) above, which is characterized by the use of a sodium spike as a measurement index for the depolarization excited by the initial excitation conduction of the cellular potential.

(3) The myocardial toxicity evaluation apparatus according to (1) or (2) above, wherein the computing means performs a comparison computing of the transmission time or the transmission rate of the excitation conduction between adjacent two time points ($Delay_n$, $Delay_{n+1}$).

(4) The myocardial toxicity evaluation apparatus according to any one of (1) to (3) above, wherein the computing means calculates fluctuation of the transmission time or the transmission rate of the excitation conduction measured between the adjacent at least two electrodes, wherein the apparatus is used for an evaluation of myocardial toxicity of a drug using the fluctuation as a means of the evaluation.

(5) The myocardial toxicity evaluation apparatus according to any one of (1) to (4) above, wherein the number for continuous measurements of the cellular potential of the cardiomyocytes over time is at least 30.

(6) The myocardial toxicity evaluation apparatus according to any one of (1) to (5) above, wherein said apparatus further comprises a stimulation electrode for enforced pulsation of the cardiomyocytes, wherein the stimulation electrode is arranged within the cell population holding area.

(7) The myocardial toxicity evaluation apparatus according to any one of (1) to (6) above, wherein the at least two measurement electrodes for comparative measurements are arranged linearly, and the cardiomyocyte network is arranged in coordination with the arrangement of the electrodes.

(8) The myocardial toxicity evaluation apparatus according to any one of (1) to (7) above, wherein said apparatus comprises a cell holding member that holds the cardiomyocyte or the cardiomyocyte population within the cell population holding area, wherein the cell holding member forms a culturing chamber whose shape is in coordination with the arrangement of the at least two measurement electrodes for the comparison measurements.

(9) The myocardial toxicity evaluation apparatus according to (8) above, wherein the at least two measurement electrodes and the stimulation electrode are arranged linearly and the stimulation electrode is arranged at end nodes, and wherein the cardiomyocyte network is arranged in coordination with the arrangement of the electrodes.

(10) The myocardial toxicity evaluation apparatus according to (1) to (9) above, wherein the cell population further comprises non-cardiomyocytes (e.g. fibroblasts).

(11) The myocardial toxicity evaluation apparatus according to (10) above, wherein the cell population forms a cardiomyocyte network comprising fibroblasts at a proportion corresponding to that of a human heart.

(12) The myocardial toxicity evaluation apparatus according to (11) above, wherein the fibroblasts make up 40 $\pm$ 10% to 60 $\pm$ 10% of the cells in the cell population.

(13) The myocardial toxicity evaluation apparatus according to any one of (1) to (12) above, wherein said apparatus comprises a plurality of cell housing members sized to hold a single cell, and channels connecting the plurality of the cell housing members in a fluid communicable fashion, wherein the measurement electrodes are respectively arranged in each of the cell housing members.

(14) The myocardial toxicity evaluation apparatus according to any one of (1) to (13) above, wherein the plurality of cardiomyocytes comprise at least four or eight cardiomyocytes.

(15) The myocardial toxicity evaluation apparatus according to any one of (1) to (14) above, wherein:

(i) the apparatus comprises two areas in which an agarose gel is arranged and an agarose gel is not arranged, respectively, within the cell population holding area on the substrate, wherein the cardiomyocytes or the cardiomyocyte population are arranged in the area in which the agarose gel is not arranged; or
(ii) the apparatus comprises two areas in which a water-repellent solid is arranged and a water-repellent solid is not arranged, respectively, within the cell population holding area on the substrate, wherein the cardiomyocytes or the cardiomyocyte population are arranged in the area in which the water-repellent solid is not arranged.

(16) The myocardial toxicity evaluation apparatus according to (15) above, wherein the water-repellent solid is a solid comprising a Teflon™ microparticle.

(17) The myocardial toxicity evaluation apparatus according to (15) above, wherein (i) the area in which the agarose gel is not arranged or (ii) the area in which the water-repellent solid is not arranged is arranged linearly, in parallel, or in a lattice pattern.

(18) The myocardial toxicity evaluation apparatus according to any one of (15) to (17) above, wherein a cell adhesive material such as collagen is applied on a surface of the substrate in (i) the area in which the agarose gel is not arranged or (ii) the area in which the water-repellent solid is not arranged.

(19) The myocardial toxicity evaluation apparatus according to any one of (1) to (18) above, wherein the apparatus comprising:

at least two potential measurement means configured to independently and continuously measure the excitation conduction which transmits through the cardiomyocyte network, a measurement data storage means configured to store measured results of the excitation conduction transmitting through the cardiomyocyte network, wherein the measured results are measured by the potential measurement means, at least two analysis means configured to analyze the measured results, an analysis data storage means configured to store analysis results analyzed by the analysis means and associate the analysis results with the measurement results, and a determining means configured to determine toxicity of the drug based on the analysis of the measurement results.

(20) The myocardial toxicity evaluation apparatus according to (19) above, wherein the determining means is configured to associate the results from the determination with the measurement results stored in the measurement data storage means.

(21) The myocardial toxicity evaluation apparatus according to (19) or (20) above, wherein the determining means is further configured to direct instructions to repeat the measurement to the measurement means via a feedback system based on the results of the determination.

(22) A cardiomyocyte network chip for use in the cardiomyocyte evaluation apparatus according to (11) or (12) above, wherein the cardiomyocytes and the fibroblasts are arranged on the chip and incubated for a given period of time for the cells to adhere onto the chip, and wherein the chip is stored at or below a temperature of about 25 °C before use, and the chip is cultured at about 37 °C to restore a function of the cells before use.

(23) The cardiomyocyte network chip according to (22) above, wherein the given period of time is at least about 12 hours.

(24) A method for preparing a cardiomyocyte network chip for use in evaluation of cardiomyocyte toxicity, comprising:

 (A) preparing a cardiomyocyte network chip comprising the following (i) to (v):

  (i) a substrate;
  (ii) a cell population comprising a plurality of stably pulsating subject cardiomyocytes or a cell population comprising the cardiomyocytes arranged on the substrate, and further comprising fibroblasts at a proportion corresponding to that of a heart;
  (iii) a cell population holding area formed on the substrate and configured to hold the cardiomyocytes or the cell population and a cell culture liquid;
  (iv) at least two measurement electrodes on each of which a single cell or a local portion of the cell population of the cardiomyocyte network comprising the plurality of the cardiomyocytes or the cell population comprising the cardiomyocytes is placed; and
  (v) lead wires connected respectively to each of the measurement electrodes,

 (B) incubating the cells to adhere onto the substrate for a given period of time, and

(C) storing, or storing and transporting the cardiomyocyte network chip at a temperature of about 25 °C or below.

(25) The method according to (24) above, wherein the given period of time is at least about 12 hours.

(26) The method according to (24) or (25) above, comprising re-culturing the cardiomyocyte network chip, which have been stored, or stored and transported, at a temperature of about 37 °C to restore the function of the cells before starting measurements of cellular potential of the cells.

(27) A method for manufacturing a cell culture chip comprising a culturing cell placed on a substrate, the method comprising arranging water-repelling materials in an area on the surface of the substrate other than the area on which the cells are to be arranged.

(28) The method according to (27) above, wherein the water-repelling material comprises a Teflon™ microparticle.

(29) A cell culturing chip for arranging culturing cells on a substrate, wherein an area of a surface of the substrate other than an area in which the cells are to be arranged has a water-repelling surface.

(30) The cell culturing chip according to (29) above, wherein the water-repelling surface is formed on the substrate such that the area on which the cells are arranged on the surface of the substrate has a linear, a parallel or a lattice shape on the substrate.

(31) The cell culturing chip according to (29) or (30) above, wherein the water-repelling surface is formed on the substrate by coating the surface of the substrate with water-repelling material.

(32) The cell culturing chip according to (31) above, wherein the water-repelling material comprises a material comprising Teflon™ microparticles.

(33) The cell culturing chip according to any one of (29) to (32) above, wherein probe micro-particles are arranged on the surface of the substrate to optically detect the cells.

Effect of the Invention

The present invention allows evaluating cardiomyocyte toxicity of a drug more effectively by measuring fluctuation of transmission rate of the excitation conduction or fluctuation of the transmission rate and using them as an index.

According to the present invention, changes in the response of cardiomyocytes and fibroblasts to an agent can be accurately evaluated by measuring fluctuations of cells.

Further, according to the present invention, *in vitro* cardiomyocyte level measurements are provided, which has made an evaluation at a level closer to an individual level possible.

In addition, the present invention has made it possible to measure an excitation conduction transmitting through a cardiomyocyte network while analyzing measurement results in real time, and further to determine toxicity of a drug based on the results analyzed. The realization of the real-time measurements and analyses has made it possible to feed-back the results of analyses immediately to a measurer, and to perform another measurement as needed.

In addition, as a result of an investigation of relationship between growth rates of fibroblasts used in the cardiomyocyte network and culturing temperatures, the present inventors have found that fibroblasts stop growing at or below a culturing temperature of about 25 °C, and the number of the fibroblasts decreases when the culturing temperature is lowered down to about 4 °C. For this reason, according to the present invention and based on this finding, it is now possible to store and transport cells while preventing growth of the fibroblasts and maintaining functions of the cells of the cardiomyocyte network chip in which fibroblasts are present at or below a temperature of 20 °C or ideally at a temperature ranging between 20 °C and 25 °C.

Further, the present invention provides a cardiomyocyte network chip in which water-repellent solids are appropriately arranged on a surface of a chip and cells are arranged only in an area in which the water-repellent solids are not arranged. With this technique, it is now possible to arrange a cellular network in which cells are arranged in a desired fashion (e.g. linearly, in a parallel or a lattice-like fashion) on the surface of the chip.

BRIEF DESCRIPTION OF THE DRAWINGS

[Figure 1] Figure 1 is a perspective view schematically showing an exemplary structure of a myocardial toxicity testing apparatus according to an example of the present invention.

[Figure 2] Figure 2 is a perspective view schematically showing an exemplary structure of a cell holding unit CH of the myocardial toxicity testing apparatus shown in Figure 1.

[Figure 3] Figure 3 is a diagram for illustrating an optical system for optically detecting a cell on the cell holding unit CH of the myocardial toxicity testing apparatus shown in Figure 1.

[Figure 4] Figures 4(a), 4(b) and 4(c) are diagrams showing signals associated with measurement of membrane potentials. Each diagram shows time along the horizontal axis and the membrane potential between the microelectrode 2 and the comparison electrode 2c along the vertical axis.

[Figure 5] Figures 5(a), 5(b) and 5(c) are diagrams showing signals associated with the changes in the volume due to cell pulsation, which is measured with the optical system.

[Figure 6] Figure 6(a) shows changes in the potentials according to the amounts of $Na^+$, $Ca^{2+}$ and $K^+$ ion in- and out-flow into/from the target cells under a normal state where the culture solution is free of agent. Figure 6(b) shows changes in the potentials according to the amounts of $Na^+$, $Ca^{2+}$ and $K^+$ ion in- and out-flow into/from the target cells under a state where the culture solution contains an agent.

[Figure 7] Figure 7 illustrates an exemplary arrangement of an optical system and a movable electrode of the myocardial toxicity testing apparatus for optically detecting the cells.

[Figure 8] Figure 8 is a schematic view for illustrating generation of an electric signal of a cell.

[Figure 9] Figure 9(a) shows an exemplary change in the membrane potentials upon addition of an agent; and Figure 9(b) shows one example of a Poincare plot for evaluating homology between two successive pulses with respect to the change in the membrane potentials upon each pulsation.

[Figure 10] Figure 10(a) is a schematic view showing an exemplary re-entry circuit prepared with an annular network of cardiomyocytes by means of a cell arrangement technique at single-cell level; and Figure 10(b) is a micrograph showing an actual exemplary arrangement of the cells on the microelectrodes.

[Figure 11] Figure 11(a) is a schematic view showing an exemplary re-entry circuit prepared with an annular network of cardiomyocytes using a cell population having a certain width; Figure 11(b) is a microscopic picture showing an actual exemplary arrangement of the cells on the microelectrodes; and Figure 11(c) is a microscopic picture showing an actual exemplary annular arrangement of the cell population on the microelectrode array.

[Figure 12] Figure 12(a) is a schematic view showing an exemplary re-entry circuit measurement apparatus using an annular electrode; and Figure 12(b) is a graph showing normal pulse data and abnormal pulse data actually measured with the electrode.

[Figure 13] Figure 13(a) is a schematic view showing an exemplary arrangement of an electrode for measuring potentials of a single cell and the cell; Figure 13(b) shows a picture of the isolated single cell on the electrode actually measured with the electrode and electric pulse data thereof; and Figure 13(c) shows a picture of a cell population measured on the electrode and a graph showing electric pulse data of one of the cells of the cell population.

[Figure 14] Figure 14 is a schematic view illustrating an example of the present invention in which a photo-sensitive element of the camera is used for measuring a potential of a single cell.

[Figure 15] Figure 15 is a schematic view illustrating an exemplary mechanism for measuring a plurality of samples with a cell measurement system of the present invention.

[Figure 16] Figure 16 is a schematic view illustrating cardiac information that can be measured with a cell measurement system of the present invention.

[Figure 17] Figure 17 is an example of a graph illustrating the addition of the agent changes to the field potential signal waveform of the cells measurable by the measurement system of the present invention cells.

[Figure 18] Figure 18 is an example of a graph illustrating an example of the average value of the changes in response to the addition of a potassium ion channel inhibitor E4031 in connection with elapsed time (FPD: field potential duration) of the peak position of the release of potassium ions from the release time of sodium ions in the signal waveform of the field potential of the cells that can be measured by the cell measurement system of the present invention.

[Figure 19] Figure 19 is an example of a graph and a formula illustrating one of the methods for evaluating quantitatively the size of fluctuation of short-term variability of adjacent pulsations on the basis of Poincare plotting in connection with elapsed time (FPD: field potential duration) of the peak position of the release of potassium ions from the release time of sodium ions in the signal waveform of the field potential of the cells that can be measured by the cell measurement system of the present invention.

[Figure 20] Figure 20 is an example of a graph and a formula illustrating one of the methods for evaluating quantitatively, based on Poincare plotting, the size of the fluctuation of elapsed time (FPD: field potential duration) of the peak position of the release of potassium ions from the release time of sodium ions in the signal waveform of the field potential of the cells that can be measured by the cell measurement system of the present invention.

[Figure 21] Figure 21 is an example of a representation of the size of the fluctuation produced by the addition of EE4031 in (a) Poincare plotting and (b) STVs in connection with elapsed time (FPD: field potential duration) of the peak position of the release of potassium ions from the release time of sodium ions in the signal waveform of the field potential of the cardiomyocytes that can be measured by the cell measurement system of the present invention.

[Figure 22] Figure 22 shows FPD and STV in the case of adding agents known to have a variety of cardiotoxicities on cardiomyocytes and a reference agent measurable by the measurement system cells of the present invention.

[Figure 23] Figure 23 shows an example of Poincare plotting of FPD against addition of an agent in terms of the difference in the shape of the cardiomyocyte network and the difference in position thereof which can be measured by the measurement system of the present invention. (a) A micrograph showing an example of an actual cellular network (a); (b) A graph showing measured changes at points A, B, C and D of (a).

[Figure 24] Figure 24 shows an example of Poincare plotting of the transmission time to a local point from the pacemaker area in response to an addition of an agent in terms of the difference in the shape of the cardiomyocyte network and the difference in the position thereof which can be measured by the measurement system of the present invention. (a) A micrograph showing an example of an actual cellular network (a); (b) A graph showing measured changes at points A, B, C and D of (a).

[Figure 25] Figure 25 is a diagram schematically showing a relationship between a conventional *in vitro* measurement method and a conventional *in vivo* measurement method, and a relationship between an FP waveform of a single cell and a composite FP waveform of a cellular network during the measurements of a network of cardiomyocytes measurable by the cell measurement system of the present invention.

[Figure 26] Figure 26 is a diagram schematically showing a configuration of an apparatus having a function to estimate membrane potentials of cells from the FP waveform of the cells collected from each electrode and a function to compose a comparison waveform of an electrocardiogram from a composite FP waveform of the cellular network during the cardiomyocyte-network measurements which are measurable by the cell measurement system of the present invention.

[Figure 27] Figure 27 shows examples of: (A) an annularly arranged cardiomyocyte network; (B) FP waveforms of cells obtained from each electrode of the network of (A); (C) a composite FP waveform composing the waveforms of (B). This example shows an example in which the pulsation signal is transmitted normally from the PM area.

[Figure 28] Figure 28 shows examples of: (A) an annularly arranged cardiomyocyte network; (B) FP waveforms of cells obtained from each electrode of the network of (A); (C) a composite FP waveform composing the waveforms of (B). This example shows an example in which the pulsation signal is transmitted abnormally from the PM area.

[Figure 29] Figure 29 is a graph showing an exemplary relationship between the beating frequency (beating frequency) of cardiomyocytes and the FPD during the cardiomyocyte-network measurements which are measurable by the cell measurement system of the present invention.

[Figure 30] Figure 30 is a graph showing an example of chronological change of FPD when forced pulsation is imparted to the cardiomyocytes during the cardiomyocyte-network measurements which are measurable by the measurement system of the present invention.

[Figure 31] Figure 31 is a photomicrograph showing an example of the cellular network arrangement when the FPD is measured when forced pulsation is imparted to cardiomyocytes during the cardiomyocyte-network measurements which are measurable by the measurement system of the present invention.

[Figure 32] Figure 32 is a schematic diagram showing an example of using a mechanism to maintain a constant potential at the microelectrodes using a feedback control of a trace electrode potential to measure the FP of cardiomyocytes during the cardiomyocyte-network measurements which are measurable by the measurement system of the present invention.

[Figure 33] Figure 33 is a graph showing an example of the relationship with the response of the beating frequency of the cardiomyocyte population when forced pulsation is given to a partial area of the cardiomyocyte population during the cardiomyocyte-network measurements which are measurable by the measurement system of the present invention.

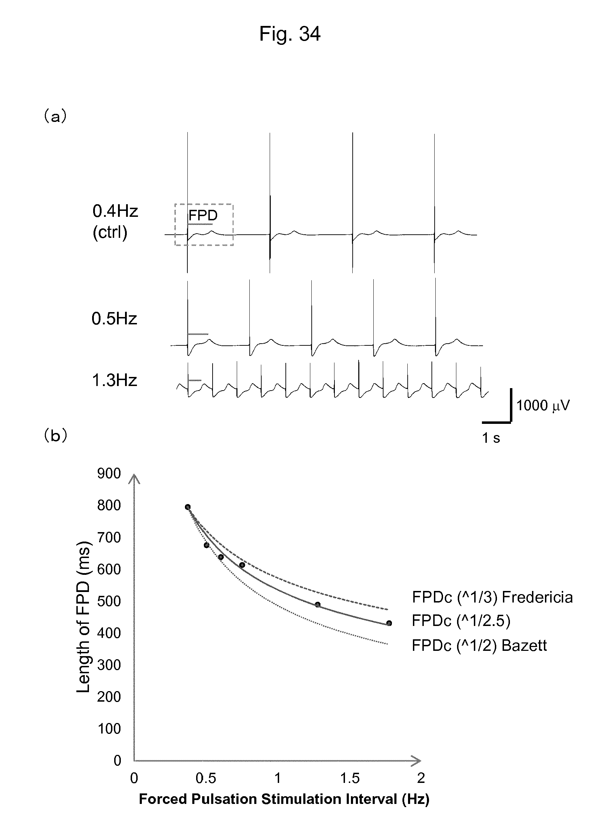

[Figure 34] Figure 34 is a graph showing an example of the change in the length of the FPD when forced pulsation is given to a partial area of the cardiomyocyte population during the cardiomyocyte-network measurements which are measurable by the measurement system of the present invention. A graph showing (a) an example of the relationship of the change in the length of the FPD and the change in the FP waveform caused by forced pulsatile stimulation; and (b) an example of the change in the length of the FPD in response to the change in the stimulation interval of the forced pulsatile stimulation

[Figure 35] Figure 35 is a table summarizing the results shown in Figure 33 and Figure 34 regarding an example of the response of the cell population when forced pulsation is given to a partial area of the cardiomyocyte population during the cardiomyocyte-network measurements which are measurable by the measurement system of the present invention.

[Figure 36a] Figure 36 schematically shows a difference circuit between a reference electrode and microelectrodes for noise removal during a measurement of an electrode potential in accordance with the present invention. (a) A schematic diagram of an example of a circuit illustrating the principles.

[Figure 36b] Figure 36b schematically shows a difference circuit between a reference electrode and microelectrodes for noise removal during a measurement of an electrode potential in accordance with the present invention. (b) A circuit diagram of an example of an amplifier circuit incorporating the difference circuit.

[Figure 36c] Figure 36c schematically shows a difference circuit between a reference electrode and microelectrodes for noise removal during a measurement of an electrode potential in accordance with the present invention. (c) A diagram showing an example in which noise is reduced by the circuit.

[Figure 37] Figure 37 is a diagram schematically showing an example of a comprehensive evaluation method for myocardial toxicity evaluation in accordance with the present invention. (a) The degree of a prolongation of the FPD from the FPD data of the cells is plotted in the X-axis, and the magnitude of temporal fluctuations of the FPD is plotted in the Y axis. (b) An example of a plot for the average data from the above results, plotted in the X-Y diagram.

[Figure 38] Figure 38 is a schematic diagram showing an example of the configuration of a system for measuring the cardiac toxicity of the present invention.

[Figure 39] Figure 39 is a schematic diagram and a photography showing an example of the configuration of the measurement chamber of the cell culture system to measure the cardiac toxicity of the present invention.

[Figure 40] Figure 40 is a diagram schematically showing a cross-section of the measuring cell culture plate.

[Figure 41] Figure 41 is a diagram schematically illustrating a configuration of the electrode wire electrode arrangement being disposed in a multi-electrode substrate.

[Figure 42] Figure 42 is a schematic diagram showing an example of a multi-electrode arrangement of electrodes on the substrate.

[Figure 43] Figure 43 is a schematic diagram showing an example of a system configuration of the present invention to simultaneously measure mechanical properties and electrical properties of cardiomyocytes.

[Figure 44] Figure 44 is an example of a data acquisition monitor screen showing an example of data obtained from an example of a system configuration of the present invention to simultaneously measure mechanical properties and electrical properties of cardiomyocytes.

[Figure 45] Figure 45 is an example of data obtained from an example of a system configuration of the present invention to simultaneously measure mechanical properties and electrical properties of cardiomyocytes.

[Figure 46] Figure 46 is a diagram illustrating an example of acquisition of direction data of cell displacements obtained from an example of a system configuration of the present invention to simultaneously measure mechanical properties and electrical properties of cardiomyocytes.

[Figure 47] Figure 47 is a diagram illustrating an example of a spatial arrangement of a myocardial cell system in the network system of the present invention to simultaneously measure mechanical properties and electrical properties of cardiomyocytes.

[Figure 48] Figure 48 is a diagram illustrating an example of waveform patterns of the extracellular potential in cells for measuring the electrical characteristics of cardiomyocytes.

[Figure 49] Figure 49 is a diagram showing an example of cellular potential and fluctuation changes in drug response of hERG ion channel of cardiomyocytes.

[Figure 50] Figure 50 is a diagram illustrating the principle of cell stimulation at any position by superposition of stimulation potentials from a stimulation electrode array.

[Figure 51] Figure 51 is a diagram illustrating effects of combining a zoom lens system and an objective lens having a numerical aperture less than 0.3 for the optical measurement of microparticles.

[Figure 52] Figure 52 is a diagram showing an example of results obtained with a cardiomyocyte network when electrophysiological extracellular potential data and changes in contractile force of cells are measured at the same time after administration of verapamil.

[Figure 53] Figure 53 is a diagram showing an example of analysis results obtained with a cardiomyocyte network when electrophysiological extracellular potential data and changes in contractile force of cells are simultaneously measured after administration of verapamil.

[Figure 54] Figure 54 is a diagram showing an example of analysis results obtained with a cardiac muscle cell network when an electrophysiological extracellular potential, its fluctuation data, the fluctuation amounts of changes in the contractile force of cells in the direction of displacement and angular direction are simultaneously measured after administration of verapamil and their use in the analysis.

[Figure 55] Figure 55 is an example of a configuration that combines extracellular potential measurement and a contractile-function-change measuring optical system.

[Figure 56] Figure 56 illustrates an example of a configuration of a high-throughput myocardial-cell network array chip comprised of a cell culture module array.

[Figure 57] Figure 57 illustrates an example of a configuration of a cellular network deployment technique using a sample loader for placing cardiomyocytes in each well effectively.

[Figure 58] Figure 58 illustrates another example of a system for measuring an extracellular potential by a multi-electrode system in which block-type wells are actually arranged.

[Figure 59] Figure 59 illustrates an example of a configuration incorporating an optical measurement module to the system of Figure 58.

[Figure 60] Figure 60 is a diagram illustrating the structure of a substrate on which microprojections are regularly disposed to prevent contraction of cardiomyocytes in a culture medium during measurements using a myocardial-cell network and a myocardial cell sheet.

[Figure 61] Figure 61 is a schematic view showing an example of an electrode arrangement on a multi-electrode substrate.

[Figure 62] Figure 62 illustrates schematically an example of an embodiment in which metal micro wires are used as electrodes.

[Figure 63] Figure 63 is a schematic illustration of an example of a procedure used for arranging cardiomyocytes.

[Figure 64] Figure 64 shows an example of a result showing a population effect to result in pulsation stability of cardiomyocytes.

[Figure 65] Figure 65 schematically shows an example of the method of measuring excitation conduction transmitting through a cardiomyocyte network.

[Figure 66] Figure 66 schematically shows an example of a result of a measurement of excitation conduction transmitting through a cardiomyocyte network.

[Figure 67] Figure 67 shows an example of a procedure of a measurement, a storing of results and an analysis of excitation conduction transmitting through a cardiomyocyte network.

[Figure 68] Figure 68 shows a relationship between a growth rate of fibroblasts used in the cardiomyocyte network and a culturing temperature. (a): A graph of growth curves; (b): Microscopic photographs of actual fibroblasts at respective temperatures taken on Day 1 and Day 7 of the culturing.

[Figure 69] Figure 69 schematically shows an example of the procedure for creation, storage and use of a cardiomyocyte network in which fibroblasts are mixed.

[Figure 70] Figure 70 schematically shows an example of a structure of a cardiomyocyte network chip on which water-repellent solids are arranged.

[Figure 71] Figure 71 shows microscopic photographs showing examples of culturing of cardiomyocytes on a chip on which Teflon™ microparticles are arranged in water-repelling areas.

[MODE FOR CARRYING OUT THE INVENTION]

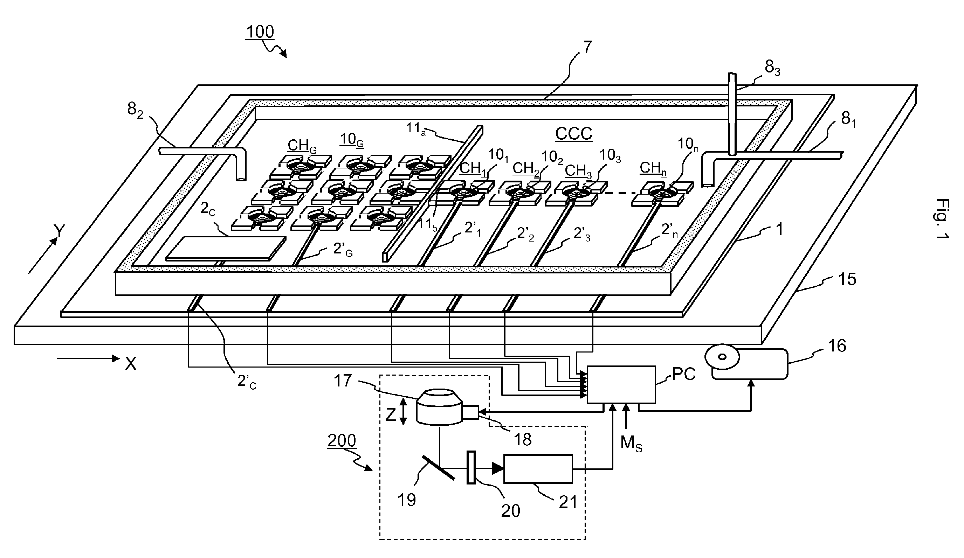

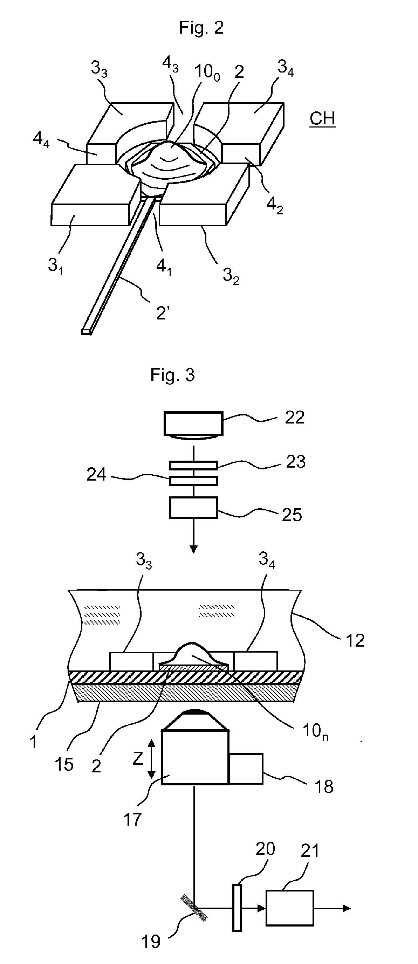

Figure 1 is a perspective view schematically showing an exemplary structure of an apparatus for testing myocardial toxicity according to an example of the present invention. Figure 2 is a perspective view schematically showing an exemplary structure of a cell holding unit CH of the myocardial toxicity testing apparatus shown in Figure 1. Figure 3 is a view for illustrating an optical system for optically detecting the cell retained in the cell holding unit CH of the myocardial toxicity testing apparatus shown in Figure 1.

Referring to Figure 1 and Figure 2, the myocardial toxicity testing apparatus 100 mainly consists of parts built on a transparent substrate 1. The transparent substrate 1 is an optically transparent material, for example, a glass substrate or a silicon substrate. The microelectrodes 2 are transparent ITO electrodes, for example, arranged on the transparent substrate 1. Reference numeral 2' denotes readout lines from the microelectrodes 2. Reference numerals $3_1$, $3_2$, $3_3$ and $3_4$ denote agarose gel walls, which are arranged around each of the microelectrodes 2 with gaps $4_1$, $4_2$, $4_3$ and $4_4$. The agarose gel walls $3_1$, $3_2$, $3_3$ and $3_4$ are cutout in the middle to form a space as cell housing. The microelectrode 2 is placed on the transparent substrate 1, as necessary, within the space as the cell housing formed with the agarose gel walls $3_1$, $3_2$, $3_3$ and $3_4$. Regardless of the presence of the microelectrode 2, a single cell 10 can be retained in the cell housing. In Figure 2, the microelectrode 2 is arranged on the transparent substrate 1 within the space as the cell housing formed with the agarose gel walls $3_1$, $3_2$, $3_3$ and $3_4$, where a cardiomyocyte 10 is additionally retained on the microelectrode 2. The microelectrode 2 is shown to be connected to the readout line 2'. A material, e.g., collagen, which enhances cellular adherence to the electrode surface or the transparent substrate, is preferably applied onto the cell-bearing surface of the microelectrode 2 or, directly onto the transparent substrate 1 when the cell is disposed in the absence of the microelectrode 2. Since the cell within the cell housing formed with the agarose gel walls $3_1$, $3_2$, $3_3$ and $3_4$ is non-adherent to the agarose gel, the cell 10 will not transfer beyond the walls even if its height is equivalent to the heights of these walls $3_1$, $3_2$, $3_3$ and $3_4$. Furthermore, since the gaps $4_1$, $4_2$, $4_3$ and $4_4$ surrounding the cell housing formed by cutting out in the middle of the agarose gel walls $3_1$, $3_2$, $3_3$ and $3_4$ are smaller than the size of the cell, the cell 10 will not move across these gaps $4_1$, $4_2$, $4_3$ and $4_4$.

With reference to Figure 1, the cell holding units $CH_1$, $CH_2$, $CH_3$ and $CH_n$ each retains a cardiomyocyte or a fibroblast $10_1$, $10_2$, $10_3$ or $10_n$ in the cell housing. Each holding unit is provided, although not evident from the figure, with the microelectrode 2 from which extends the readout line $2'_1$, $2'_2$, $2'_3$ or $2'_n$. These cardiomyocytes or fibroblasts form a tandemly arranged cell communication channel CCC. Here, "n" is, for example, 20. Although these twenty tandemly-arranged cardiomyocytes and fibroblasts may be allocated randomly, the cells in the cell holding units $CH_1$ and $CH_{20}$ are preferably cardiomyocytes. On the left side of this cell communication channel CCC are provided 3 x 3 cell holding units $CH_G$ to form a region that retains a cardiomyocyte population $10_G$ where each cell holding unit CH retains a cardiomyocyte 10. This cell population $10_G$ serves as a stably-pulsating pacemaker. Among the cell population $10_G$, only one of the cell holding units CH is provided with the microelectrode 2 from which extends the readout line $2'_G$. In addition, the right middle cell holding unit CH of the cell population $10_G$ is arranged to face the cell holding unit $CH_1$ of the cell communication channel CCC. A barrier $11_a$ is provided on the right of the cell population $10_G$ and the left of the cell communication channel CCC. A small opening $11_b$ is formed in the lower middle part of this barrier $11_a$. On both sides of this opening $11_b$, the right middle cell holding unit CH of the cell population $10_G$ is facing the cell holding unit $CH_1$ of the cell communication channel CCC to allow physical contact/intercellular interaction between the cells retained in the cell housings via the gaps 4 at the periphery of the housings. A comparison electrode 2c is provided below the cell population $10_G$, from which the readout line 2'c extends.

Reference numeral 7 denotes a surrounding wall that surrounds the cell population $10_G$, the cell communication channel CCC and the comparison electrode 2c. Reference numerals $8_1$ and $8_2$ denote pipes for supplying a cell culture solution into the region surrounded by the wall 7 and for draining the cell culture solution from the region surrounded by the wall 7. In the case of this figure, a culture solution is supplied from the pipe $8_1$ extending toward the bottom surface of the substrate 1 and drained from the pipe $8_2$ extending from the bottom surface of the substrate 1. A pipe $8_3$ is connected to the culture solution-supplying pipe $8_1$ near the culture solution outlet so that an agent that acts on the cells is supplied via this pipe $8_3$. Accordingly, the cells 10 are exposed to the cell culture solution supplied from the pipe $8_1$ into the region surrounded by the wall 7, while being stably retained on the microelectrodes 2. Once the cells no longer need to be exposed to the culture solution, the culture solution can be drained from the region surrounded by the wall 7 with the pipe $8_2$. Moreover, when the culture solution needs to be exchanged with a fresh culture solution, the culture solution may be supplied after or while draining the cell culture solution. On the other hand, if one wants to affect the cells with an agent, the agent for affecting the cells may be added to the culture solution via the pipe $8_3$ for supply together with the culture solution via the pipe $8_1$ while draining the cell culture solution from the pipe $8_2$. In this case, due to the barrier $11_a$ provided between the cell population $10_G$ and the cell communication channel CCC, when the culture solution containing the agent is supplied into the region surrounded by the wall 7 from the pipe $8_1$, the cells of the cell population $10_G$ are less influenced by the agent than the cells of the cell communication channel CCC. Specifically, when an agent-containing culture solution is supplied via the pipe $8_1$, this culture solution flows through the spacing between the wall 7 and the both edges of the barrier $11_a$ as well as over the top of the barrier $11_a$ toward the cell population $10_G$. Thus, the cells of the cell population $10_G$ are also affected by the agent. This influence, however, is indirect compared to the influence on the cells of the cell communication channel CCC, and thus it does not affect the function as a pacemaker. The structures and arrangements of the pipes $8_1$, $8_2$ and $8_3$ may arbitrarily be changed depending on the measurement configuration. For example, the pipes $8_1$ and $8_3$ may be separated, or the pipe $8_2$ may be omitted by using the pipe $8_1$ for both supply and drainage.

PC refers to a personal computer (potential measurement means, control/recording means), which measures and records the membrane potentials between the readout lines 2' from the microelectrodes 2 of the cell holding units CH and the readout line 2' from the comparison electrode 2c. Furthermore, operation signals Ms from an operator are input into the personal computer 9.

The myocardial toxicity testing apparatus 100 may be mounted on an XY stage 15 of the optical observation device 200 where the pulsation of a certain cell 10 of the cell communication channel CCC can be observed with an optical system. The XY stage 15 is optically transparent and may be moved to a given position with an X-Y drive unit 16 according to the signal given by the personal computer PC reflecting the operation signal Ms from the operator. Figure 3 shows an exemplary configuration for observing the pulsating state of a cell $10_n$ of the cell communication channel CCC. Reference numeral 12 denotes a culture solution.

Reference numeral 22 denotes a light source of a phase-contrast microscope or a differential interference microscope. Generally, a halogen lamp is used. Reference numeral 23 denotes a band pass filter that only allows transmission of light with a specific wavelength from the light source for observation with a stereoscopic microscope such as a phase-contrast microscope. For example, in the case of observing the cell $10_n$, narrow-band light having a wavelength in the vicinity of 700 nm is used to prevent damage to the cell $10_n$. Reference numeral 24 denotes a shutter that has a function of blocking irradiation light when image measurement is not executed, for example, while moving the XY stage 15. Reference numeral 25 denotes a condenser lens, where a phase ring is installed for phase-contrast observation or a polarizer for differential interference observation. The myocardial toxicity testing apparatus 100 formed on the substrate 1 is mounted on the XY stage 15 which can be moved with the X-Y drive unit 16 to observe and measure a certain location of the myocardial toxicity testing apparatus 100. The pulsating state of the cell $10_n$ in the myocardial toxicity testing apparatus 100 is observed with an objective lens 17. The focal position of the objective lens 17 can be transferred in the Z-axis direction with a drive unit 18 according to the signal from the PC. The magnification of the objective lens 17 may be 40 or higher. The objective lens 17 allows observation of a phase-contrast image or a differential interference image of the cell $10_n$ obtained with light transmitted from the light source 22. A diachronic mirror 19 and a band pass filter 20 that reflect light having the same wavelength as the light that passes through the band pass filter 23 allow observation of only a phase-contrast microscope image or a differential interference microscope image with a camera 21. The image signal observed with the camera 21 is input into the personal computer PC. In addition, although it is not illustrated in a diagram, images are displayed on a monitor or a display connected to the PC.

Exemplary dimensions of the structures of the myocardial toxicity testing apparatus 100 shown in Figure 1 are as follows. In this example, the size of a cell is 10 $\mu m\varphi$. The transparent substrate 1 has dimensions of 100 mm x 150 mm, the microelectrode 2 has dimensions of 8 $\mu m$ x 8 $\mu m$ and each of the agarose gel walls $3_1$, $3_2$, $3_3$ and $3_4$ has dimensions of 20 $\mu m$ x 20 $\mu m$ x 10 $\mu m$ (height). Each of the gaps $4_1$, $4_2$, $4_3$ and $4_4$ has a width of 2 $\mu m$, the cell housing formed with the agarose gel walls $3_1$, $3_2$, $3_3$ and $3_4$ has a 12 $\mu m\varphi$ cylindrical space, and the wall 7 has external dimensions of 5 mm x 5 mm with a height of 5 mm. The height of the barrier $11_a$ is 1 mm. Although the microelectrode 2 has a square shape of 8 $\mu m$ x 8 $\mu m$ in this example, it may be an annular electrode of 10 $\mu m\varphi$ that corresponds to the shape of the cell housing made with the agarose gel walls $3_1$, $3_2$, $3_3$ and $3_4$ and the widths of the gaps $4_1$, $4_2$, $4_3$ and $4_4$.

Hereinafter, an exemplary structure of the cell response measurement apparatus 100 of the present invention and a specific example of measurement using the same will be described.

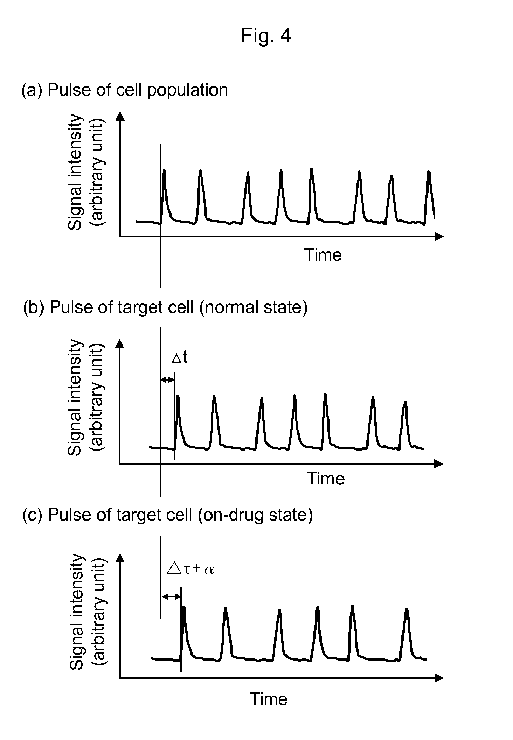

Figures 4(a), 4(b) and 4(c) are diagrams showing signals associated with measurement of membrane potentials. Each diagram shows time along the horizontal axis and the membrane potential between the microelectrode 2 and the comparison electrode 2c along the vertical axis. Figure 4(a) shows membrane potentials resulting from the pulses of the cell population $10_G$. Here, a potential refers to an electric difference between the readout line $2'_G$ extending from one of the cell population $10_G$ and the readout line $2'_C$ extending from the comparison electrode 2c shown in Figure 1. The diagram shows stable pulses indicating that the cells are capable of serving as a pacemaker. Figure 4(b) shows membrane potentials resulting from the pulses of a target cell in a normal state where the culture solution does not contain an agent. Here, a cell targeted for measurement is the cell $10_n$ of the cell communication channel CCC, where the potential between the readout line $2'_n$ extending from the cell $10_n$ and the readout line $2'_C$ extending from the comparison electrode 2c are measured. As can be appreciated from comparison with the waveform of Figure 4(a), the time required for conducting the pulse of the cell 10 of the cell communication channel CCC is delayed by $\Delta t$. Meanwhile, Figure 4(c) shows membrane potentials resulting from the pulse of the target cell in a state where the culture solution contains an agent. Again, the cell targeted for measurement is the cell $10_n$ of the cell communication channel CCC for the sake of facilitating comparison with Figure 4(b). As can be appreciated from comparison with the waveforms of Figures 4(a) and 4(b), the time required for conducting pulse of the cell 10 of the cell communication channel CCC is found to be delayed not just by $\Delta t$ but by $\Delta t + \alpha$. This means that the level of the Na-ion inhibition due to the agent acting on the cell of the cell communication channel CCC appears as the increase in the delayed time, i.e., $+\alpha$. Specifically, toxicity of an agent on a cardiomyocyte can be assessed as sodium-ion inhibition. It should be noted that microelectrodes that are used for observation may be referred to as observation electrodes herein.

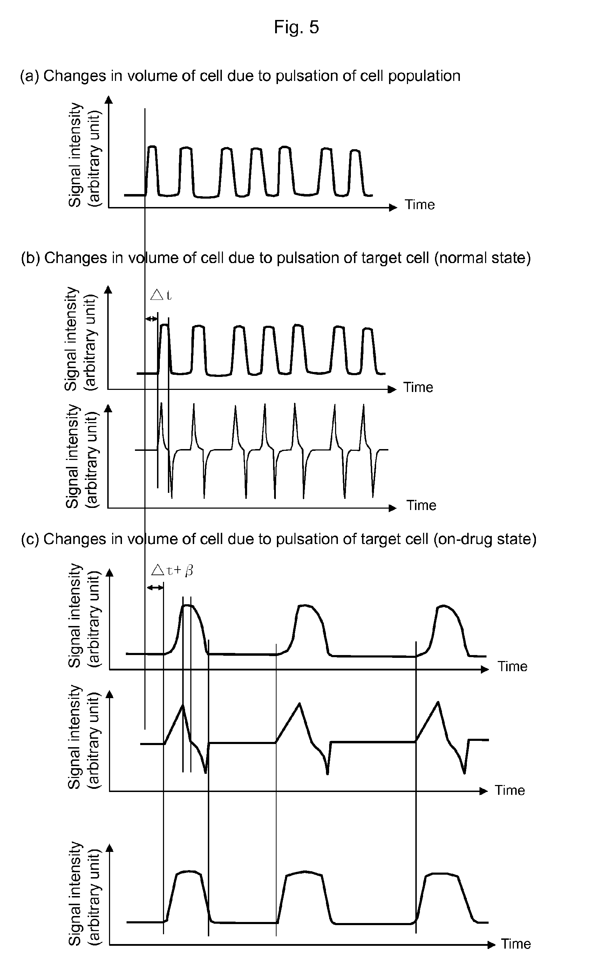

Figures 5(a), 5(b) and 5(c) are diagrams showing signals associated with the changes in the volume due to pulse of cells, which is measured with the optical system. Figure 5(a) shows the change in the volume associated with pulse of a cell of cell population $10_G$, where the pulse of one of the cells of the cell population $10_G$ is optically detected with the configuration shown in Figure 3. The contraction and dilatation associated with the pulsation of the cell can be observed as pulse-shaped changes. The cycle of this waveform is the same as the cycle of the changes in the membrane potential associated with the pulsation shown in Figure 4(a). Figure 5(b) shows, in the upper diagram, the change in the volume associated with the pulsation of the target cell under the normal state where the culture solution is free of the agent, and shows, in the lower diagram, a waveform of the same in time-differential values for evaluation as electric signals. Again, the cell targeted for measurement is the cell $10_n$ of the cell communication channel CCC, where the pulse of the cell $10_n$ is optically detected with the configuration shown in Figure 3. As can be appreciated from comparison with the waveform shown in Figure 5(a), the time required for conducting pulse of the cell 10 of the cell communication channel CCC is delayed by $\Delta t$. Meanwhile, Figure 5(c) shows diagrams for evaluating changes in the volume associated with the pulsation of the target cell under the state where the culture solution contains an agent. In Figure 5(c), the time axes are extended when compared to those in Figures 5(a) and 5(b). The upper diagram represents a waveform corresponding to the waveform of the upper diagram of Figure 5(b), where the time required for conducting pulse of the cell 10 of the cell communication channel CCC is further delayed by $\beta$ in addition to $\Delta t$ as can be appreciated by comparison with the waveform shown in Figure 5(a). The influence on the change in the volume associated with the pulsation of the target cell is more prominent in a smaller inclination of the change in the volume rather than the increase in the delay. This is apparent from comparison with the change in the volume with an agent-free culture solution shown as a reference waveform in the lower diagram in Figure 5(c). The middle diagram of Figure 5(c) shows the waveform of the upper diagram processed as time-differential values for evaluation thereof. As can be appreciated from comparing the time-differential values with those shown in the lower diagram of Figure 5(b), smaller peak values are associated with increased gentleness in the inclination. This means that the agent decreased the contraction rate of cardiac muscle and therefore the cardiac output is also decreased. In other words, toxicity of an agent on the cardiomyocyte can be evaluated as a decrease in the contraction rate.

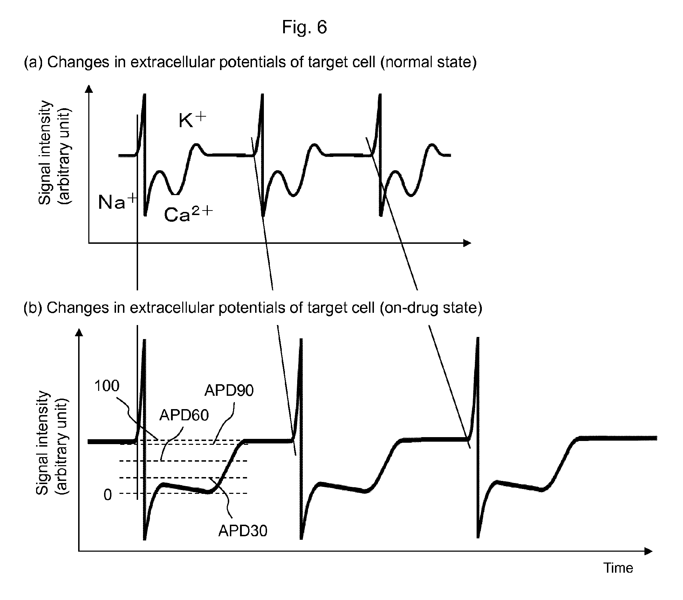

Figure 6(a) shows changes in the potentials according to the amounts of $Na^+$, $Ca^{2+}$ and $K^+$ ion in- and out-flow into/from the target cells under a normal state where the culture solution is free of agent. Figure 6(b) shows changes in the potentials according to the amounts of $Na^+$, $Ca^{2+}$ and $K^+$ ion in- and out-flow into/from the target cells under a state where the culture solution contains an agent. As can be appreciated by a cursory comparison of Figures 6(a) and 6(b), QT prolongation emerges where the waveform is extended along the time axis. Moreover, the waveform is largely deformed due to in- and out-flow of the $K^+$ ions. In order to evaluate this as an electric signal, the durations of the detected 30%, 60% and 90% values are shown as APD30, APD60 and APD90, respectively, with respect to the broken lines indicating the values between "0" and "100" in the diagram. Here, APD stands for action potential duration. Evaluations of the magnitudes and percentages of these values can provide evaluation of influence of the agent on the amounts of the $Na^+$, $Ca^{2+}$ and $K^+$ ion in- and out-flow.

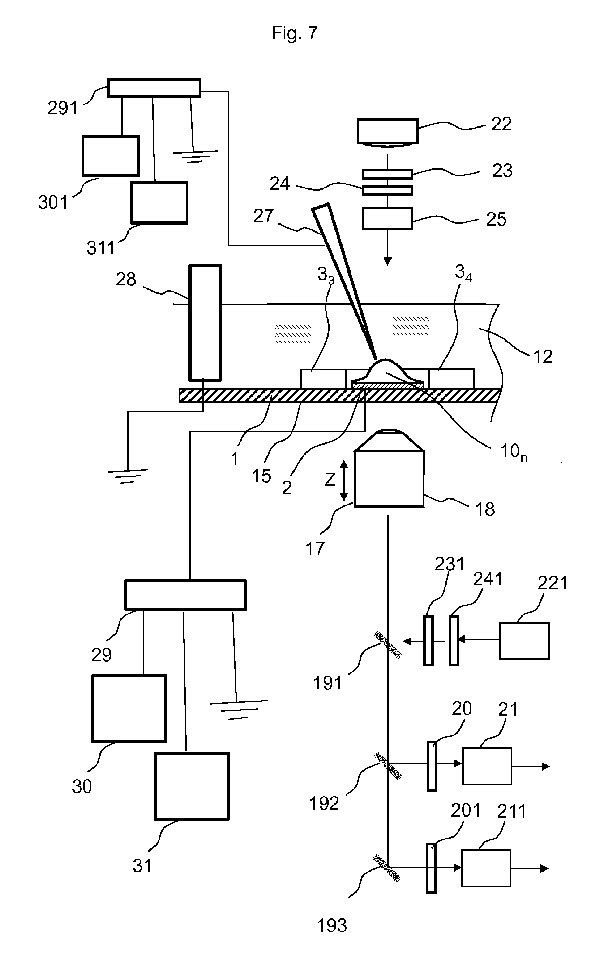

Figure 7 is a view illustrating an exemplary arrangement of an optical system and a movable electrode of the myocardial toxicity testing apparatus for optically detecting the cells, in which observation of the pulsating state, for example, of the cell $10_n$ to be measured is exemplified. Reference numeral 12 denotes a culture solution. Reference numeral 22 denotes light source for a phase-contrast microscope or a differential interference microscope, which is generally a halogen lamp. Reference numeral 221 denotes a fluorescent light source for fluorescent measurement of the cells, which is generally a mercury lamp, a monochromatic laser, an LED light source, or the like. Reference numeral 23 denotes a band pass filter that allows transmission of only light with a particular wavelength from the light source for observation with a stereoscopic microscope such as a phase-contrast microscope, while reference numeral 231 denotes a band pass filter that allows transmission of only light with an excitation wavelength that excites particular fluorescence from the fluorescent light source 221. For example, when observing the change in the shape such as information of change in the volume of the pulse of the cell $10_n$, an image that passed the band pass filter 20 that allows only light with a wavelength for measuring the cell shape is measured with the camera 21 on a real-time basis, where narrowband light having the wavelength in the vicinity of 700 nm is used for measurement to prevent damage of the cell $10_n$. Reference numerals 24 and 241 denote shutters that have a function of blocking irradiation light when image measurement is not executed, for example, while moving the XY stage 15. Reference numeral 25 denotes a condenser lens, where a phase ring is installed for phase-contrast observation or a polarizer for differential interference observation. In the case of fluorescent measurement, for example, in the case of intracellular calcium release measurement, a combination of a band pass filter that selectively passes light with the excitation wavelength of approximately 500 nm and a band pass filter that selectively passes light with the fluorescent measurement wavelength of approximately 600 nm is used, to measure, with the camera 201, the fluorescent image that passed through the band pass filter 201 that only selectively passes light with the fluorescent wavelength. In this case, if calcium release per cell unit in the cell network is to be measured in terms of time to determine the pathway of the signal conduction in the cell network, continuous high-speed images can be acquired with the time resolution of the camera being 0.1 ms or less. The myocardial toxicity testing apparatus 100 formed on the substrate 1 is mounted on the XY stage 15 which can be moved with the X-Y drive unit 16 to observe and measure certain location of the myocardial toxicity testing apparatus 100. The pulsating state of the cell $10_n$ in the myocardial toxicity testing apparatus 100 is observed with an objective lens 17. The focal position of the objective lens 17 can be transferred in the Z-axis direction with a drive unit 18 according to the signal from the personal computer PC. The magnification of the objective lens 17 may be 40 or higher. The objective lens 17 allows observation of a phase-contrast image or a differential interference image of the cell $10_n$ obtained with light transmitted from the light source 22. A diachronic mirror 192 and a band pass filter 20 that reflect light with the same wavelength as the light that passes through the band pass filter 23 allow observation with a camera 21 of only a phase-contrast microscope image or a differential interference microscope image. The image signal observed with the camera 21 is input into the personal computer PC. Moreover, according to this example, a movable electrode 27 for stimulating a cell is arranged with a position controlling mechanism for adjusting the coordinates of the movable electrode with respect to not only within the plane parallel to the plane of the XY stage but also with respect to its height. Using this position controlling mechanism, the tip of the movable electrode is transferred to stimulate one or more particular cells in the cell network. The movable electrode may be a metal electrode provided with an insulating coating except for the tip, a glass electrode having the opening size of the tip of about 5 micrometers or less, or the like, where any electrode that can apply electrical stimulation only to a particular cell or cells in the vicinity of the tip of the movable electrode can be used. When a metal electrode is used, platinum black or the like may be applied to the tip surface for effectively transmitting electrical stimulation to the cell(s). The positioning of the tip of the movable electrode can be adjusted according to the level of the response of the cell(s) to the electrical stimulation, and may make a contact with the cell(s) or placed near the cell(s). In addition, in order to accurately apply stimulation from the stimulation electrode to the target cell(s), the electrode 2 for measuring the membrane potentials may be used as a ground electrode by switching the electrode at the moment of applying electrical stimulation, or a separate ground electrode 28 may be provided. Moreover, in order to stimulate a particular cell, the existing microelectrode 2 may be used as a stimulation electrode. In this case, the switching circuit 29 connected to the microelectrode is switched upon stimulation so that the microelectrode that is usually connected to an electric signal measurement circuit 30 is connected to an electrical stimulation circuit 31 for applying square-wave stimulation signals to the microelectrode 2. Furthermore, when the movable electrode 27 is used to provide stimulation, the switching circuit 29 may be switched to a grounding state. On the other hand, the movable electrode may also be used not only as a stimulation electrode, but also as an electrode for measuring the electric signal of the cell(s) or as a ground electrode. In this case, the movable electrode is connected to a switching circuit 291, and switched, according to its use, i.e., for membrane potential measurement, for cell stimulation or as a ground electrode, is connected to an electric signal measurement circuit 301 to measure the membrane potential, is connected to an electrical stimulation circuit 311 for applying a square-wave stimulation signal to the cell(s), or is grounded for use as a ground electrode, respectively. The timing of the electrical stimulation applied to the cells with the electrical stimulation circuits 31 and 311 can be employed primarily for the following two applications. One is to apply irregular stimulations between the pulse intervals of the normal cardiomyocyte network in an autonomous pulsation configuration. The other is to provide pulse interval to the cardiomyocyte network without an autonomous pulsation configuration. In both cases, changes in the response of the cell network can be traced through measurement by gradually shortening the cycle of the pulse interval (time interval between two pulses) by 5 ms. In order to do so, the electrical stimulation circuits 31 and 311 can analyze the pulsation cycle information acquired with the electric signal measurement circuits 30 and 301 and conduct feedback regulation based on the acquired results to determine the timing of the stimulation. Moreover, when the movable electrode 27 is used for the electric signal measurement, measurement can equivalently be carried out in the present system without the microelectrode 2. Since the pulsation cycle of each cell in the cell network can be measured by the optical measurement installed in the system, a change from a stable state to an unstable state such as abnormal cardiac rhythm in this pulsation cycle can be measured only with the optical measurement device arranged in the system. Then, if necessary, the movable electrode is used to acquire the data of the electric property of the particular cell from these results. In this case, the number of the microelectrodes arranged on the system in the first place is not limited, and a larger cell network can be configured freely as long as optical measurement is possible.

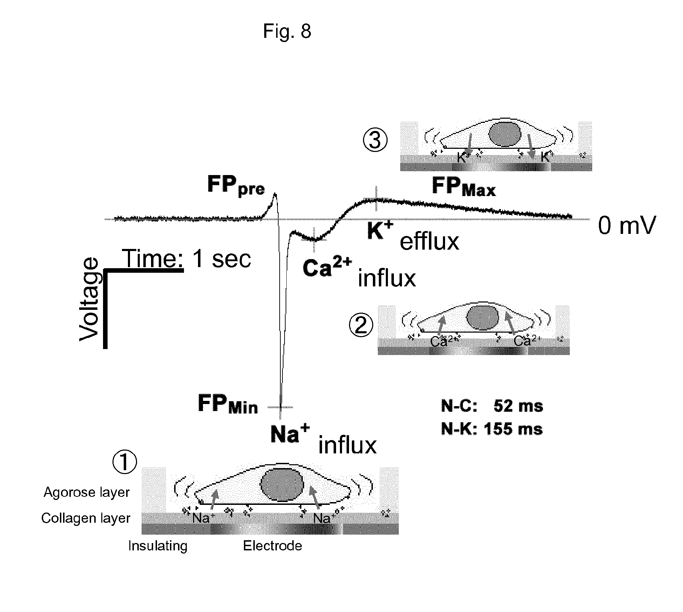

Figure 8 shows a schematic view of an example of generation of an electric signal of a cell. First, inflow of sodium ions into a cell occurs via sodium-ion channels on the cellular membrane, where the membrane potential is rapidly decreased. Then, the membrane potential is decreased after a slight delay due to inflow of calcium ions, and then as the subsequent step, outflow of potassium ions from the cell occurs where the membrane potential is increased. The changes in the membrane potentials occur due to the different response imparted by the properties of various ion channels present in the cardiomyocyte membrane. By analyzing the positions of the peaks of change in the potentials caused by the respective ion channels as time characteristic of the ion channels, the changes in the waveforms of the electric signals can be measured for each type of the ion channels that are blocked due to the effect of the agent. As a result, an inhibition effect of the agent on the ion channels can be estimated. There are four particularly important ion channels for evaluation of an agent, i.e., FastNa, SlowNa, Ca, IKr and IKs. Blocking of these four types of ion channels can be measured.

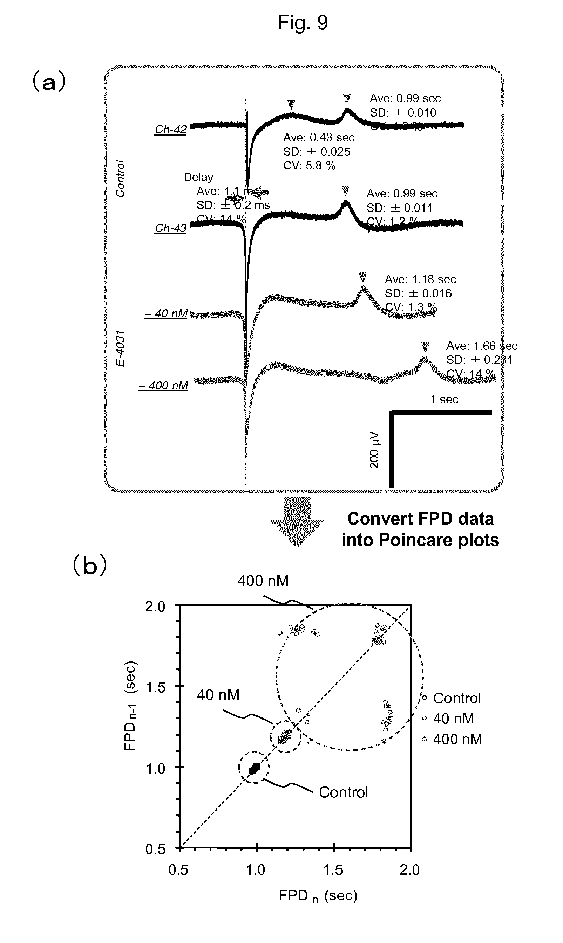

Figure 9(a) shows the influence on the electric signals of the cell shown in Figure 8 upon actual addition of reagent E-4031 at various concentrations that selectively inhibits the potassium-ion channels. Since the IKr ion channel that is responsible for outflow of K-ion from the cells and that increases the membrane potential is inhibited, a change in the membrane potentials can be observed to be gradually delayed in the positive direction as the concentration of the agent increases. Figure 9(a) shows data of a particular single pulsation of a cellular response. In practice, the magnitude of the fluctuation width of the responses between the successive pulses is an important index for estimating the influence of the agent. Figure 9(b) shows one example of an analysis technique where successive pulse data called Poincare plots are compared correlatively. Here, the X-axis represents plots of response time of a particular ion channel upon the n-th pulse while the Y-axis represents plots of the response time of the same ion channel upon the (n + 1)-th pulse. Accordingly, if the properties of the successive pulses are the same, the plots will be drawn along the Y = X line represented by the broken line in the graph. If there is a significant fluctuation in the responses between the successive pulses, the plots observed will be placed distant from the Y = X line. In fact, in this example, although addition of 40 nM results in the delay of the response time as compared to the control without addition of the agent, homology between the successive pulses remains the same. At the same time, these plots reveal that addition of the agent up to 400 nM further delays the response time, and homology is no longer retained between the successive pulses, resulting in generation of an unstable pulsation cycle. This result agrees with the results of prolongation in the QT interval measurement representing cardiac toxicity. Generation of a prolongation of the QT interval can be estimated by using the Poincare plots as an index of increase in the fluctuations of the successive pulses at a cellular level. This phenomenon can be described as follows: when a particular ion channel is blocked with an agent, only a phenomenon of decrease in the ion outflow ability is observed where the degree of the blocking is small and the cell response is not yet unstable. In contrast, when the degree of blocking increases as the number of functioning ion channels becomes extremely decreased, the reproducibility of the ion outflow ability deteriorates and fluctuation for the same cell increases. Hence, the magnitude of this fluctuation can be used as an index of likelihood of generating a prolongation of QT interval.

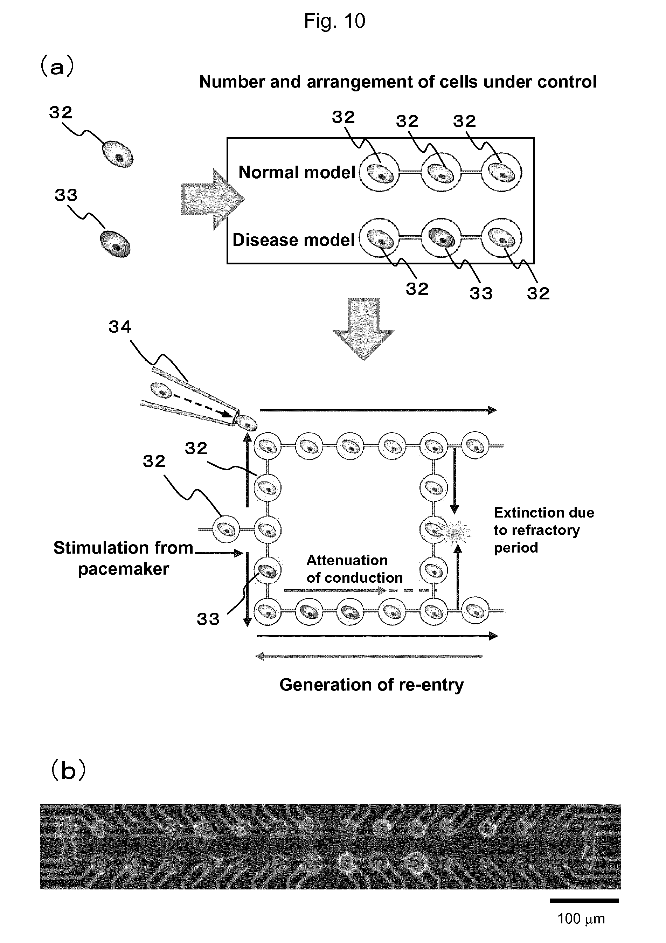

Figure 10(a) is a schematic view showing an example of an agent for a re-entry circuit with an annular network of cardiomyocytes using a cell arrangement technique at a single-cell level. An annular network produced with only cardiomyocytes is used as a normal network model. A pathologic model such as cardiac hypertrophy is realized by incorporating fibroblast cells into the cell network. The fibroblast cells present in the network will cause delay of the conduction velocity or attenuation of the conduction of the cardiomyocyte network, as a result of which, generation of premature contraction can be estimated. Figure 10(b) is a microscopic picture showing an example of actual arrangement of cardiomyocytes on the microelectrodes. In fact, when the cells are arranged on the microelectrodes in cell units as shown in this picture, delay in the signal conduction between the adjacent cardiomyocytes can be measured. Since this conduction velocity depends on the magnitude of the first electric signal generated upon pulsation, data for delay in this signal conduction can be interpreted as the inhibitory effect on the sodium-ion channel.

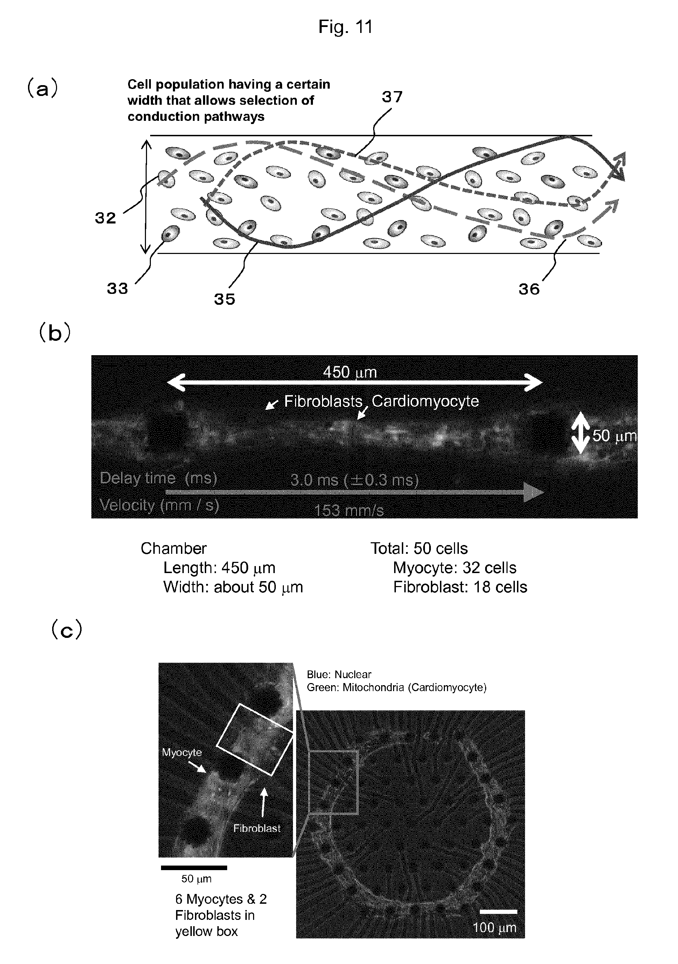

Figure 11(a) is a schematic view showing an exemplary re-entry circuit by an annular network of cardiomyocytes using a cell population having a certain width. In the annular cell network in cell units shown in Figure 10, pulsation signals of the cardiomyocytes are uniquely transmitted, and the cells will transmit pulsation signals between the adjacent cells while maintaining the same property unless there are fluctuations in the pulses of the cells themselves as shown in Figure 9. On the other hand, when the cells were arranged with a certain width to form an annular network as shown in Figure 11, the cell population will be imparted with the flexibility to have different conduction pathways for different pulses as represented by solid line 35, broken line 36 and dotted line 37. In particular, when a large fluctuation occurs in the response property of each cardiomyocyte due to the addition of an agent as described with reference to Figure 9, the cells that are likely to respond differ in response to the travel of the stimulation signals through the annular network, thereby rendering the difference in the pathways significant. Since this is the same mechanism as the mechanism of premature contraction, i.e., a fatal cardiac status called spiral/re-entry, measurement of spiral/re-entry becomes possible by particularly using an annular network based on cell population having such a width. Figure 11(b) is a microscopic picture showing an actual exemplary arrangement of the cell population on the microelectrodes, in which the cell population has about 60% cardiomyocytes and about 40% fibroblasts. In fact, such an arrangement increases fluctuations between successive pulses in the conduction velocity between adjacent electrodes. Since the increase in the fluctuation becomes significant particularly by the addition of the agent, generation of spiral/re-entry can be estimated according to the change in the fluctuation width of the conduction velocity between successive pulses. Figure 11(c) is a microscopic picture showing another example of the actual annular arrangement of the cell population on the microelectrode array. For actual measurement of spiral/re-entry, calcium spike firing in each cell of the cell population network can be estimated at the single-cell level by using the high-speed fluorescent measurement camera shown in Figure 7. As a result, actual analysis of the pathway taken by the signal conduction of the cells and actual analysis of the change in the pathways at each round can be realized.

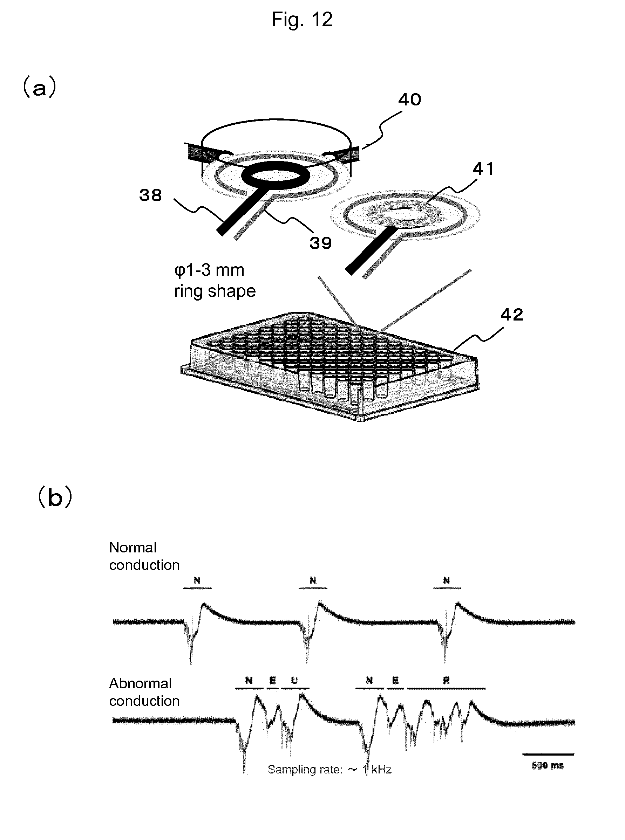

Figure 12(a) is a schematic view showing an exemplary re-entry circuit measurement device using an annular electrode. In this example, an annular electrode 38 with an electrode width of 50-100 micrometers is formed into a ring shape to have a diameter of 1-3mm and arranged on each of the bottom surfaces of a 96-well plate 42. The bottom surface of the plate other than the electrode is coated with a non-cell-adhesive material such as agarose so that the cell population 41 is annularly placed only on the electrode surface. A reference electrode ring 39 is placed concentrically on this non-cell-adhesive coated region, and a flow passage 40 is provided for entrance and exit of a reagent. By using such an electrode, abnormal pulsation of a cardiomyocyte can be simply and conveniently measured. Figure 12(b) is a graph showing normal pulse data and abnormal pulse data actually measured with the electrode. Although an annular electrode is used in this example, a system for optically measuring abnormal pulsation which is equivalently effective as this annular electrode can be constructed by using the optical measurement system shown in Figure 7. In this case, an electric signal to be measured can be acquired by allowing the moving electrode shown in Figure 7 to make contact with the annular cell network.

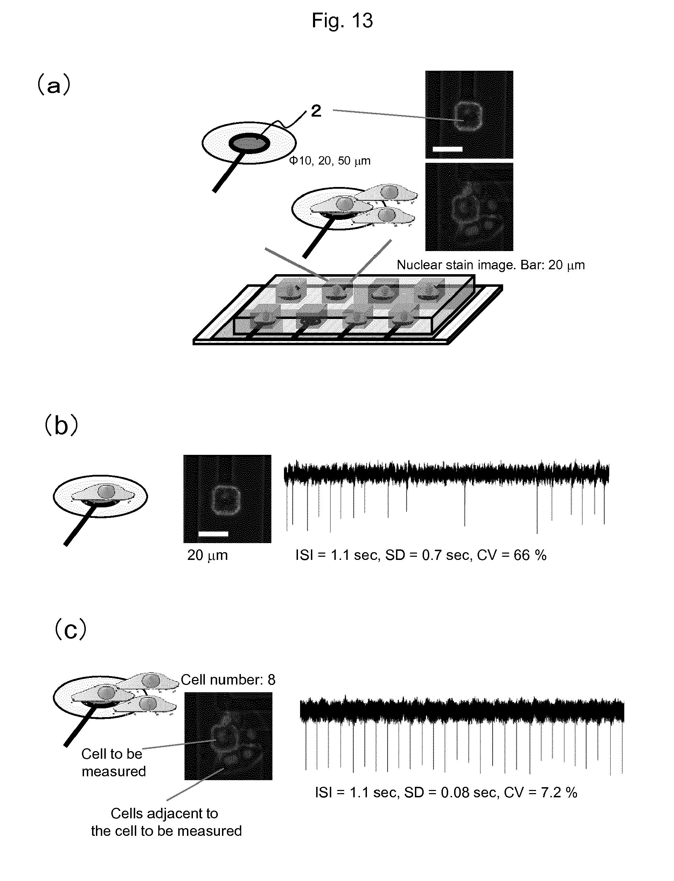

Figure 13(a) is a schematic view showing an exemplary arrangement of a cell and a microelectrode 2 for measuring a potential of a single cell, which illustrates a measurement technique in which a single cell targeted for measurement is arranged on the microelectrode 2 with a diameter of 10 to 50 micrometers. Again in this example, likewise in other examples, the area of the bottom surface other than the electrode is coated with a non-cell-adhesive material such as agarose such that the cell is retained on that place on the electrode. Figure 13(b) is a view of an isolated single cell on the electrode which was actually measured with the microelectrode 2, and electric pulse data thereof. Signals from the isolated single cell are unstable and pulses undergo a large fluctuation as shown in the graph. On the other hand, in Figure 13(c), a single cell is placed on the microelectrode 2 as in Figure 13(b) but to thereby form a cell population with other cells and realize stability of the pulsation cycle as can be appreciated from the pulsation signal graph. In an actual pulse measurement at the single-cell level, the magnitude of the fluctuation between successive pulses serves as an index as shown in Figure 9. Therefore, as described in the present example, a measurement system is useful in that only a specific cell to be measured is placed on the microelectrode while other cardiomyocytes are not provided on the electrode to thereby maintain stability of the specific cell. Accordingly, pulse data of a single cell can be acquired while realizing stability by providing a cell population.

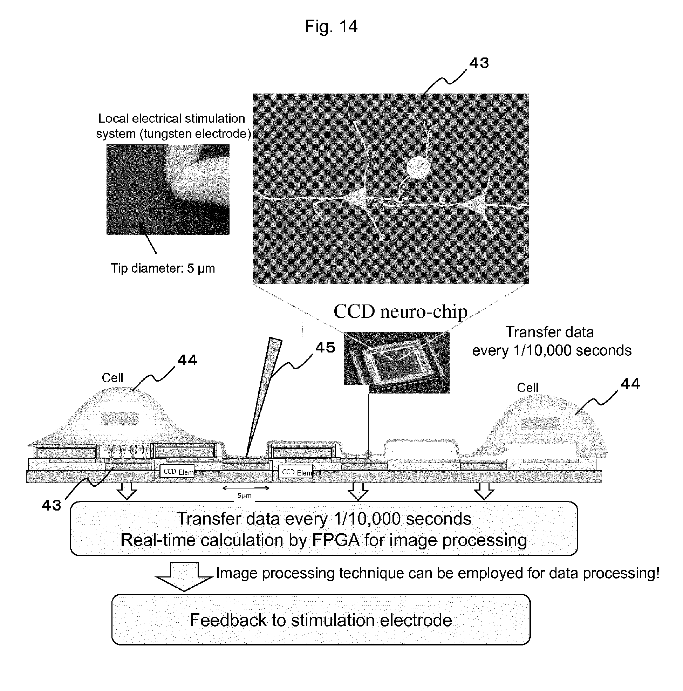

Figure 14 is a schematic view for illustrating an example using a photo-sensitive element of the camera for measuring a potential of a single cell according to the present invention. In general, a photo-sensitive element of the camera converts a light signal into an electric signal on a photoelectric conversion surface to use this electric signal for measurement. This photoelectric conversion surface can be removed and an electric signal array can be used to obtain an electric signal in two dimensions. Therefore, since an electrode array at the single-cell level can be used, for example, a change in the signal conduction pathway in the cell population network with certain spaced intervals as shown in Figure 11, i.e., generation of spiral/re-entry, can be measured, which requires simultaneous measurement of electric signals of respective cells in the cell population. The required interval for pixel measurement in an actual measurement is about 1/10,000 seconds and thus a photo-sensitive element of a high-speed camera with a shutter speed of 1/10,000 seconds is required. In this case, an image processing technique employed in conventional cameras can directly be applied to the acquired signal data of the cells, which allows real-time processing using FPGA for image processing. In addition, feedback stimulation can be applied to the stimulation electrode based on the data obtained by this real-time processing.

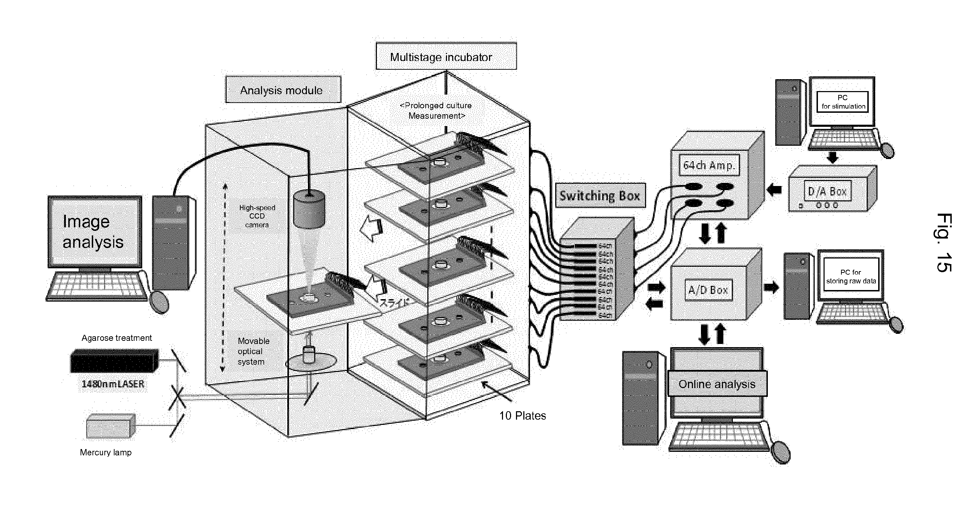

Figure 15 is a schematic view for illustrating an exemplary mechanism for measuring a plurality of samples with a cell measurement system of the present invention. The system of this example comprises an analysis module, a multistage incubator, an electro-analysis module and an online analysis module connected thereto via an online network. Here, the analysis module comprises a phase-contrast microscope or a differential interference microscope for measuring changes in the cellular shape, optical measurement means associated with a fluorescent microscope and a camera photography analysis, and an agarose processing technique that can locally dissolve agarose at a micrometer scale with a microscopic system. Multiple cell culture baths are arranged in the multistage incubator, where microelectrode chips are arranged in the cell culture bath such that measurement of electric signals of each cell and electrical stimulation can be sequentially processed in parallel in the incubator. The obtained electric signals are subjected to real-time measurement in the electro-analysis module, and resulting data are recorded in a storage that is accessible online such that the results of optical measurement data and electric measurement data are recorded with the same time stamp. The analysis module can appropriately access to these record data online for analysis.

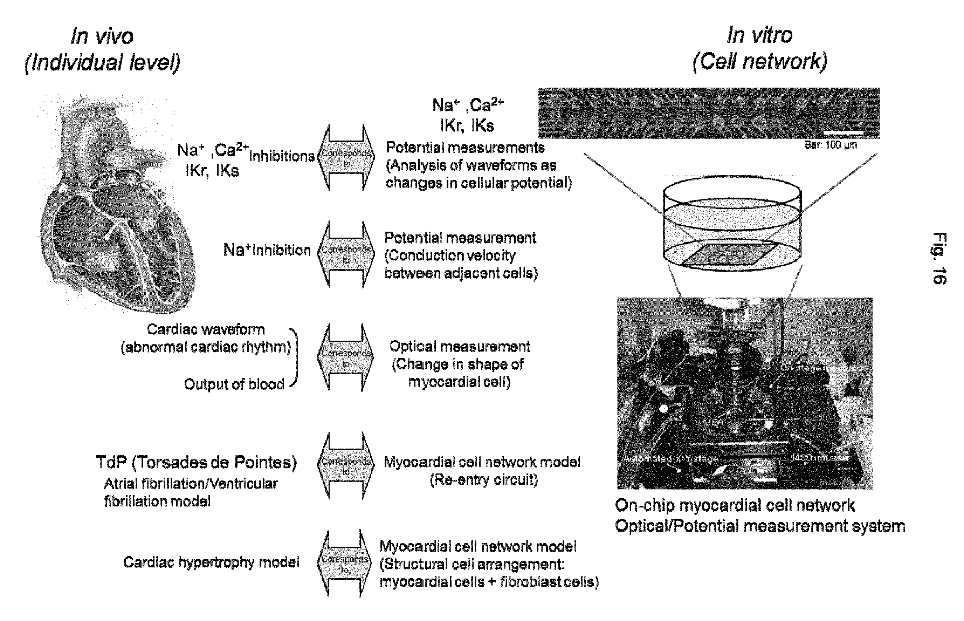

Figure 16 is a schematic view for illustrating information of heart measured with a cell measurement system of the present invention. Electric signal measurement for a single cell on a microelectrode enables measurement of signal data of ion channels such as Na-, Ca-, IKr- and IKs-ion channels and sodium-ion channel inhibition can be measured by measuring the changes in the signal conduction velocities between adjacent cardiomyocytes. In addition, optical measurement of the change in the shape of a single cell allows measurement of the generation of abnormal cardiac rhythm as well as estimation of cardiac output. Furthermore, generation of re-entry can be measured by annularly arranging the cell network. Moreover, measurement as a cardiac pathologic model such as cardiac hypertrophy can be realized by adding fibroblast cells to the cell arrangement.

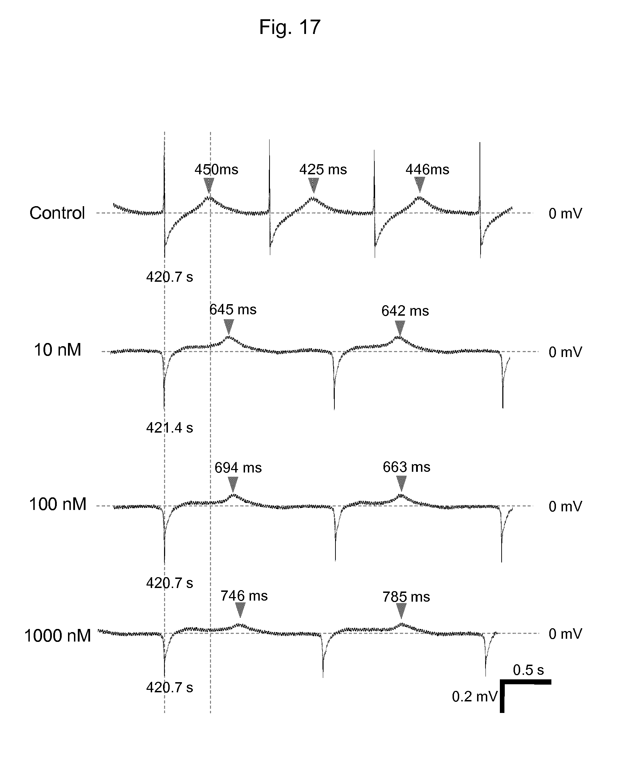

Figure 17 is a graph illustrating an example of changes in the field potential (FP) signal waveform of cells obtained from autonomously pulsating cardiomyocytes in response to the addition of agents in accordance with the cell measurement system of the present invention. The signal waveform of the field potential of the cells shows a change in a membrane potential generated by ions flowing into the cells and ions flowing out of the cells as shown in Figure 8. The signal waveform represents a differential value of the membrane potential, i.e. the sum of an ion current flow per unit time. In this case, the inward ion current, such as sodium or calcium ions or the like in the process leading to depolarization, tends towards negative, and the outward ion current, such as potassium ions in the subsequent process of repolarization, tends towards positive. As shown in Figure 17, information for the FP signal waveform of the cells is usually extracted as one FP waveform as a mean value of a plurality of adjacent waveforms to eliminate the effects of noise components or differences in adjacent waveforms, rather than focusing on the differences between each other for each adjacent pulsation, and detailed analysis of one waveform reflecting the average value is used to estimate the state of each of the ion channels. However, in the present invention, rather than acquiring the average value of the adjacent FP signal waveforms, the adjacent FP signal waveforms are compared and any difference due to the fluctuation of the response of the ion channel is extracted. Based on the size of the fluctuation, the amount of the blocked ion channel is estimated quantitatively. The magnitude of the fluctuations is in general represented by $[1/(n)^{1/2}]$, the reciprocal of the square root of n elements, to facilitate comprehension thereof. That is, given that when $10^4$ channels of the number of ion channels in the cell surface are working, for example, the magnitude of the fluctuation of the function as a sum of the ion channels will be 1% $[1/(10^4)^{1/2}]$, while if the number of the working ion channels decreases to $10^2$ due to blockage by an agent, then the magnitude of the fluctuation increases sharply to 10% $[1/(10^2)^{1/2}]$, resulting in a big change in its feature of the adjacent FP waveform. In other words, if it is possible to estimate the magnitude of the fluctuation by comparing the change in the adjacent FP waveform, then the total amount of ion channels that are blocked can be estimated from the magnitude of the fluctuation.

For the change of the adjacent waveform, focusing on the location of the peak of the outward ion current generated by the release of potassium ions, in particular, taking the time at which sodium ions flow into the cell as a reference (zero), for example, and defining the time from the reference point to the peak of the emission potassium ions as field potential duration (FPD), then the change in the length of the FPD will be the peak value of the inflow of potassium ions subsequent to in- and out- flows of ions such as sodium ions and calcium ions. It can thus be used as an indicator of the amount of change as the sum of the change in in- and outflows of the ions generated by blocking of various ion channels on cells by an agent. In addition, this fluctuation of the position of the FPD reflects the sum of the fluctuations of the adjacent FP waveforms of all the involved ion channels of the cell. In fact, when the position of the FPD (position of the red arrowhead) in Figure 17 is checked, it is seen that the FPD is between 425-450ms prior to the addition of E4031, which is an inhibitor of potassium ion channels, but then became 642-645ms due to the addition of 10nM, 663-694ms due to the addition of 100nM, and 746-785ms due to the addition of 1 $\mu$M E4031. Thus, the value of the FPD increased monotonically due to the addition of the inhibitor. Consequently, adjacent FPDs will not take the same value, but will take a different value to reflect the fluctuation.

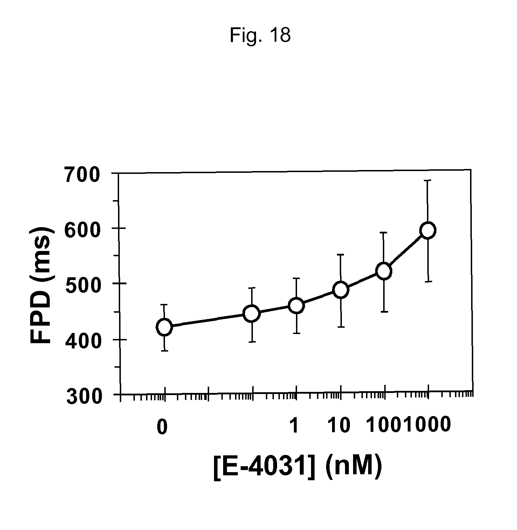

Figure 18 shows an actual example of the experimental results of E4031-concentration-dependency on the prolongation of the FPD when potassium ion channels of the cell were inhibited by an E4031 agent having the ability to specifically inhibit potassium ion channels. Here, it is estimated that the ion outflow is delayed by the inhibition of the potassium ion channels, and the FPD is prolonged in a concentration-dependent manner. Next, measurements of fluctuations in relation to the results of this experiment will be described in the same manner as above.

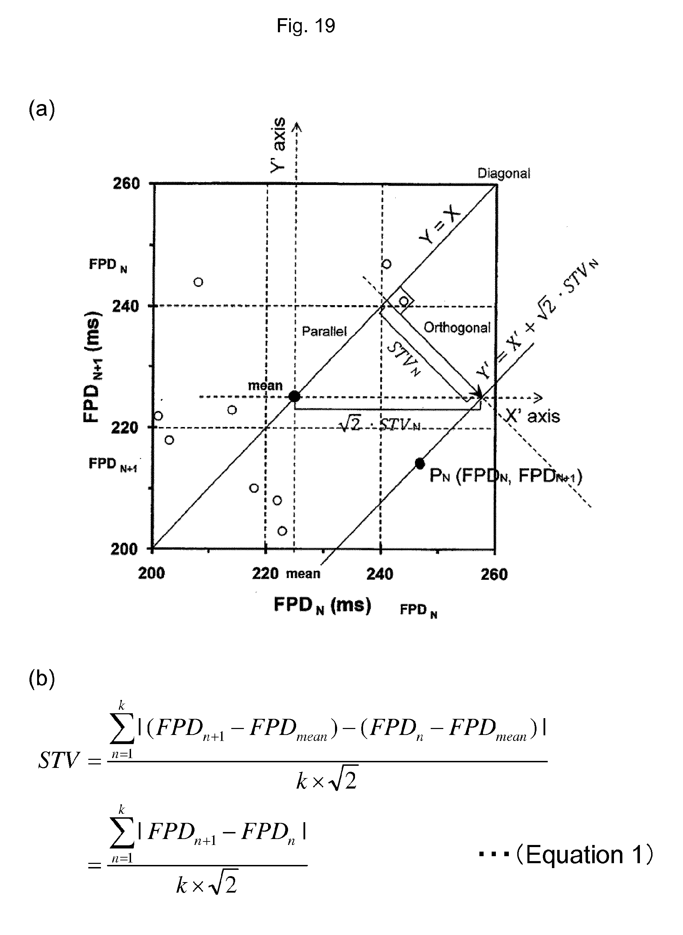

Figure 19 illustrates the estimation of the magnitude of the fluctuation of the adjacent pulsations (short-term variability: STV) among other points of interest in estimating to what extent the FPD of adjacent pulsations shift from a homologous state when the fluctuation of the FPD is observed using the Poincare plotting for measuring the fluctuation of pulsation in the electrocardiogram in general to evaluate the value of the FPD in the FP waveform. In Figure 19 (a), the diagonal, in which X = Y, corresponds to the case where the size of adjacent pulsations $FPD_n$ and $FPD_{n+1}$ have exactly the same FPD size, and the vertical distance of the magnitude of the difference between two FPDs (i.e., $FPD_{n+1} - FPD_n$) from the diagonal is the size of the standardized fluctuation of the adjacent pulsation itself. In particular, for the number of samples "k", it can be evaluated by a formula such as the formula (1) shown in Figure 19 (b).

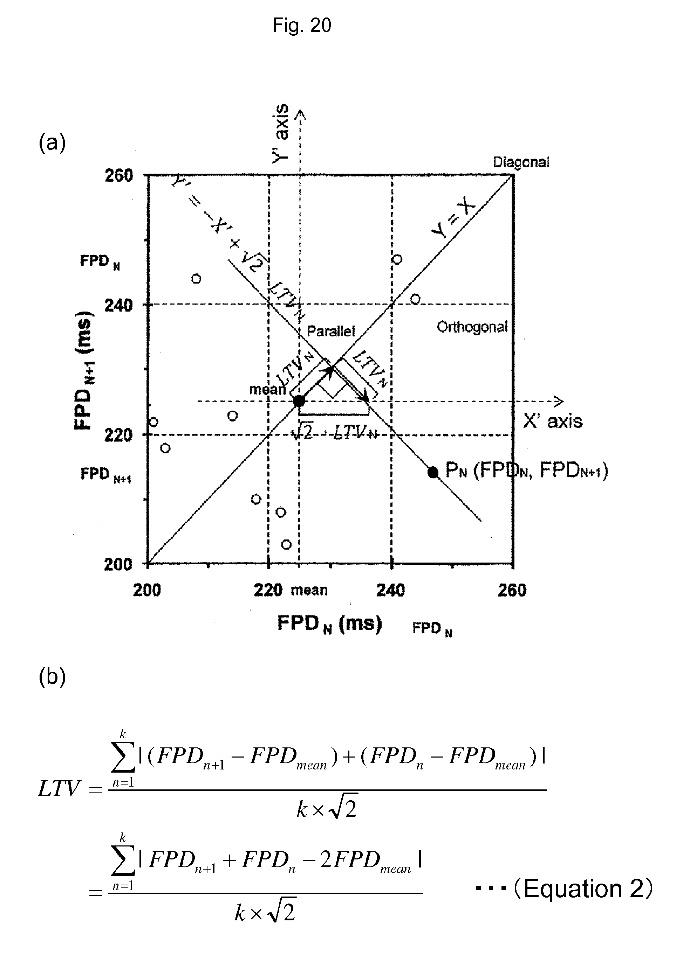

On the other hand, Figure 20 illustrates, among other methods for estimating to what extent the FPD of adjacent pulsations shifted from a homologous state, how to estimate the magnitude of the fluctuation of pulsations (: Long-Term Variability: LTV) in terms of to what extent each adjacent pulsation is shifted from the average value of the pulsations (the sum of all samples and corresponding to the ideal value of the response of the ion channel) when the fluctuation of the FPD is observed using the Poincare plotting. In Figure 20(a), the magnitude of $[(FPD_{n+1} - FPD_{mean}) + (FPD_n - FPD_{mean})]$, which are the two distance values between two FPD values, i.e., adjacent pulsation $FPD_n$ and $FPD_{n+1}$, respectively, and $FPD_{mean}$, the average value of the FPD, which corresponds to the diagonal X = Y, is the magnitude of fluctuation from the mean value of the FPD and the vertical distance from the diagonal which has been normalized. In particular, with respect to the number of samples "k", it can be evaluated by the formula 2 of Figure 20 (b). This shows the deviation from the symmetry of X = -Y, and this size can be used as an index to find out whether or not it is merely a fluctuation of beating near the average value, or whether there is an historical correlation.

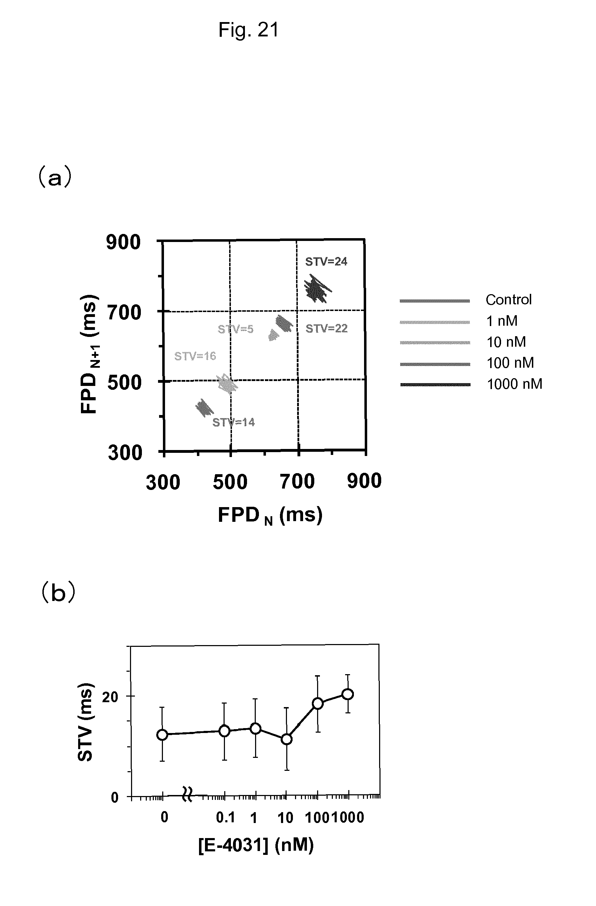

Figure 21 shows, in Poincare plotting, one example of the fluctuation of the FPD of the response of cardiac muscle cells when E4031 was actually added stepwise; and a quantitative summary as STV. It can be seen that it is estimated that ion channels are blocked in response to the addition of E4031 by an prolongation of the length of time of the FPD, while the value of the STV increases rapidly by, in particular, the addition of high concentrations.

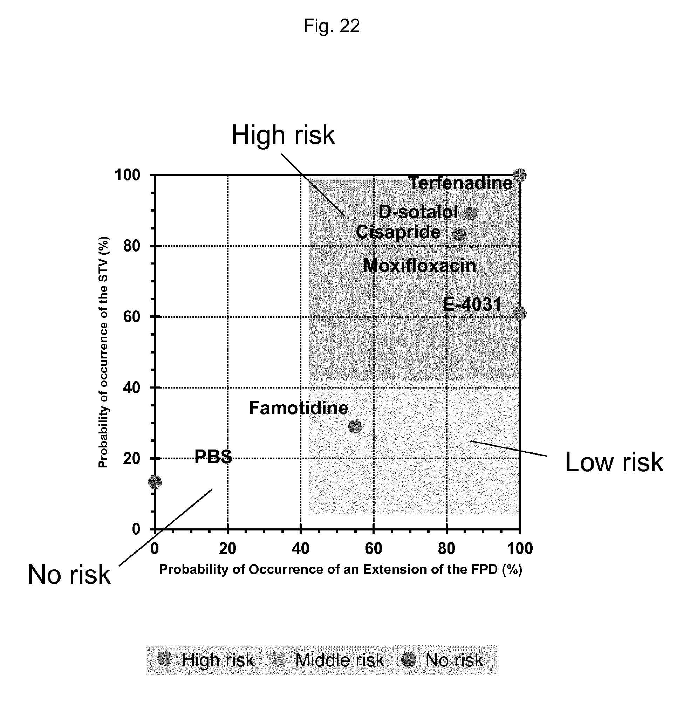

Figure 22 shows an example of an evaluation of chemical agents that are known to have cardiac toxicity and those that are known to have no cardiac toxicity wherein the X-axis is the percentage (%) of observed prolongation of the FPD using the cardiomyocytes, which corresponds to conventional measurement of QT prolongation, and the Y-axis is the percentage (%) of observed increase in the STV. In a conventional agent toxicity test, the evaluation is made only with the results of the data of the FPD on the X-axis. When the evaluation is made with additional results of the STV on the Y-axis, as can be seen from the figure, it is found that there are three areas, i.e., areas for high risk (High risk), low risk (Low risk) and no risk (No risk) of myocardial toxicity in a two-dimensional mapping on a graph, the same distribution as the known result from the relevant literature. From this result, it is found that a more accurate and simplified prediction on the probability of cardiac toxicity of an agent is possible by use of the STV in addition to the conventional FPD.

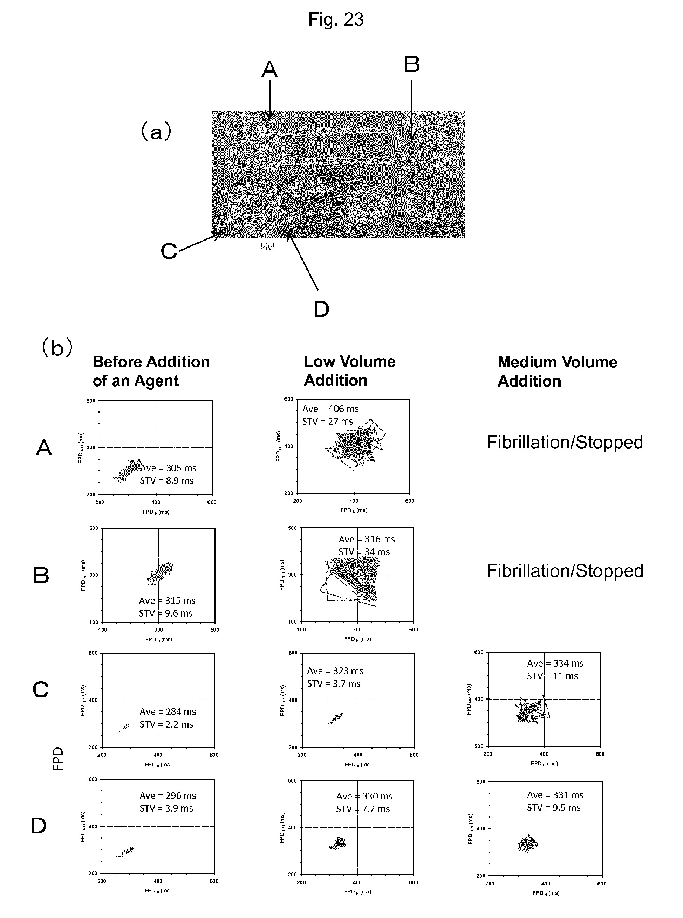

Figure 23 shows the differences in the responses of the STV with regard to the FPD in response to addition of agents. Figure 23 (a) shows an example of a Poincare plotting (A, B) for the FPD for a local portion where a cardiomyocyte-network has been constituted, and (b) a Poincare plotting (C, D) of a local portion of a myocardial sheet having a two-dimensional sheet configuration. In this example, B and D are located near, and A and C are located separated from the pacemaker area PM. In (b), a large fluctuation in FPDs, that were distributed on the diagonal of X = Y of a Poincare plotting prior to the addition of the agent, is observed to occur in both the annular models (A, B) by the addition of low volume of a cardiac toxic agent, and an increase in the STV is observed, while little fluctuation occurs in the two-dimensional sheet model (C, D). In response to addition of an agent in a medium volume, the pulsation changes to a fibrillation state or a stopped state in the annular model (A, B), while an increase in the STV is observed in the area C located away from the PM in the two-dimensional sheet model (C, D). A lower rate of an increase in the STV than the area C is observed in the vicinity of the area D. As can be seen from this example, as for prediction of cardiac toxicity of an agent by measuring the STV of the FPD, it is evident that a population (network) of cells which are arranged linearly from the pacemaker area reflects more accurately the effects of the agent than a sheet-like two-dimensional cell population (network).

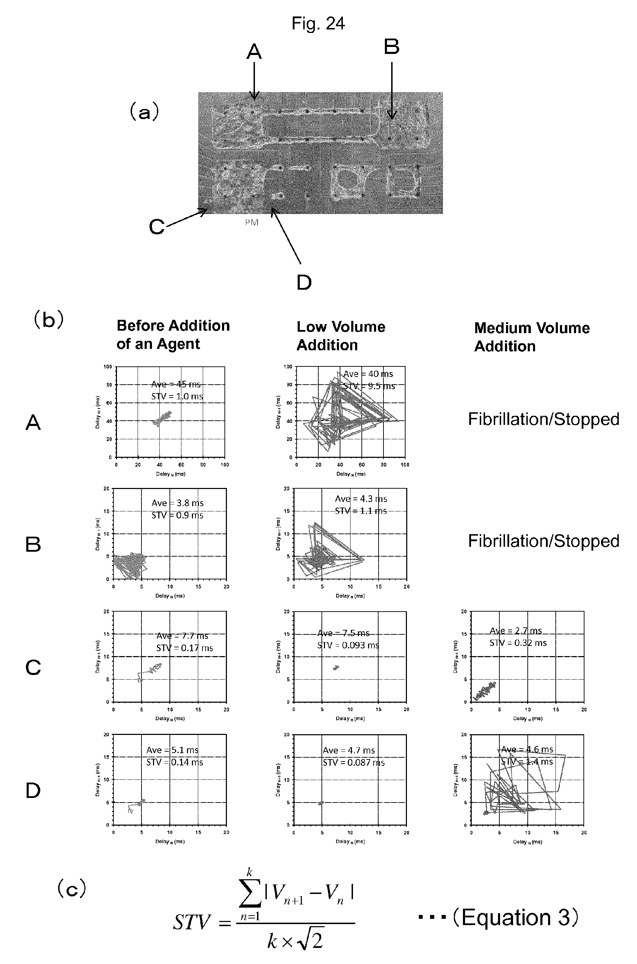

Figure 24 shows the difference in the response of the STV for the transmission speed (V) of pulsatile stimulation from the PM area in response to an addition of an agent. Torsade de Pointes (TdP), which is caused by cardiac toxicity, is a transmission defect in myocardial tissue, and represents a method for estimating the agent toxicity by checking to what extent the fluctuation of the transmission speed from the PM area is actually generated. In this case, as shown in (Equation 3) in Figure 24 (c) in relation to the definition of the STV, the transmission time T from the PM area or (an apparent transmission rate V at the observation point, which is the distance from the PM divided by this transmission time) is used for the measurements instead of the FPD. The definition of LTV is also derived from changing the FPD in terms of T or V in the same way as the STV. As an example of measurement results, a Poincare plotting of transmission time T for a local point in the case of an annularly-constituted cardiomyocyte network is shown in Figure 24 (a) (A, B); and a Poincare plotting for a local point in the case of a two-dimensionally spread myocardial sheet is shown in Figure 24 (b) (C, D). In the same manner as Figure 23, B and D in this example are also located in proximity to, and A and C are located separated from the pacemaker area PM. In Figure 24(b), for the FPDs, which were distributed on the diagonal of X = Y of a Poincare plotting prior to the addition of an agent, a large increase in the fluctuation is observed to occur for both the annular models (A, B) as well as an increase in the STV showing a great fluctuation in transmission time by the addition of a low volume of the cardiac toxic agent, while little fluctuation is observed to occur for the 2-dimensional sheet model (C, D). In response to the addition of a medium volume of the agent, the pulsation changes to a fibrillation state or a stopped state in the annular model (A, B), while an increase in the STV is observed in the area C located away from the PM in the two-dimensional sheet model (C, D). A lower rate of an increase in the STV than the area C is observed in the vicinity of the area D. As can be seen from this example, as for the prediction of cardiac toxicity of an agent by measuring the STV of the transmission time T (or an apparent transmission time at each local point), a population (network) of cells which are arranged linearly from the pacemaker area reflects more accurately the effects of the agent than a sheet-like two-dimensional cell population (network), and at the same time, it can be seen that a generation of the fluctuation, which exhibits a spatial-dependent arrangement, can be measured more effectively.

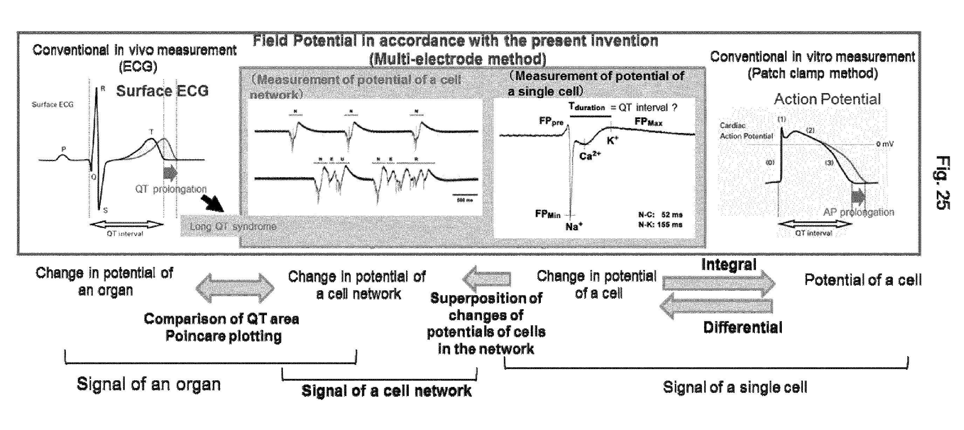

Figure 25 schematically illustrates an electrical FP waveform obtained from a cardiomyocyte in accordance with the cardiomyocyte network of the present invention in relation to a conventional *in vitro* measurement technique (e.g., patch clamp technique) and a conventional *in vivo* measurement technique (e.g., electrocardiogram). The waveform obtained by measuring the FP of the cell in accordance with the present invention indicates the magnitude of ion current per unit time into and out from the cell, which is equivalent to information on changes in the potential of the cell (which is electrically ion current), and which has the differential-and integral-relationships with the electric potential of the cell obtained from conventional *in vitro* measurements on a cell base as depicted in Figure 25. Then, a composite waveform of the FP for the cellular network can be obtained by superposing the FP waveform which is measured for each cell (or a local point of the cell network) and collected from one electrode on each of the FP waveforms collected from a plurality of electrodes that are arranged in a plurality of areas of the cellular network. This data has a homology with the electrocardiogram data of the QT area corresponding to a response of a ventricular tissue portion of the electrocardiogram which is a signal waveform of a potential change obtained from the heart.

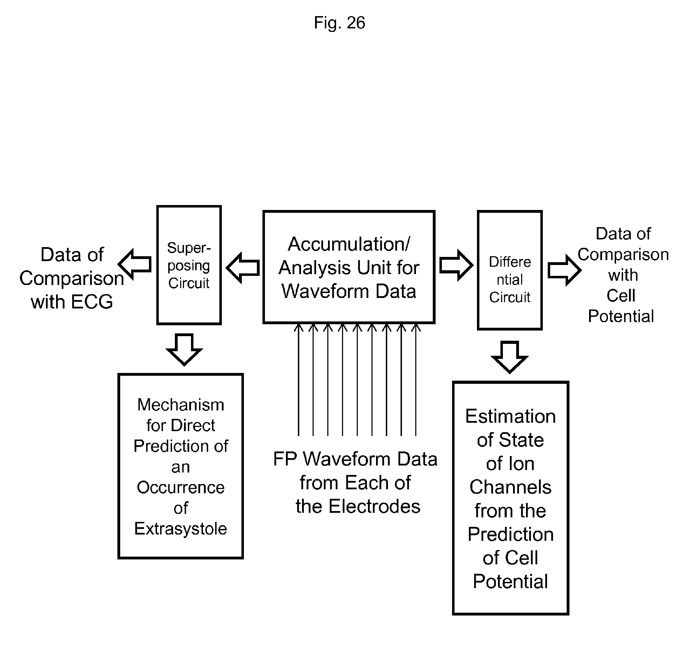

Figure 26 schematically shows a configuration of an apparatus system for estimating a correlation between information measured by the conventional technique as described in Figure 25 above and the FP data obtained with the apparatus of the present invention. The apparatus system is comprised of an arithmetic circuit that has a function to integrate the FP data obtained from each one of the plurality of microelectrodes which are arranged to be able to measure the FP of one cell or a local portion of the cellular network to estimate the membrane potential by differentiating their respective FP data; or an arithmetic circuit that is capable of comparing the data of each electrode with an electrocardiogram waveform of the ventricular portion (Q-T portion) of the electrocardiogram by superposing each electrode data. In particular, in addition to analysis of the FP data of a single electrode that is made possible by using the superposing circuit, it is also possible to makes predictions similar to electrocardiogram analysis using the results obtained by, for example, composing data of an array of a plurality of microelectrodes which are arranged in series and equally spaced on a cellular network so that data reflecting the state of intercellular transmission as well as results of the FP of the cell on each electrode can be displayed; and in particular, by transferring the information of the results of the superposing circuit directly to the prediction mechanism after occurrence of extrasystole for estimating arrhythmia which is an abnormal transmission between cardiomyocytes. This is due to the fact that an abnormality in the transmission is reflected in the waveform of the FP. On the other hand, data of a membrane potential obtained from the differentiation circuit is used to assess the state of ion channels which have different activated states in a membrane potential dependent manner.

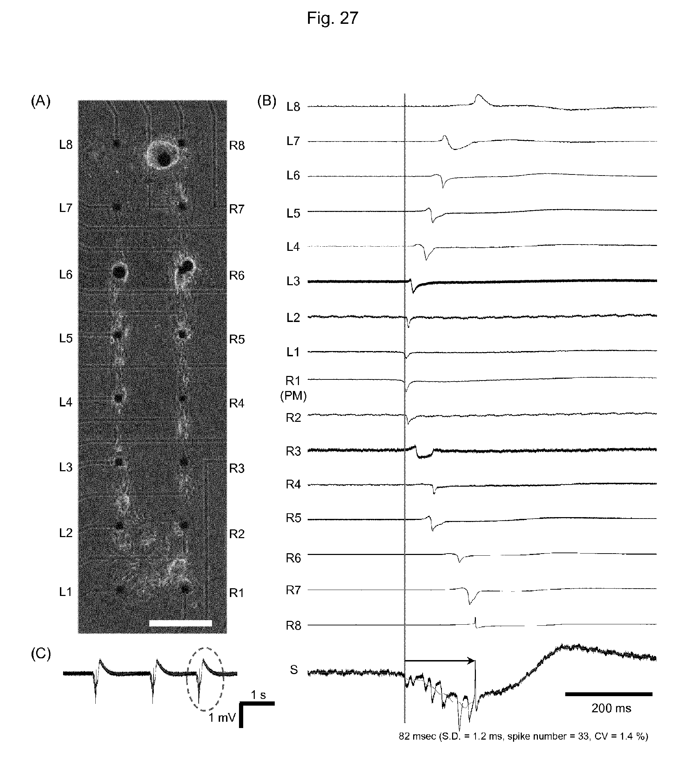

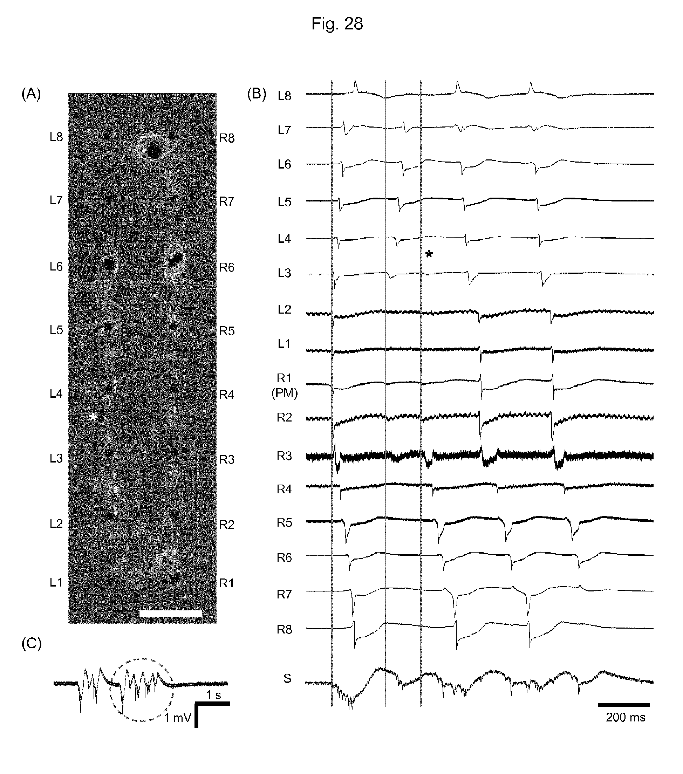

Figure 27 and Figure 28 show an example in which the FP data from each electrode are actually superimposed by an arithmetic circuit as described in Figure 26. In Figure 27, as shown in Figure 27(A), the cardiomyocyte network is annularly arranged; microelectrodes are arranged at regular intervals along the network. In the annular cardiomyocyte network in which the pacemaker (PM) area is located at electrode R1, it can be seen from the FP waveform of each electrode shown in Figure 27(B) that the pulsation signal is transmitted from R2 → R8 or L1 → L8. The S waveform at the bottom is the superimposed waveform. Figure 27 (C) shows the result of a long-term measured composite waveform. This is an actual composite FP waveform which includes information on the FP transmission required for estimating the waveform for the QT area in the electrocardiogram. As can be seen from this figure, when the pulsation signal is transmitted from the area PM in a normal way, the composite waveform is a smooth waveform as can be seen from Figure 27(C). On the other hand, in an arrhythmia state where the pulsation signal from the PM area is no longer transmitted in a normal way, the S, a composite FP, becomes a very disturbed-waveform as can be seen from Figure 28(B). Also in Figure 28(C), which corresponds to the electrocardiogram, the composite FP waveform is a waveform with a similar shape to the waveform for arrhythmia. It should be particularly noted here that when arrhythmia is predicted based only on one electrode data of each microelectrode of Figure 28 (B), it is difficult to predict the occurrence of a significant arrhythmia in some observed electrodes (L5, for example). However, a more accurate prediction is possible when a composite FP waveform is used as can be seen in Figure 28(B) S or Figure 28(C).

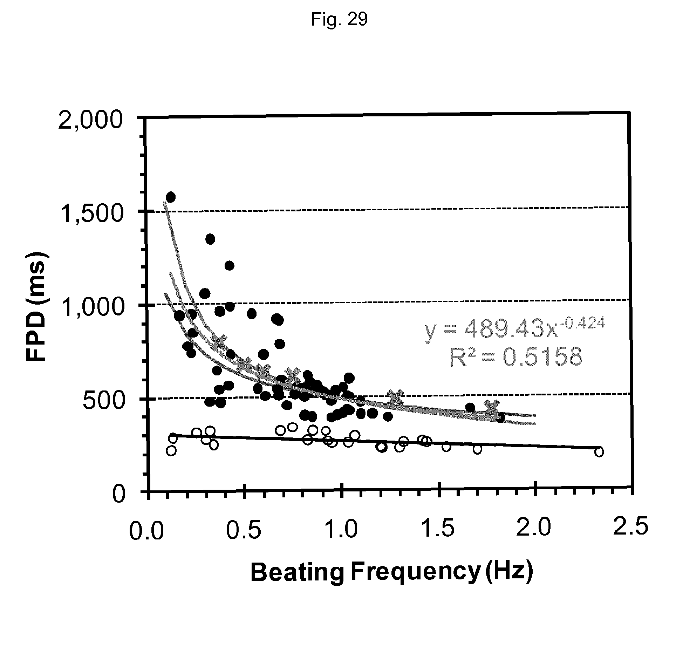

Figure 29 is a graph showing the size of the FPD in relation to the pulsation period of cardiomyocytes. The result of the measurements of the FPD for cardiomyocytes with various autonomous pulsations by the apparatus system of the present invention is indicated in black circles. As can be seen from this result, it can be seen that the cells varied their pulsation period depending on the value of the FPD. This suggests that when the measurement is performed with cardiomyocytes with various autonomous pulsations, side effects such as stopping or destabilization of the pulsation period by an agent raise the possibility that the FPD changes are due to a cause other than natural blocking of ion channels. In addition, the red × mark denotes the value of the FPD when the pulsation period of the cell was forced to change by forced pulsation. It can be seen that the FPD becomes stable by maintaining a certain pulsation period over a certain period of time by continuous external stimulation.

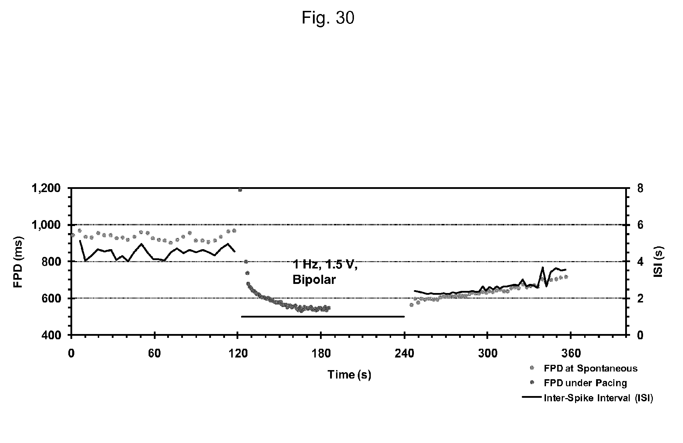

Figure 30 shows an actual example of the change over time of the FPD of cardiomyocytes when external forced pulsatile stimulation is given using the system of the present invention to cardiomyocytes which are autonomously pulsating. It can be seen in this example that initially the autonomous pulsation interval is about 4 seconds, then the value of the FPD significantly changes immediately after the cell was given a forced pulsation stimulus of 1Hz, then the FPD is stabilized at the position of 550ms at approximately 30 seconds after the start of stimulation. It can also be seen that even after the forced pulsatile stimulation, the autonomous pulsation period varies, and the FPD steadily increases. As can be seen from these results, it is desirable to test the agent toxicity after 30 seconds from the start of forced pulsatile stimulation where the FPD is stable.

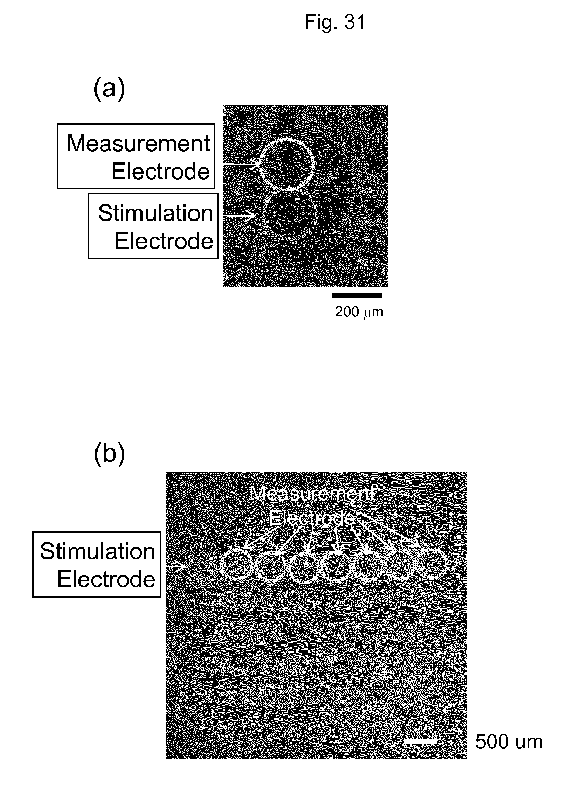

Figure 31 shows an actual example of the arrangement of cells when measuring the FPD or transmission time T or transmission velocity V while giving external forced pulsatile stimulation using the system of the present invention. Figure 31 (a) is an example of measurement of stimulation with cell populations that are disposed to cover at least two microelectrodes. While providing forced stimulation signals at a fixed interval of 60 beats per minute from the stimulating electrode, for example, the FPD of the cells at the adjacent measurement electrode, or the transmission time T or the transmission speed V from the time of stimulation at the stimulating electrode to the cells on the measuring electrode are measured. Figure 31 (b) shows an example in which forced pulsatile stimulation is given by a stimulation microelectrode disposed at the end point of the network of cardiomyocytes which are arranged in a straight line, the FPD, T and V of cardiomyocytes on each electrode are measured for the transmission by a microelectrode array disposed along the network of the cardiomyocytes at regular intervals, and for example, prediction of the occurrence of arrhythmia by a composite FP of the FPs of each recording electrode, the relationship between T and V of each electrode as well as the data of each of the electrodes to the stimulation signals of the stimulating electrode can be estimated. However, what is shown here is only an example of the arrangement of the cells. It is also possible to make similar measurements by providing forced pulsations in the PM area of the annular cellular network shown in Figure 27, or alternatively, it is also possible to make measurements of the FPD on the minimum number of cells using the stimulating electrode, on which the cells are placed, as a measurement electrode.

For all examples so far, the cardiomyocyte network is described only for cardiomyocytes. However, it is intended to include embodiments where fibroblasts are added to have properties similar to biological tissues.

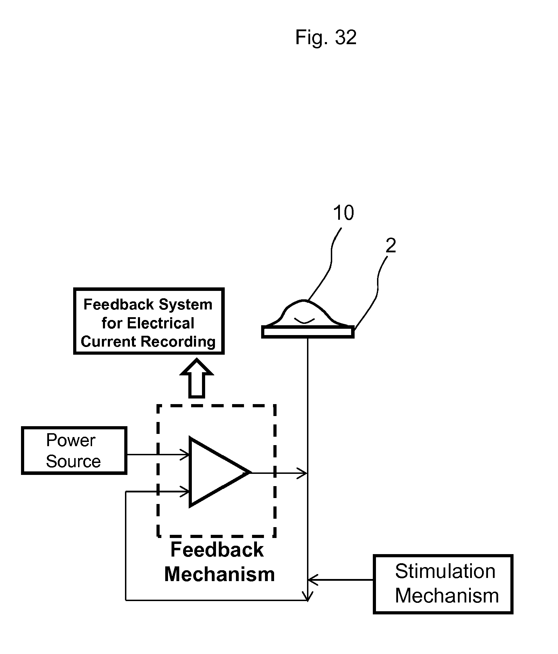

Figure 32 shows a potential clamp-type feedback control mechanism to maintain a constant voltage of microelectrode 2 and make measurements for the FP of cells disposed on the microelectrode 2. Here, the FP of cells is estimated by analyzing the result in real time by monitoring the current supplied from the external power supply to maintain the potential of the electrode 2 instead of measuring the amplified signal from the electrodes in the conventional configuration. It shows an example of the change over time of the FPD of the cardiomyocytes. As the potential is to be kept constant, in this context, it is normally chosen to take a value of zero; however, in the case that the state of cells is changed, for example, by changing the potential of depolarization, it is also possible to adjust to those different potentials.

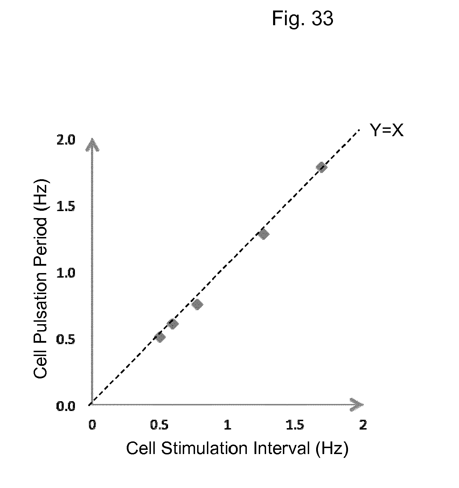

Figure 33 is a graph of an example of the results of actually measuring the change in the period of pulsation of the cell population when forced pulsatile stimulation is provided using the system of the present invention described above in a partial area of the cell population which has differentiated from human ES cells into cardiomyocytes. As can be seen from this graph, it is found that for the normal population of cardiomyocytes, when the forced stimulation of 0.6Hz to 1.8Hz e.g., is given as in this example, the pulsation follows linearly in response to the forced stimulation in all of this range.

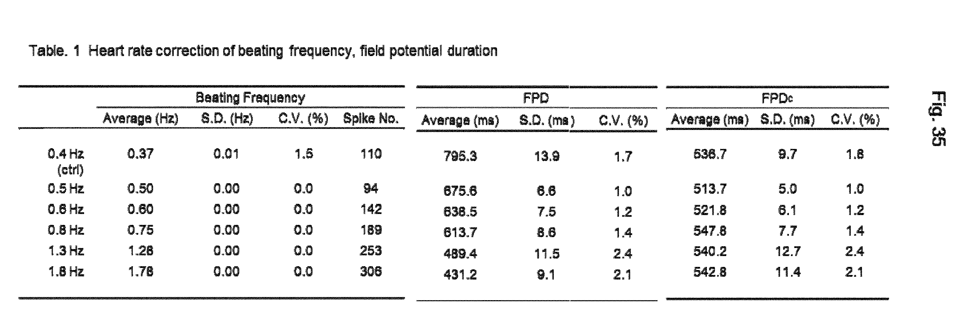

Figure 34 (a) shows changes in the waveform of the FP and in the length of the FPD of the cardiomyocyte population under forced pulsatile stimulation where the pulsation period of the cell population is the same as the interval of the forced pulsatile stimulation when forced pulsatile stimulation is actually provided. As can be seen from the graph, the FP waveform changes and the length of the FPD shortens by shortening the interval of the forced pulsatile stimulation. As shown in Figure 34(b), a graph of the change of the FPD indicates that this shortening depends on the cycle of the forced pulsation interval (RR). According to a known study of the relationship between a heart rate and the length of the QT interval in a human heart [Patrick Davey, How to correct the QT interval for the effects of heart rate in clinical studies. Journal of Pharmacological and Toxicological Methods 48 (2002) 3- 9], a Fredericia correction with respect to this compensation relationship, i.e., in order to make a correction to the length of QT ($QT_c$) during cardiopulsation at a pulsation period of 60 beats per minute, mainly depends on whether or not it conforms with the converted value of $QT_c = QT/(RR)^{1/3}$, or with the converted value of $QT_c = QT/(RR)^{1/2}$ which has been proposed by Bazett due to the fact that it is not possible to make a relative comparison because of change in the length of the QT due to the difference in the pulsation rate. As described above, the length of QT *in vivo* corresponds to the superposition of the length of the FPD measured across the cellular network measured by the present system. That is, it suggests that the FPD itself of each cell should enter into the range of the correction of Fredericia or Bazett. In fact, however, the result of Figure 34(b) indicates that $QT_c = QT/(RR)^{1/2.5}$, showing that it lies between the correction of Fredericia and the correction of Bazett. In addition, Figure 35 is a table summarizing the data shown in the graphs of Figure 33 and Figure 34.

These results indicate that evaluation of the quality of cardiomyocytes to be actually used for screening or in regenerative medicine can be addressed by measuring the response of the cardiomyocytes when forced pulsatile stimulation is given to the cardiomyocytes. In other words, the following procedures are noted:

1) providing forced pulsatile stimulation to a cardiomyocyte or a cardiomyocyte population; evaluating as to whether the cell or the population of the cells respond to the forced pulsatile stimulation and respond at the same interval as the forced pulsatile stimulation; verifying what frequency range of the response of the cells to the forced pulsation signal; and determining that one of the sufficient conditions for a healthy cardiomyocyte is met when it is demonstrated that the pulsation follows the stimulation; more specifically, determining that one of the sufficient conditions for a healthy cardiomyocyte is met when it is demonstrated that the pulsation follows the stimulation up to at least 1.8Hz, for example.

2) determining that one of the sufficient conditions for a healthy cardiomyocyte is met when it is verified that the change in the FPD in response to the forced pulsatile stimulation is between $FPD/(RR)^{1/3}$ and $FPD/(RR)^{1/2}$ within a range of the frequency at which the follow-up of the pulsation of the cells in response to the forced pulsatile stimulation interval (RR) has been confirmed.

By using the above procedures, quality control of cardiomyocytes can be achieved. A healthy cardiomyocyte is a cell that is capable of making a stable pulsation. Here, the cell population that underwent differentiation induction may be used as the cell population to be evaluated, or the cardiomyocytes that underwent a differentiation induction may be dispersed for measurement and evaluation on a single cell basis, or the dispersed cardiomyocytes may be collected and used as a cell population for measurement, or alternatively, the dispersed cardiomyocytes may be mixed with fibroblasts derived from a human heart and used as a new cellular population for the measurement and the evaluation. These cardiomyocytes can be used for the myocardial toxicity test.

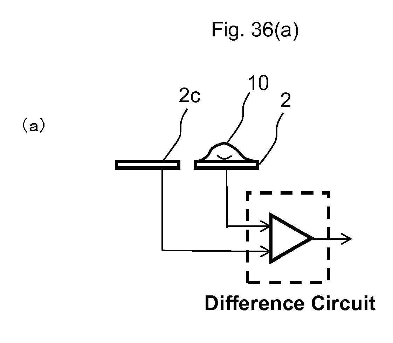

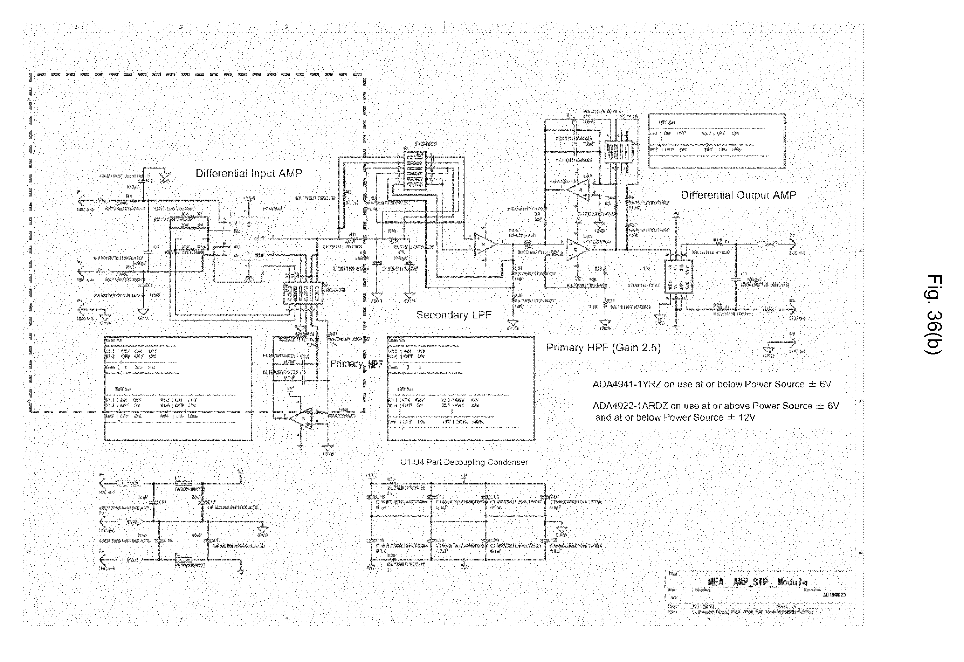

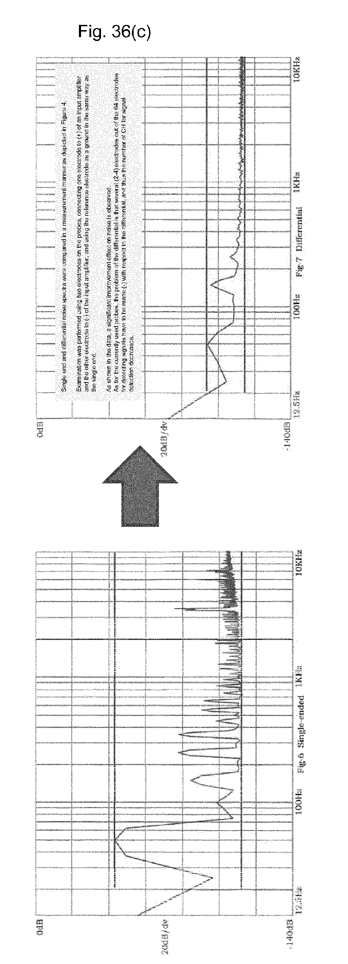

Figure 36 (a) schematically shows a circuit for outputting a value of the difference in electric potential between a microelectrode 2 on which a cell 10 is disposed and a comparison electrode 2c, which is in the vicinity of the microelectrode 2, and on which no cell is disposed, for use in electrically reducing noise in cell signals. In fact, as shown in Figure 36(b), by incorporating this circuit in the first stage of the amplifier circuit, it is found that the noise reduction does not depend on a specific frequency, as shown in Figure 36(c). The position of the reference electrode 2c is preferably in the vicinity of the microelectrode. For example, it is fully functional if it is located at a distance of $50\mu m$, and it can function to reduce noise if it is within a distance of 1mm.

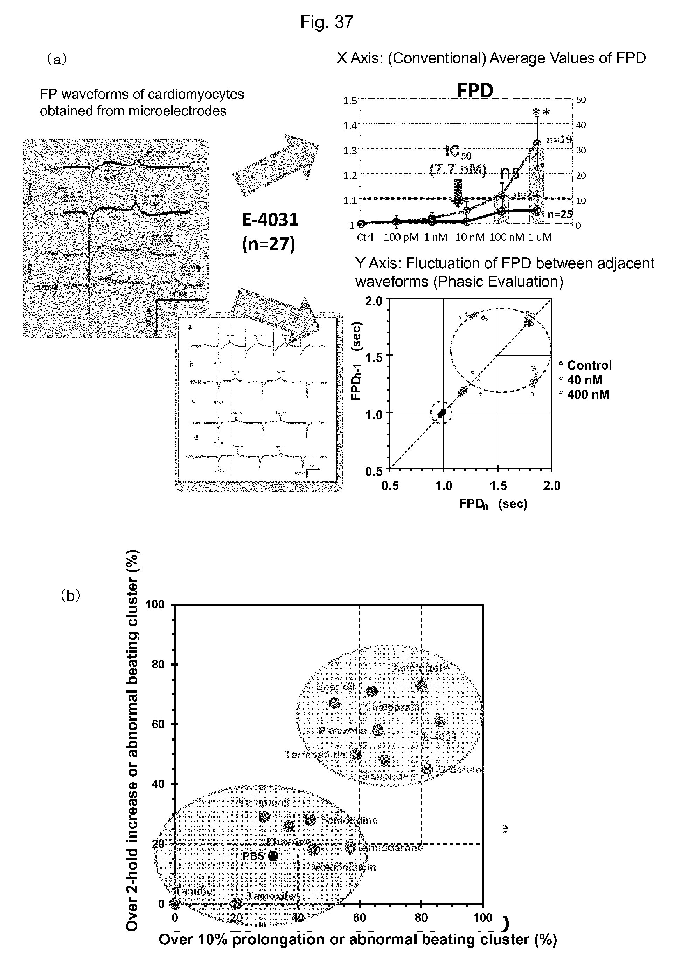

Figure 37 is a diagram schematically showing an example of a comprehensive myocardial toxicity evaluation method of the present invention. Regarding the values for the FPD obtained from the results of measurements of membrane potential of cardiomyocytes after addition of an agent of a particular concentration, the results are plotted taking a level of the FPD prolongation as a value on the X axis and taking STV, which is derived from Poincare plotting of the magnitude of the fluctuations with time of the FPD described above, as a value on the Y axis. Figure 37 (b) is one example of the results plotted in an X-Y diagram for a variety of agents. As can be seen from the figure, an agent in the area where the increase in the prolongation of the FPD and the fluctuation (STV) is decreased can be determined as having a QT prolongation but no cardiac toxicity, while cardiac toxicity such as TdP can be predicted when prolongation of the FPD and the fluctuation of (STV) occur simultaneously (upper right in the X-Y diagram).

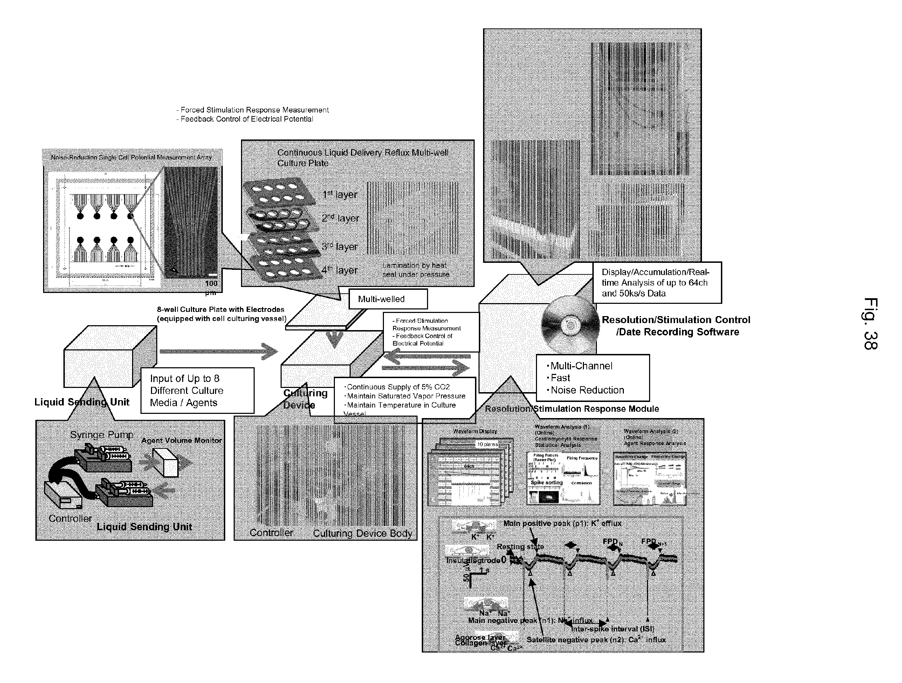

Figure 38 is a schematic diagram showing an example of the configuration of a system for measuring the actual cardiac toxicity. The system of this embodiment includes a liquid sending unit, a cell culture measurement unit, and a cell analysis/stimulation unit.

The liquid sending unit can send liquid by a syringe pump system or a peristaltic pump system or a HPLC pump system by which the culture solution is continuously fed to each of the cell culture chambers in which cells are cultured in the measurement unit. In addition, a resistive heating wire for temperature control is wound around the outer circumference of the pipe of for sending liquid, and a solution is always introduced at a constant temperature by monitoring the temperature of the liquid in the tube continuously with a detecting mechanism of heat such as a micro-thermocouple type K or a thermistor, and adjusting the temperature of the liquid to be introduced in terms of the degree of resistance heating for controlling solution temperature. In addition, the liquid sending unit includes piping in which mechanisms such as junction pipes and switching pipes are arranged for addition of agents to be tested, and through which desired concentrations of agents can be introduced into each of the cell culture chambers. Further, the quantitative determination of the concentration of the agent solution desirably includes addition of a mechanism in which a portion of an inlet pipe of the liquid is optically transparent, and by which quantitative evaluation can be made by spectrophotometric measuring in the range of a wavelength of 280nm - 800nm. Likewise, it is also desirable that a mechanism for waste liquid is added in which a part of the waste tube is optically transparent, and by which quantitative evaluation is possible by measurement of spectroscopy absorption in the range of a wavelength of 280nm to 800nm. The controlled temperature of the agent solution preferably approximates a normal temperature of a human body, and from this point of view, it is desirable to be able to control the temperature in the range of 30 degrees to 45 degrees centigrade.

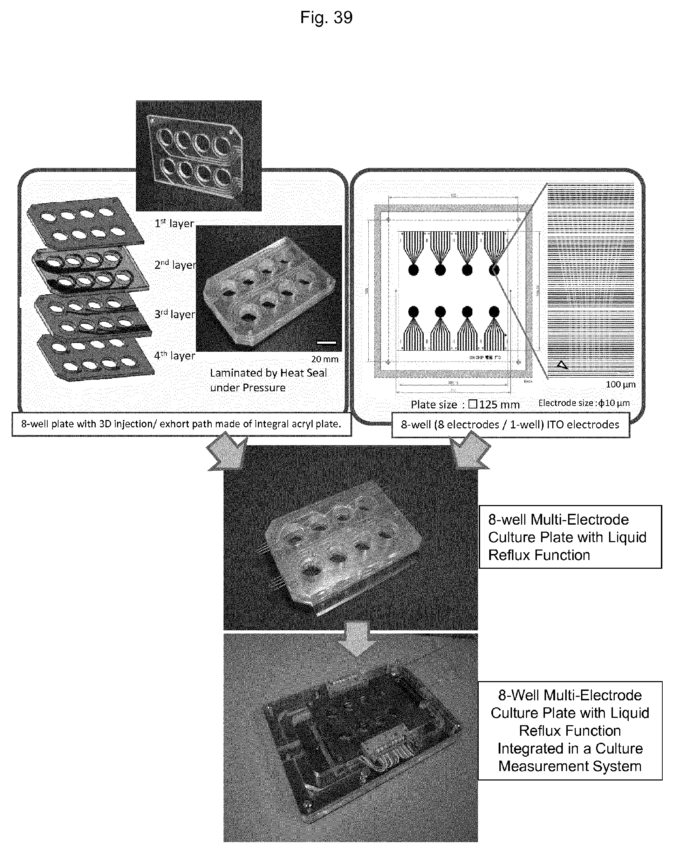

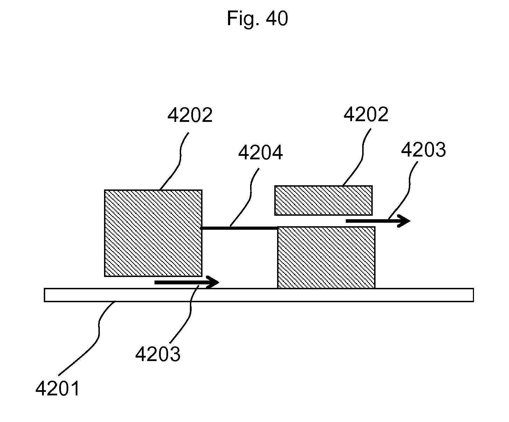

Figure 39 shows schematic diagrams and photographs of an example of a configuration of a measurement chamber of a cell culture system for measuring cardiac toxicity of the present invention. The cell culture vessel 4202 on which an introducing mechanism and a draining mechanism of the culture liquid are arranged has been adhered to the multi-electrode substrate 4201 on which a plurality of membrane potential measurement electrodes is arranged (see Figure 40), forming a cell-culture-measurement plate capable of measuring 8 samples at the same time. As shown in Figure 40 in which a cross-sectional view of a cell culture measurement plate is schematically shown, in the cell culture vessel 4202, the inlet of the solution is arranged in a fan-shaped form spread in the bottom surface closest to the multi-electrode substrate 4201, while the liquid draining mechanism has been deployed in a fan shape in the same direction as the direction of the interface of the liquid surface at a position that determines the height of the liquid surface 4204 at the top.

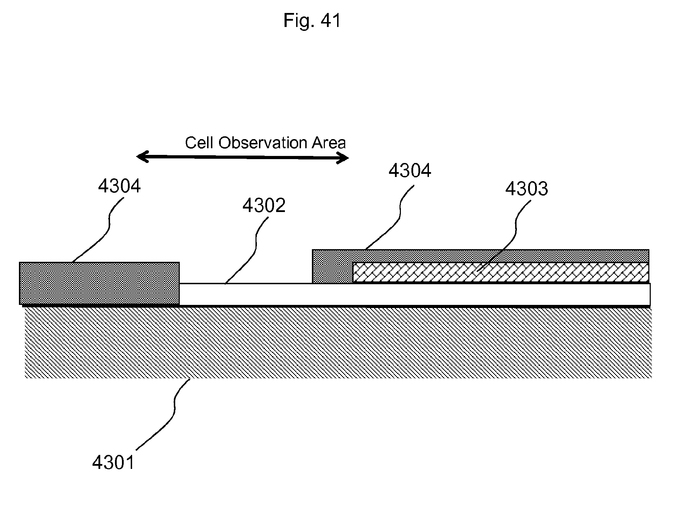

Figure 41 is a diagram schematically illustrating a configuration of electrode wires of the electrode arrangement arranged in a multi-electrode substrate. In the present invention, in order to observe the shape of the cell, transparent electrodes such as ITO electrodes are used. However, an increase in the length of the wiring will result in a high resistance compared to normal metal electrodes due to their characteristics as a transparent electrode, and as a result the impedance becomes very large especially for a large plate such as the multi-electrode substrate. In order to avoid this problem, a metal layer may be disposed in the same arrangement as the transparent electrode to reduce the resistance value owing to the conductivity of the metal electrode. In fact, in the area for culturing the cells, a wiring using a transparent electrode 4302 on a glass substrate 4301 is disposed in order to perform an optical observation, while in an area not used for observing cells, a metal layer 4303 is disposed thereon to overlap the transparent electrode and the upper surface of which is coated with an insulating film. The metal electrode materials as used herein may be, for example, gold, platinum, titanium, copper, aluminum, and the like.

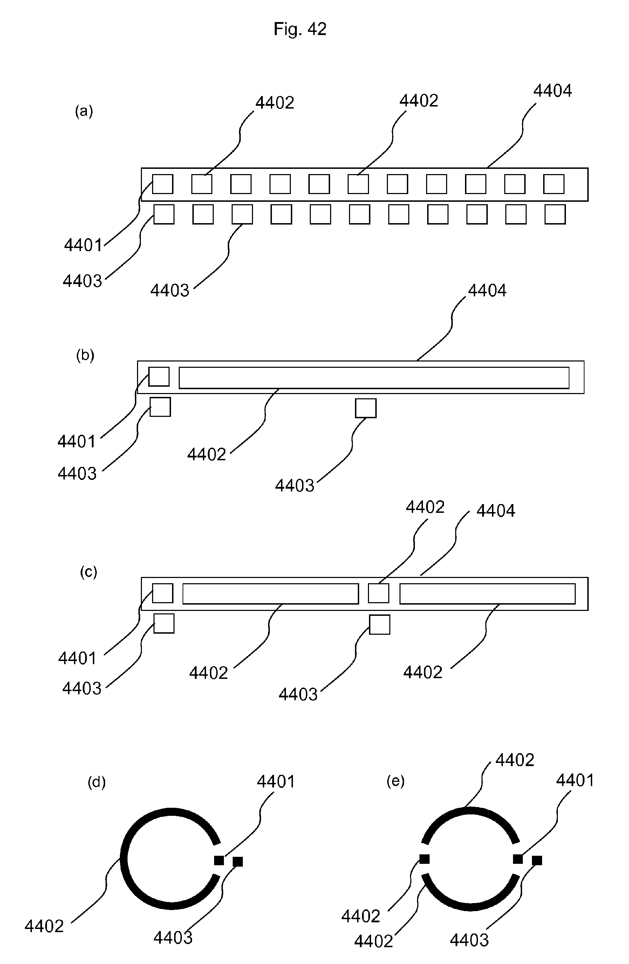

Figure 42 is a schematic diagram showing an example of electrode arrangement disposed on a multi-electrode substrate. First, in Figure 42(a), there are arranged a stimulating electrode 4401 to locally stimulate the end of the myocardial cardiomyocyte network arranged in series in a cell culture area 4404, measuring electrodes 4402 for measuring the excitation conduction of cardiomyocytes stimulated by stimulation electrodes and a reference electrode 4403 for noise reduction. It is possible to measure the results of a plurality of local responses of a cardiomyocyte network obtained from each of the measurement electrodes 4402, and a fluctuation of the transmission rate can be determined by a comparative analysis of the degree of the transmission rate between the measurement electrodes. In Figure 42 (b), there is shown a configuration where the measurement electrodes are connected in a straight line. By this configuration, it is possible to measure the waveform similar to the waveform of an electrocardiogram of the ST area (ventricular area) of the electrocardiogram. In Figure 42 (c), there is shown a configuration that facilitates acquisition of the FP waveform of a local point of cardiomyocytes by separating a part of Figure 42(b). Figure 42 (d) is an example of an electrode arrangement for measuring cells arranged in a ring form on a ring-shaped electrode. Although these are intended to measure the cardiomyocyte network arranged in a ring shape as shown in Figure 11 and Figure 12, they differ in that a part of the ring-shaped measurement electrode is cut out, and a stimulating electrode for providing local forced stimulations is arranged therein, and a reference electrode is arranged for noise reduction. Further, in Figure 42 (e), there is shown a configuration in which the measurement electrodes are split and it is possible to measure responses of a local portion of the cardiomyocytes.

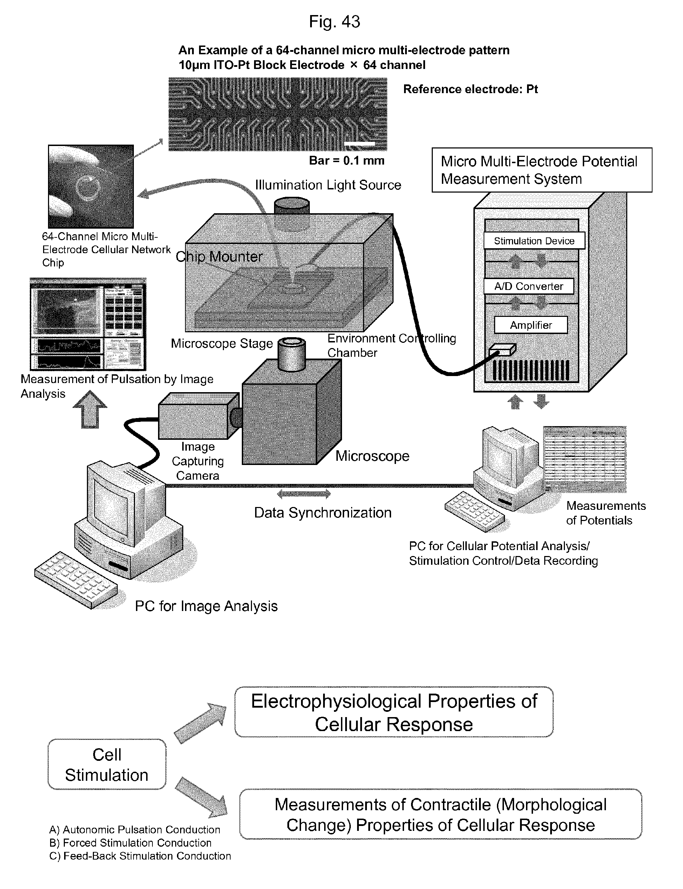

Figure 43 is a schematic diagram showing an example of a system configuration of the present invention to simultaneously measure the mechanical properties and electrical properties of cardiomyocytes. The present system includes (1) a cellular network chip that can be used for culturing a cell population, includes a plurality of micro-electrodes disposed on a substrate and can be used for acquiring cellular potential data of a small area of the population; (2) a chip mounter for mounting the chip and joining electrically with cell-stimulating and/or cellular potential measuring system; (3) an environmental control vessel which can be used to control the environment such as temperature, humidity, oxygen concentration and carbon dioxide concentration of the cell population being cultured; (4) a micro multi-electrode potential measurement system that can give stimulation to a specific cell of a cardiomyocyte population, and allows simultaneous continuous measurements of cellular potentials of various small areas in the cell population; (5) one or more position-coordinate probe microparticles configured to measure changes in shape of myocardial cells and allow easy identification of cardiomyocytes using a plastic, polymer, glass or metal microparticles such as polystyrene microparticles, glass microparticles or gold microparticles of sizes from at minimum about 0.1, 0.2$\mu$m, 0.3$\mu$m, 0.4$\mu$m, 0.5$\mu$m, 0.6$\mu$m, 0.7$\mu$m, or preferably about 0.8$\mu$m, more preferably about 0.9$\mu$m, or most preferably about 1$\mu$m, to at most about 500$\mu$m, 400$\mu$m, 300$\mu$m, preferably about 200$\mu$m, more preferably about 100$\mu$m or most preferably about 50$\mu$m, wherein the particles are disposed in the cell population by mixing them on the surface of the cardiomyocyte population in the cell network chip or in the cell population and culturing them; (6) an optical image capturing system for optically measuring the microparticles, wherein the system includes a light source, an optical microscope and an image-capturing camera to capture the images of the microparticles; and (7) a computer system for image analysis, cellular potential analysis, stimulus control and/or integrated data acquisition, wherein the system is capable of measurements of cellular potential and analysis of waveforms of the potential, measurements of cell displacements by image analysis and feedback stimulation based on the analysis results. In the measurements using the device system, as for the stimulus to cause depolarization of the myocardial cells, the stimulus can be provided by selecting mainly from three means, i.e., (A) measurements using conduction of autonomous pulsation of the cardiomyocyte population, (B) measurements of conduction by providing a forced electrical stimulation from outside to a specific cell in the cardiomyocyte population and (C) measurements of conduction by providing a feed-back stimulus at a specific timing to meet the relationship of the delay time and the value of the cellular potential based on the cell voltage measured data to the specific cells in the cardiomyocyte population.

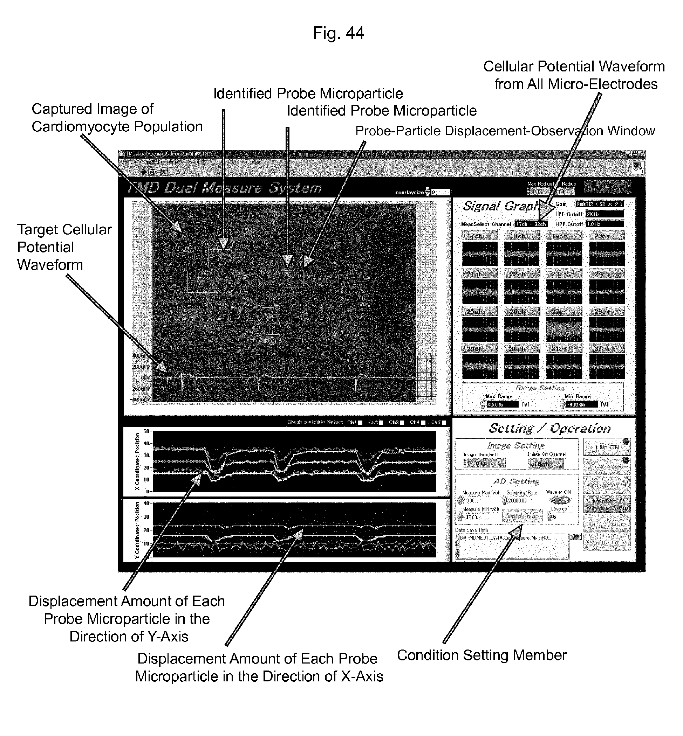

Figure 44 is an example of a data acquisition monitor screen showing an example of data obtained from an example of a system configuration of the present invention to simultaneously measure the mechanical properties and the electrical properties of cardiomyocytes. In a setting of this example, a large number of polystyrene microparticles are placed on the surface of the cardiomyocyte population, a probe-particle displacement-observation window for five of the polystyrene microparticles selected as probes is set, and the center of gravity of the window moves with the movement of the microparticles in the window, thereby displacement of the particular probe microparticles can be measured continuously as a change in vector time in the directions of the X-axis and Y-axis. Further, by measuring the cellular potential data of a particular target cardiomyocyte at a position being at the optical measurement, any change in conduction stimuli response of Na ion channels, Ca ion channels and K ion channels can be measured in correlation with changes in cell shape. In particular, the capability of simultaneous measurements of displacements of a plurality of probe microparticles and measurements of changes in the displacement direction in addition to the change in the displacement amount makes it possible to estimate quantitatively whether the changes in the contractile strength in response to addition of a drug, which occurs due to the variation of the response characteristics of the myocardial cells in the cell population, occur uniformly or non-uniformly.

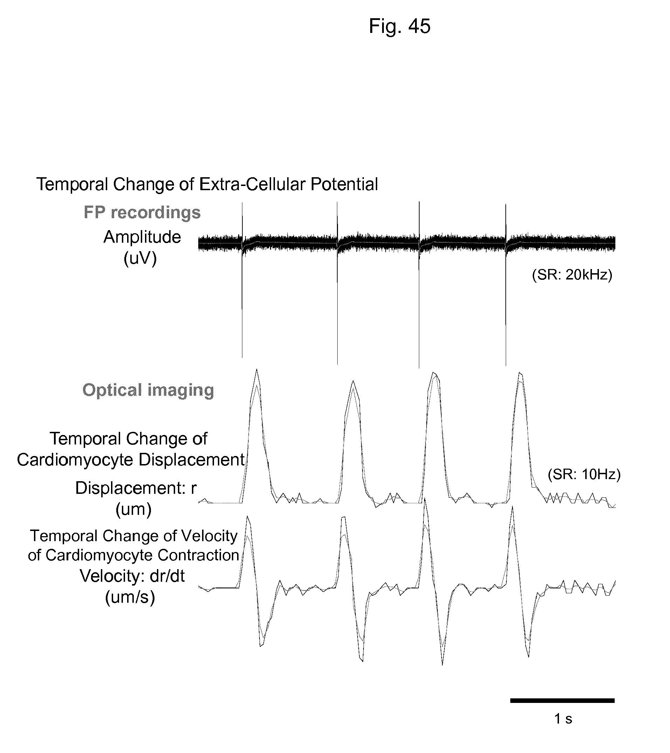

Figure 45 is an example of data obtained from an example of a system configuration of the present invention to simultaneously measure the mechanical properties and electrical properties of cardiomyocytes. As can be seen from the figure, in addition to the cellular potential data, at the same time, the amount of displacement of the cardiomyocytes and a displacement velocity data obtained by time differentiating the amount of displacement can be obtained.

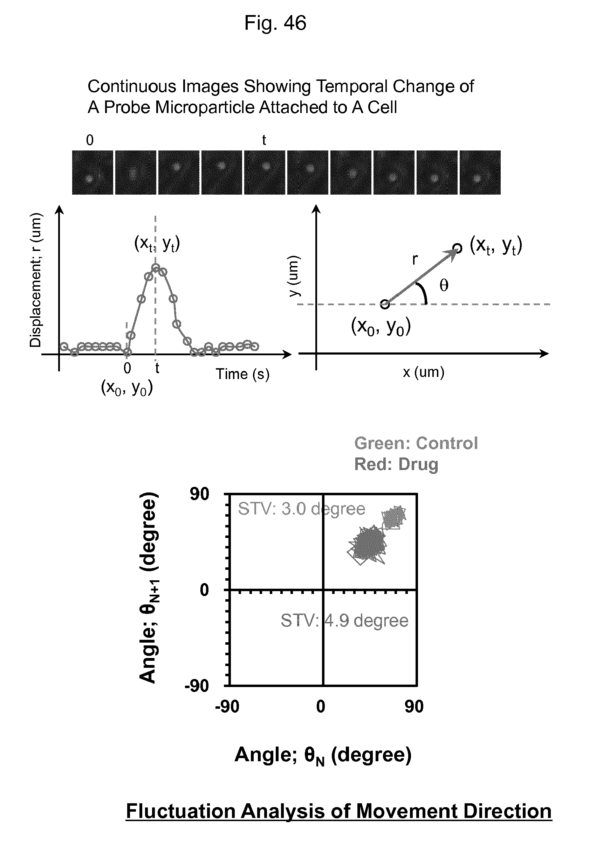

Figure 46 is a diagram illustrating an example of acquisition of data of directions of cell displacements obtained from an example of a system configuration of the present invention to simultaneously measure the mechanical properties and electrical properties of cardiomyocytes. In the upper part of the figure for continuous images which show time variation of probe microparticles attached to the cells, the displacement data is obtained as components of (X, Y); and by converting the components to a polar coordinate system (r, $\theta$) comprised of displacement length r and angular change $\theta$, it is possible to estimate quantitatively the effect of a drug using two parameters of the displacement amount and the angular change. The lower part of the graph illustrates an example of a phenomenon observed when the fluctuations of the angle change of the displacement of the microparticles are in fact increased by the addition of the drug.

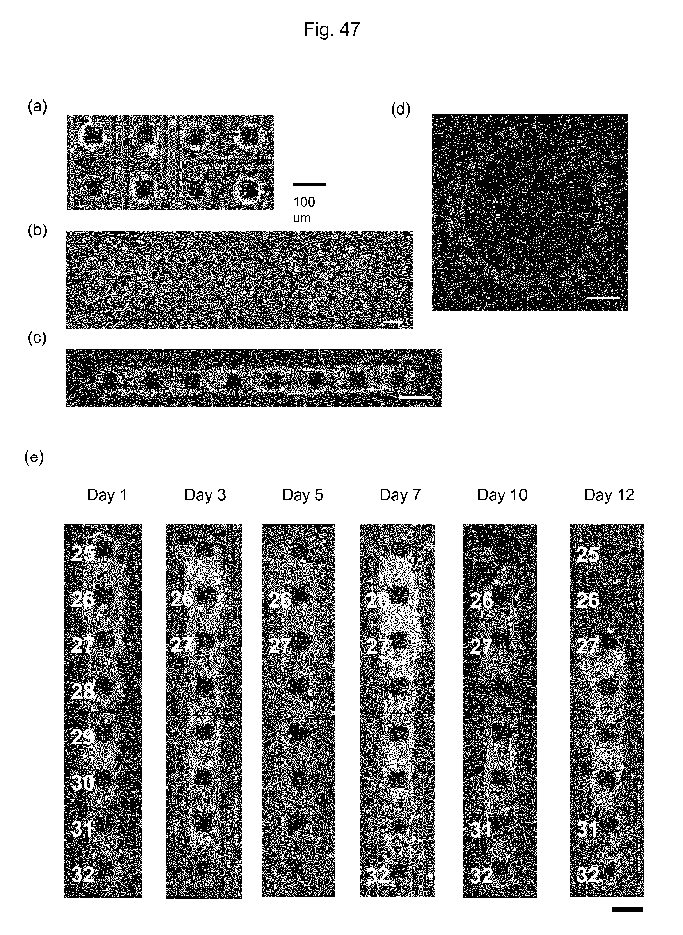

Figure 47 is a diagram illustrating an example of a spatial arrangement of myocardial cells in the network system of the present invention to simultaneously measure mechanical properties and electrical properties of the myocardial cells: (a) one micro-cluster of myocardial cells is arranged on one microelectrode; (b) cells are arranged in a myocardial cell-sheet spread two-dimensionally with respect to the two-dimensionally arranged microelectrode arrays; (c) the myocardial cells are arranged in a straight line on a one-dimensionally or linearly arranged microelectrode array, in which firing of myocardial cells at one end point is conducted to the other end; and (d) a myocardial-cell network is placed in a ring-like fashion on a ring-shaped microelectrode array, in which the ring-like arranged cardiomyocyte network can be arranged as a closed loop, or a part of the ring-like arranged cardiomyocyte network can be cut out to form an open loop. Here, when the cells are placed in a straight line as shown in (c) in particular, if adhesion of the cardiomyocytes to the substrate surface is not sufficient, then as shown in (e), the cells shrink gradually and become unable to maintain the spatial arrangement of cellular network to form a cell mass because the cell-to-cell contractile force of the cardiomyocytes is too strong as compared with their adhesion to the substrate. In order to avoid this result, it is effective to release contractile force by arranging the cells in a ring-shape fashion as shown in (d). Further, it is also effective to use a collagen vitrigel in place of conventional collagen for the collagen layer on the substrate surface.

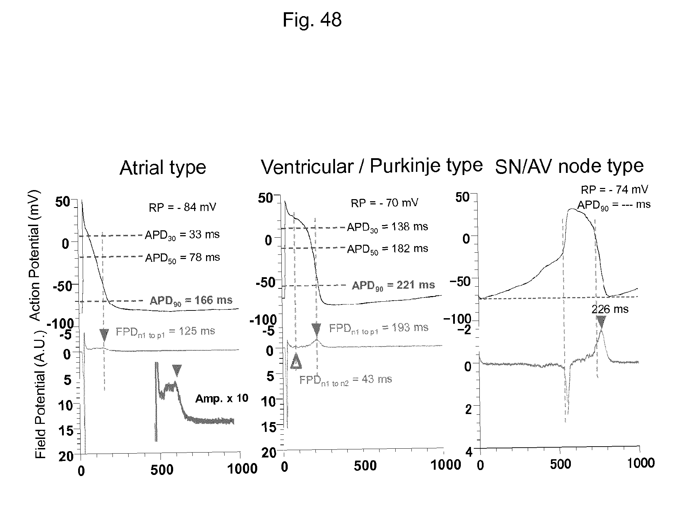

Figure 48 shows graphs of cellular potential (Action Potential) of human stem cell-derived cardiomyocytes (top three) and graphs of extracellular potential (Field Potential) obtained by temporal differentiation of the cellular potential (bottom three) as classified based on the cellular potential measurements of myocardial cells, which is the measurements well known in practice. Atrial muscle type cells are shown on the right, ventricular muscle type cells (Purkinje cells) are shown in the middle and atrioventricular node type cells are shown on the left of the graph. Therefore, when performing measurements of drug response of ventricular muscle or conduction response of the ventricular muscle, it is desirable that the measurements are performed using cells that have the feature as shown in the lower middle graph as measured in extracellular potential. This feature is characterized by generation of sharp inward current of Na ions within 20ms after the start of depolarization associated with a clear sudden depolarization, subsequent generation of gradual inward current of Ca ions within 100ms after the start of depolarization, and generation of prominent outward current of K ions observed on or after 100ms after the start of depolarization, in the absence of addition of a drug.

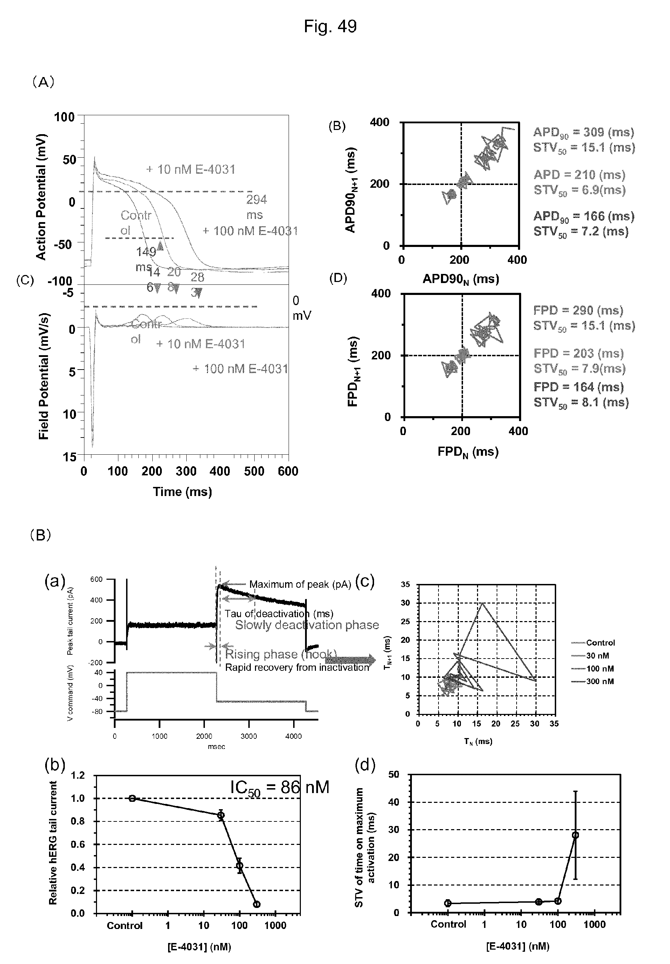

Figure 49 is a diagram showing cellular potential of cardiomyocytes, which is an example of the fluctuation changes in drug response of hERG ion channel. As shown in FIG 49(A), with an addition of an agent E4031 which specifically inhibits the hERG ion channel, a response of extracellular potential (Field Potential: FP), as already mentioned, generates a large fluctuation between adjacent pulsation cycles (the response stability is lost). In the same way, it is also found that cellular potential (Action Potential: AP) of human stem cell-derived cardiomyocytes also generates a fluctuation of a similar trend and scale to that of the FP. Therefore, even with a conventional electrophysiological cellular potential measuring method such as the patch clamp method, it is also possible to measure and estimate quantitatively the degree of fluctuation of the data obtained, rather than taking the average value of the response(s) observed. Further, in order to adjust the stability of the responses of the cells, even when measuring by a patch clamp method, for example, rather than performing the measurement with an isolated single cardiomyocyte, it is desirable to perform the measurement with a single cell which is within a cardiomyocyte population. Figure 49(B) also illustrates an example of the results of measured changes in cellular potential of whole cells in response to the inhibition by E4031 for CHO cells forcibly expressing only hERG ion channel. As can be seen from this result, in a conventional tail current measurement, an average of measured data is obtained, but the measurement becomes difficult as the current itself is reduced with a progress of blocking of ion channels. However, when a fluctuation measurement of cellular responses is performed, an increase in the fluctuation increases abruptly when the blocking probability of the hERG ion channel increases. Therefore, it is effective to use a combination of the two measurement techniques, in which tail current measurements are performed when the current amount is large, and measurements of fluctuation are performed when the current-based measurements are difficult due to a large amount of blocking.

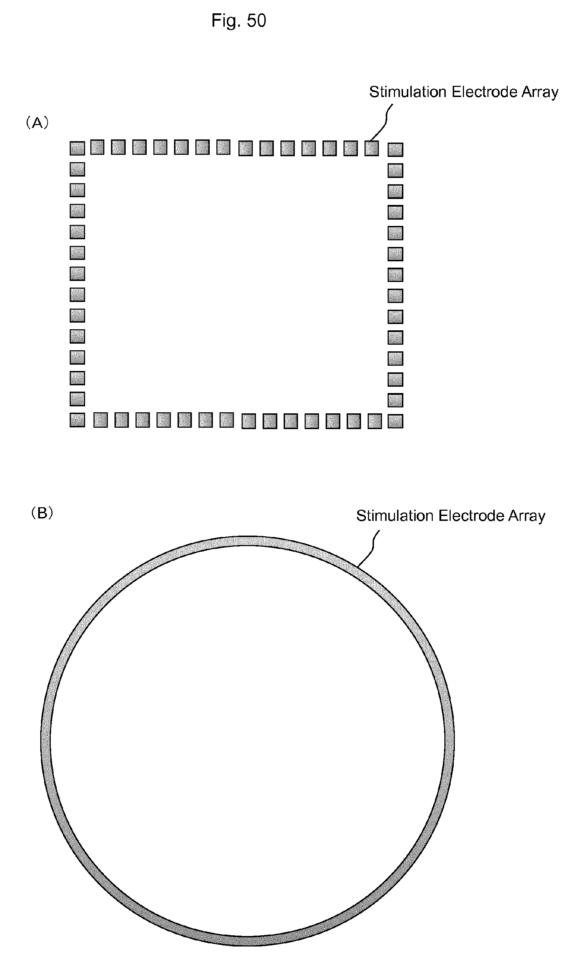

Figure 50 is a diagram illustrating the principle of cell stimulation at any position by superposition of stimulation potentials from a stimulating electrode array. As shown in FIG 50(A), a large number of stimulation electrode arrays, in which the electrode potential and the phase between the adjacent electrodes can be controlled, are arranged two-dimensionally on a substrate. From each electrode, a weak potential change, which does not cause to depolarize cells with the single electrode, is generated and superimposed. In this way, the superimposed potential sufficient to provide a cellular stimulation is specifically generated at a certain position in a two-dimensional surface. For this purpose, the field strength and a phase pattern of an electrode that need to be generated by each electrode are calculated based on the rules of Fourier synthesis, and thereby it is possible to stimulate only a specific location. As an example, as shown in Figure 50(B), for example, the electrode array is arranged in a circular ring shape, and is controlled by the method of the above, and stimulation by a focused electric field may be provided at the center of the ring. When this electrode array is arranged as if it is floating in space in the Z-axis direction from the (R-axis direction) XY plane in a cell culture layer, the arrangement of the stimulation electrode array for electric field focusing in a plane on which an electrode array is arranged (R-axis direction) and in a plane of the electric field irradiation direction (Z-axis direction) is specifically as follows. First, when an electric field is focused at a point $Z_f$ on the Z axis, the distance to $Z_f$ from Ro is defined as $m\lambda$ where the point at which a perpendicular line to the R-axis plane from the focal point $Z_f$ is Ro. Here it is assumed that $\lambda$ is the wavelength of the stimulation signal wave based on the conduction velocity in the cell population to be generated from the stimulation electrodes, and m is a natural number. If the stimulating electrodes are placed at the position of Ro, the radius of the micro-electrodes arranged concentrically is expressed as follows:

$$R_n = \sqrt{2m \cdot n + n^2} \cdot \lambda$$

Here, $R_n$ is the radius of the location of the n-th phase from the center. The position $R_n$ represents the concentric location of the n-th stimulation electrode and the width of the radius is at most $\pm \lambda/4$ approximately in terms of conduction velocity and in terms of the distance from the position of the focal point for stimulation. Of course, it is possible to specifically stimulate the focal point of the concentric circle even if the stimulation electrode is not arranged as a reference at the position of Ro. Also, as for $Z_f$, a stimulation electrode array may be arranged on the same substrate as the substrate on which a cell network is located so as to have $Z_f = 0$. Further, when the focal position is displaced from the center of the circle, it is possible to provide stimulation to any particular position within stimulating ring electrodes by converting the phase of the stimulation signal of each stimulation electrode from the conduction velocity in accordance with the displacement. In the above description, although a cardiomyocyte network is described as an example, as long as cells have transmission capability of excitation conduction between cells, the same procedure is applicable.

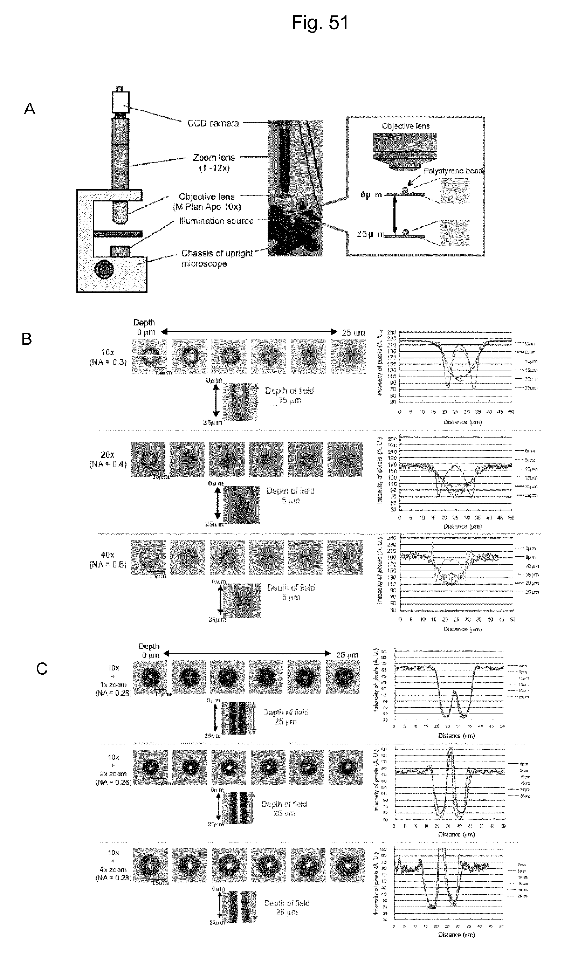

Figure 51 illustrates effects of a combination of a zoom lens system and an objective lens having a numerical aperture less than 0.3 for optical measurements of microparticles. In an ordinary optical system, an image from the objective lens is directly formed on an image-recording device such as a CCD camera. In such cases, the depth of focus corresponds to the number of aperture (NA) of the objective lens, and when the magnification is magnified, there is a problem that the depth of focus at which blur of the image does not occur becomes shallow. In order to solve this problem, the image obtained by the objective lens of low magnification (i.e., a low numerical aperture) may be magnified by a zoom lens system which is added downstream. The spatial resolution of the image is defined by the numerical aperture. In the present invention, because probe particles whose shape is already known are used, as long as the image of the microparticles does not blur at the expense of some spatial resolution, the exact space coordinates of the microparticles can be obtained, and thus there is no problem. Figure 51A illustrates an example of a configuration of an optical system of the present invention. A zoom optical system is arranged downstream of an objective lens, and a video camera is arranged downstream of the zoom optical system. Figure 51B is a result from direct observation of microparticles using objective lenses with different magnifications (numerical apertures) and observation of image blur in a depth direction. As can be seen from the result, an image of a focal depth of up to $15\mu m$ can be observed without blur with a $\times 10$ objective lens (numerical aperture 0.3). However, for either with a $\times 20$ (numerical aperture 0.4) or a $\times 40$ (numerical aperture 0.6), it is only possible to obtain an image without blur for up to the depth direction of about $5\mu m$. Figure 51C is a result of observation of an image of an optical system in which a zoom system is arranged in practice in addition to a $\times 10$ objective lens (numerical aperture 0.28). As can be seen from this result, even when the magnification is magnified to a level of magnification equal to those with a $\times 20$ objective lens or a $\times 40$ objective lens (positional coordinate resolution) using a zoom system, the depth of focus of about $25\mu m$ is maintained, making it possible to track the probe microparticle without losing its coordinates and with resolution of positional coordinates by using a similar image processing even for large displacement in the thickness direction, especially for a cardiomyocyte network which engages in contractile motion.

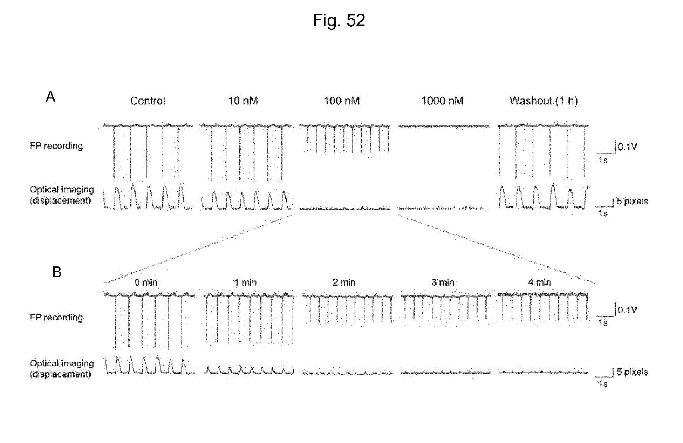

Figure 52 shows an example in which cellular potential measurement and mechanical measurement were simultaneously performed using the system described above. Figure 52A shows a result of simultaneous measurement of the extracellular potential (FP) and change in the developed tension (optical imaging) when verapamil is added as an example of an agent that disperses the contractile tension of the cardiomyocytes. The time variation of the loss of contractile force at a drug concentration of 100nM is shown in Figure 52B. As can be seen from Figure 52B, the electrical firing of excitatory conduction of cells is maintained, contractile forces disappear rapidly, and although an electrophysiological excitation conduction continues eventually, it can be seen that the mechanical contraction force disappears. Further, as can be seen from the data for 1000nM in Figure 52A, in this case, it can be seen that electrophysiological excitation does not occur and mechanical contraction does not occur. As can be seen from this example, use of only electrophysiological measurement causes difficulty in accurately predicting at what point the mechanical contraction is actually lost while the electrical excitation is maintained. Further, with only the mechanical measurement, it is difficult to identify whether the contraction force is lost at the time the mechanical contraction is lost while the electrical excitation is maintained, or the electrical excitation itself is lost and the contraction is lost. However, as shown in FIG 52, it is possible to quantitatively evaluate how the contractile force is lost while there are still electrical excitation stimuli, if both the electrophysiological measurement and the mechanical measurement can be simultaneously measured.

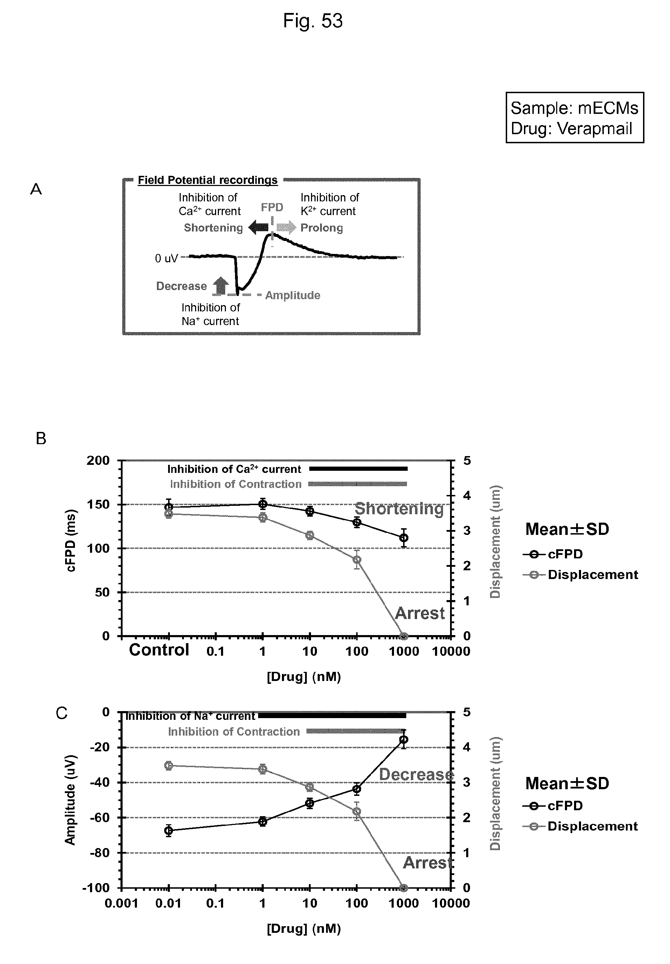

Figure 53 is graphs summarizing the results obtained in Figure 52. Figure 53A is an extracellular potential waveform actually obtained by electrophysiological extracellular potential. With administration of a drug, the following changes occur in extracellular potential. If the drug has a sodium ion channel blocking activity, a reduction of the first portion of the spike waveform occurs, and if the drug has a calcium ion channel blocking activity, the waveform changes in the direction to reduce FPD (time to the inward current peak position from the first spike of sodium), and if the drug has a potassium ion channel blocking activity, the waveform changes just opposite, i.e., in the direction to extend the FPD. Thus, if a drug generates inhibition of calcium ion channels, that causes contraction force and the drug causes potassium ion channel blocking at the same time, apparently reduced FPD cannot be simultaneously measured with respect to a decrease in the contractile force (because the extension effect of potassium counteracts the reducing effect of the FPD due to inhibition of calcium). Therefore, in practice, it is necessary to simultaneously measure the mechanical measurement in addition to electrical measurement.

Figure 53B is a graph summarizing the correlation of actual changes in the FPD and the changes in contractile force (displacement). It is clear that the loss of contractile force is present where the decrease in FPD is not so prominent.

Figure 53C is the analysis result of the loss of contractile force from another perspective. Specifically, as mentioned in the description of Figure 52, the relationship of the change in the intensity of the first spike of sodium and the change in contractile force is illustrated. As can be seen from the figure, the increased inhibition of sodium ion channels is dependent on the concentration of the drug, but in view of the results of Figure 53B, it is found that the first spike maintains sufficient strength to elicit an electrophysiological response of cells (responses of calcium ion channels and of potassium ion channels), and therein loss of tension occurs.

From the above comprehensive analysis, inhibition of sodium ion channels occurs by drug administration; however it is an inhibition at the level where there is still sufficient spare capacity for the generation of stimulation. Further, when the inhibition of calcium ion channels and the inhibition of potassium ion channels are in the same level, there is not a lot of movement in the position of the FPD, and therefore there is no problem about the position of the FPD. It is expected that the occurrence of QT prolongation risks associated with the FPD is not observed. However, in practice, it is analyzed that the loss of contractile force occurs because the inhibition of calcium ion channels occurs.

As the results indicate, it is possible to estimate the relationship between the inhibitory effects of a calcium ion channel and inhibitory effects of a potassium ion channel by combining the simultaneous measurements of electrophysiological extracellular potential waveform analysis and measurements of tension generation, which conventionally could not be estimated by only electrical measurement.

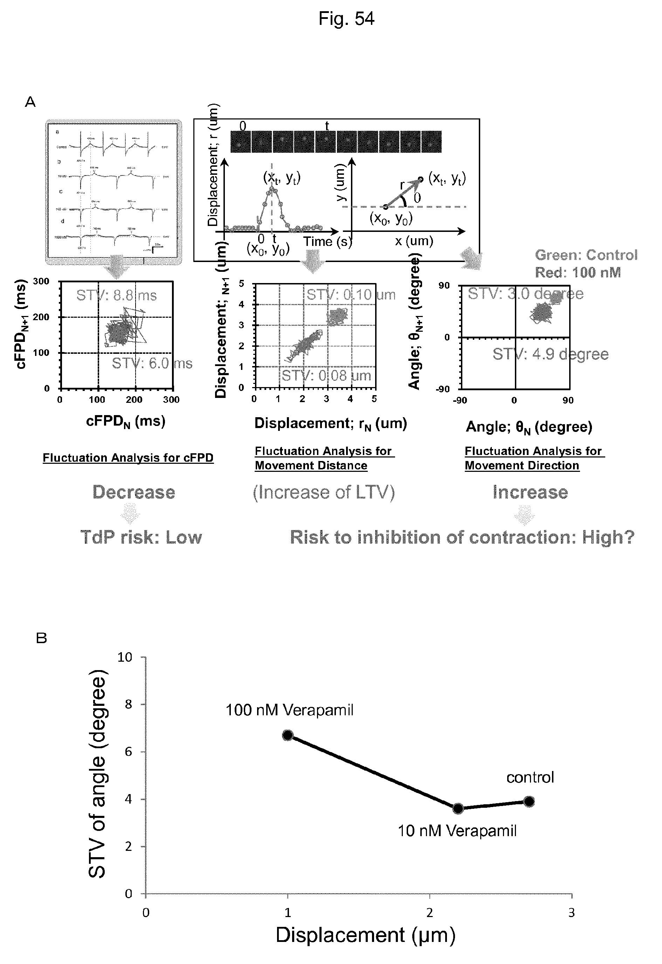

Figure 54A further summarizes points of view of pro-arrhythmic risk measurement by electrical/optical simultaneous measurement in addition to the above point of view. The measurements of time fluctuation of FPD obtained by electrophysiological measurements, fluctuation (variation) of movement distance (displacement amount) between contraction intervals for muscle contraction obtained by optical measurement and fluctuation (variation) of movement direction (angle) between contraction intervals can be performed simultaneously in the system of the present invention. In particular, in addition to the conventional pro-arrhythmic effect due to electrophysiological legacy reasons, it is also possible to analyze to what extent any loss of uniformity in the contractile forces of the cells is due to variation in the original quality of the cardiomyocyte population caused by drug administration as compared to the variation of the FPD by electrophysiological measurements. For example, as a viewpoint which could not be estimated by conventional measurement methods, as also shown in the figure, it is observed that the angle fluctuation also increases, and the variation of the contractile force takes a maximum value with the administration of a drug, while it is observed that fluctuation increase of FPD is not seen. From this, it can be quantitatively estimated that the behavior of myocardial cell population becomes non-uniform in accordance with the decrease in contractile force, and a failure in pump function which requires cooperativity occurs.

In this way, the "fluctuation" analysis obtained for both the displacement direction and angular direction together becomes an indicator for the determination of drug properties which cannot be obtained with only electrophysiological measurements.

Figure 54B is a further graphical presentation of how the increase in fluctuation changes with respect to the reduction of the displacement amount. Upon administration of 10nM verapamil, angle fluctuation barely increases against a decrease in contractile force. However, it can be seen that upon administration of 100nM verapamil, a rapid fluctuation of contractile direction in the angular direction (i.e., randomization of contraction direction of the population) occurs.

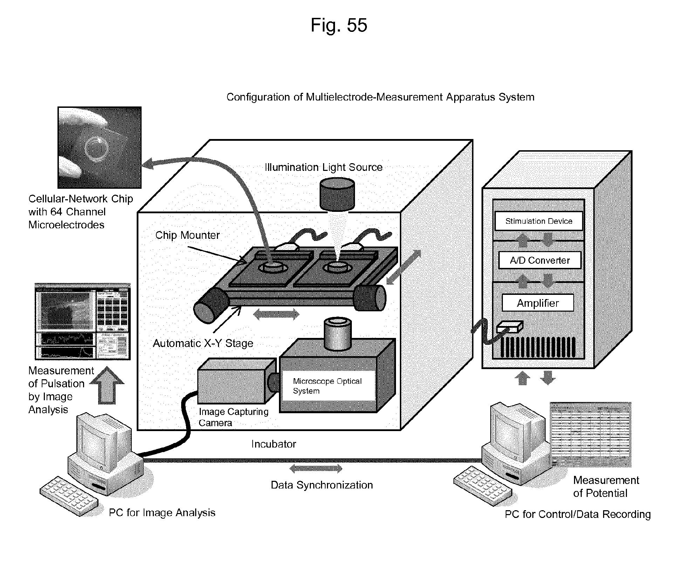

Figure 55 shows an example of a device configuration that combines measurements of extracellular potential and an optical system for measurements of changes in contractile function. Two or more cardiomyocyte-network chips incorporating an extracellular potential measurement capability are placed on a stage that can be moved in three dimensions, a chip mounter is combined with each of the chips and successive measurements of the potential can be performed continuously. As for the optical measurement, it is possible to perform the measurements of displacement (contraction distance) and direction of the displacement (contraction angle) of the myocardial cell in each chip by periodically moving the stage. Here, the measurement of fluctuation can be performed by obtaining pulsation data for n = 50 times for each chip.

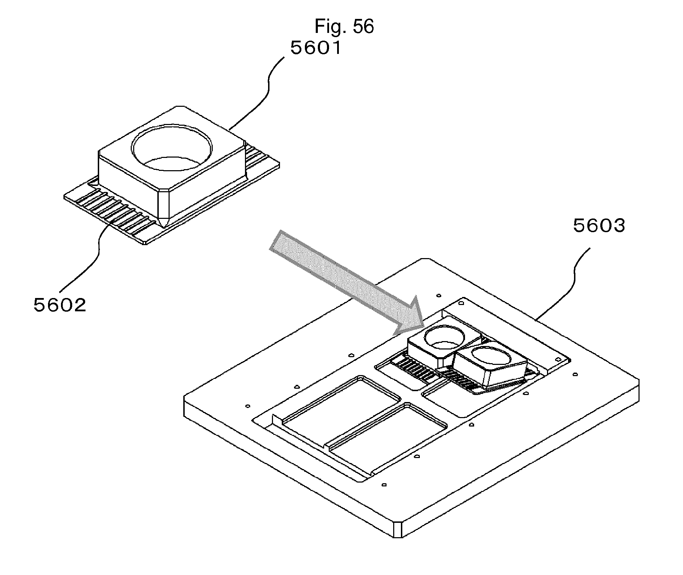

Figure 56 illustrates an example of a structure of a high-throughput cardiomyocyte-network array chip composed of a cell culture module array. In general, when measuring responses to drugs, use of multiwall-type cell culture plates is common in order to increase the measurement throughput. In fact, however, for cell culture plates with a multi-well structure, there is a possibility that all wells may not necessarily be utilized effectively because of problems such as that the state of the cell culture not being good in some well(s), or that cells may not adhere to the plate. In order to solve this problem, each well in the multi-well plate may be separated from each other in advance, and after starting culturing, only wells with good conditions are chosen to combine to make a multi-well plate, thereby making it possible to have all the plates in a good condition available. One example shown in Figure 56 simply illustrates this. Cell(s) are cultured in a well 5601 which is separated in advance to make the smallest unit. Wells which have cultured cells in good conditions are arranged in a plate 5603 to form a high-throughput cardiomyocyte network array chip. Here, since the electrode array 5602 for electrical measurements of extracellular potentials and cell stimulation is arranged in each well, contacts for connecting them are arranged in advance in the plate to place the well. Particularly, since wells can be replaced conveniently in unit of a single well (as one block), economical effects can be achieved by replacing wells of a cell network fatigued with drugs on the plate in a well-by-well manner rather than replacing the wells in a plate-by-plate manner.

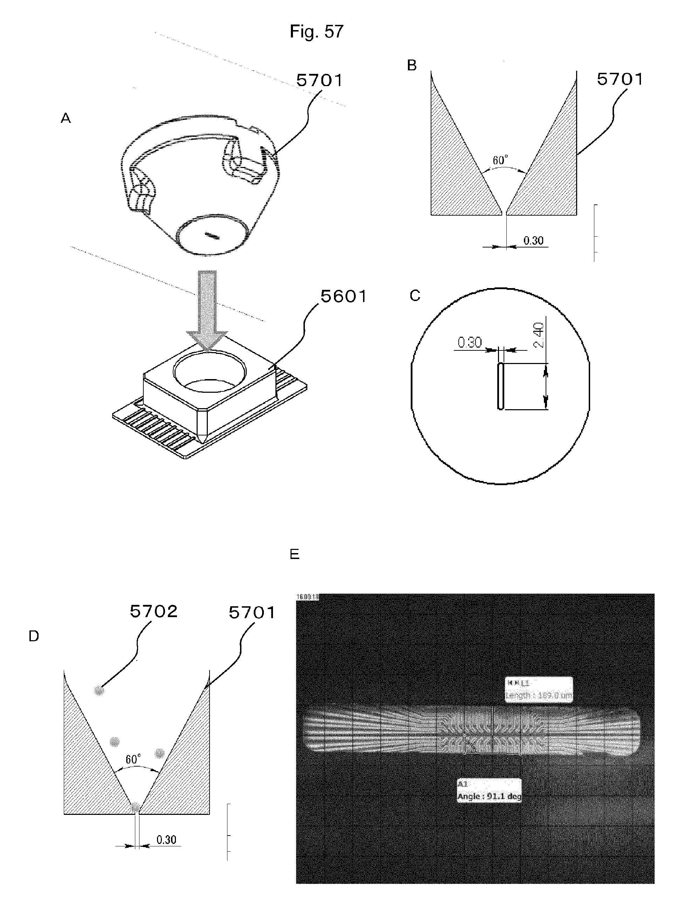

Figure 57 illustrates an example of a configuration of a cellular network deployment technique using a sample loader 5701 to place cardiomyocyte(s) effectively to each well 5601. As also shown in FIG 57A, the sample loader 5701 has a structure that can be inserted into the upper surface of the well 5601. Further, as also shown in Figure 57B and Figure 57C, the bottom surface of the sample loader has a slit with a width of $100\mu m$ to $300\mu m$ and a length of $500\mu m$ to about 3mm, and the inner surface of the sample loader is configured in a funnel-like shape. Thus, as shown in Figure 57D, by dropping a liquid 5702 containing cells onto the inner surface, cells are allowed to settle, and are arranged linearly in the same manner as the shape of the slit. While in the present example, it is configured that the cells precipitate effectively by a steep slope of 30 degrees from the vertical direction, it is possible to precipitate the cells effectively in the slit of the bottom surface by a slope of 40 degrees or less. Here, as long as the cell concentration in the liquid containing the cells is adjusted quantitatively in advance, it is possible to adjust the total number of cells to be arranged by only adjustment of the amount of the liquid, and a monolayer of a cardiomyocyte network can be constructed by minimizing the amount of cells, and a multilayer (e.g., two or three layers) of the cellular network can be constructed by increasing the amount of cells. Further, as shown in Figure 57E, if the structure of the sample loader is adjusted such that the orientation of the slit matches with the orientation of the electrodes which are arranged in a straight line as shown in the present example, cells can be arranged effectively to the electrode array by just inserting the sample loader in the wells. Further, as shown in this example, in order to perform effectively optical measurements and perform solution exchange effectively, the sample loader is effective to precipitate and dispose the cells effectively on the chip and it is preferable that the sample loader is removed when measurements of the cells are performed.

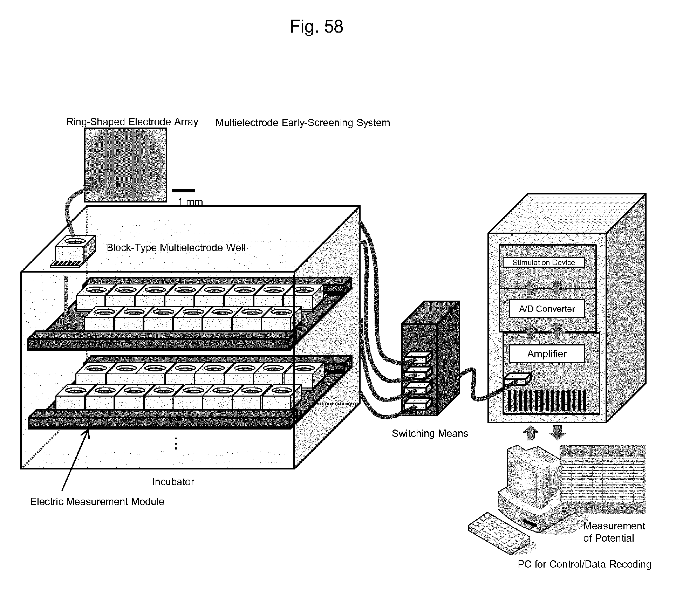

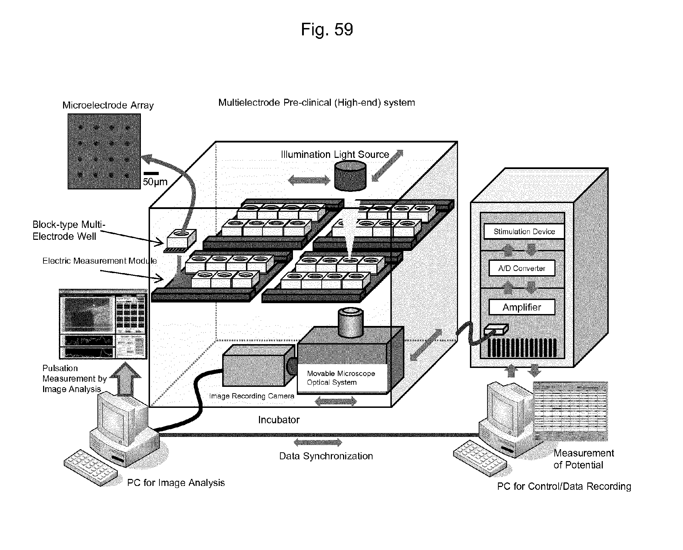

Figure 58 illustrates another example of a system for extracellular potential measurement by a multi-electrode system by arranging the actual block-type wells described above. In Figure 57 above, although the arrangement of the electrodes has discrete electrodes arranged in series, in this example, the electrodes in the well are arranged in a ring-like fashion as shown in Figure 12 above and the like. To arrange the cells in a ring-like fashion in the well, a sample loader with a ring-shaped slit on the bottom surface and not the linear slit on the bottom surface as shown in Figure 57 is used. Figure 59 illustrates a configuration in which an optical measurement module is further incorporated, and measurements of mechanical properties in each well can be performed by moving the optical system.

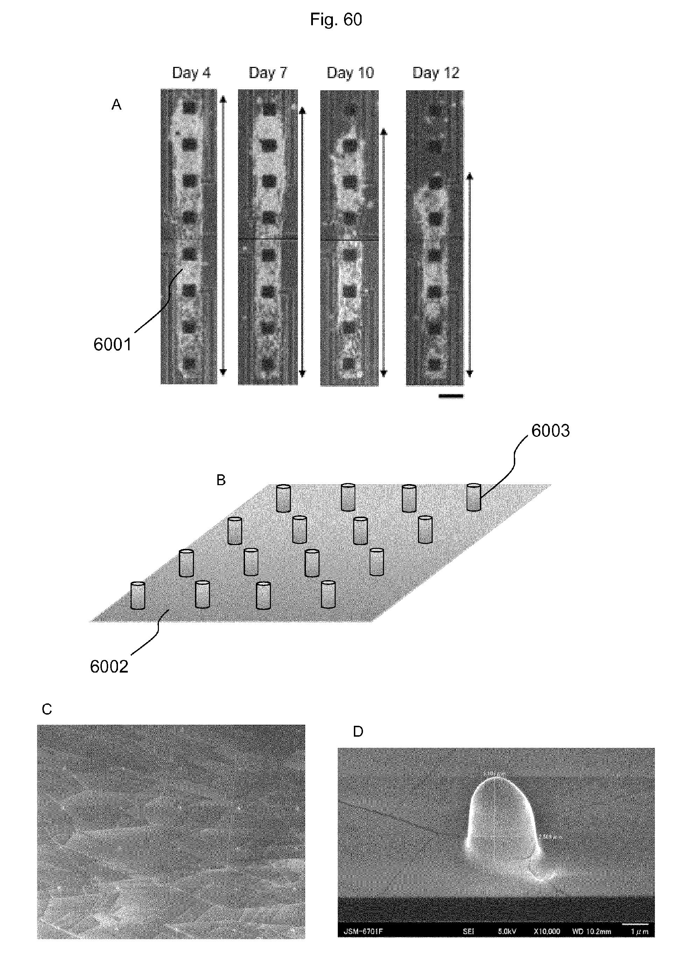

Figure 60 is a diagram illustrating the structure of a substrate on which microprojections are disposed regularly to prevent contraction of cardiomyocytes in a culture medium during measurements using a myocardia-cell network and a myocardial cell sheet. Figure 60A shows, as also shown in Figure 47, an example of experimental results showing a state in which a cardiomyocyte network 6001 gradually peeled off from the collagen layer on the bottom by its generation of contractile force, and gradually contracted to a mass. If the cardiac muscle cells are allowed to contract in clumps like this, they disappear from the microelectrode array that is arranged, making it difficult to measure the extracellular potential, excitatory stimulation conduction velocity from the network, and the time fluctuation thereof. In order to avoid this, as shown in Figure 60B, micro-protrusions (pillars) 6003 may be arranged regularly in the region of the substrate surface 6002 in which the myocardial cell sheet or the cardiomyocyte network is cultured. Figure 60D and Figure 60C are actual electron micrographs of an example of arranging pillars with $3\mu m$ in diameter, $5\mu m$ in height and with a pitch of $50\mu m$. Here, it is preferable that the diameter of the pillar is $5\mu m$ or less and the height is $3\mu m$ or more, and the pitch of the arrangement on the substrate of the pillars is $50\mu m$ or less. Although a cylindrical shape is shown in this example, a rectangular parallelepiped shape may also be used.

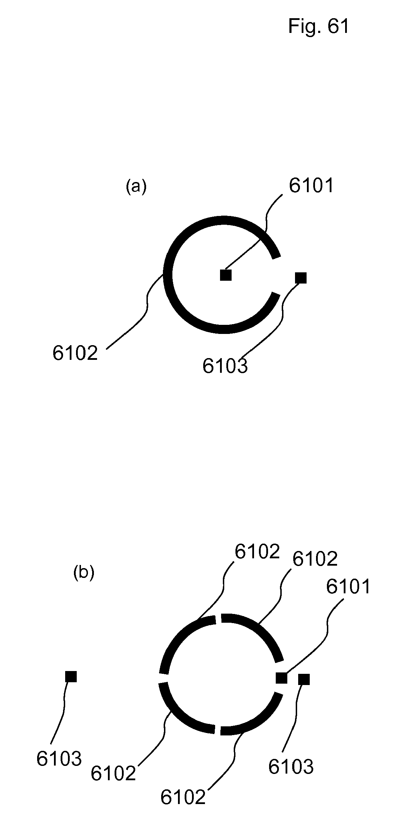

Figure 61 is a schematic diagram showing another example of the electrode arrangement of the multi-electrode substrate shown in FIG 42. First, in Figure 61(a), a measuring electrode 6102 is arranged in a circular ring form, and a stimulation electrode 6101 is disposed in the center of the circular ring. When cardiomyocytes are cultured in a two-dimensional sheet-like fashion on these electrodes, it is possible to measure by the measurement electrodes 6102 how the excitation conduction of myocardial cells ignited by forced stimulation from the center electrode 6101 propagates concentrically. Here, if fluctuation of the conduction occurs by an addition of a drug, waveform disturbance can be measured by the measurement electrode 6102 which is disposed on the circumference. Here, the distance from the center electrode 6101 of the annular measuring electrode 6102 is preferably $200\mu m$ or more. Figure 61(b) is a schematic diagram showing the annular measurement electrode 6102 divided into four portions. Figure 61(a), a 2-dimensional cardiomyocyte sheet is used. In this example, cardiomyocytes are annularly arranged on the annular measurement electrodes 6102 and the stimulation electrode 6101. As for the propagation of the excitation conduction of the annular cardiomyocyte network, it is possible to estimate the rotation direction of the excitation conduction by measuring the time difference of excitation conduction between the four-divided measurement electrodes 6102.

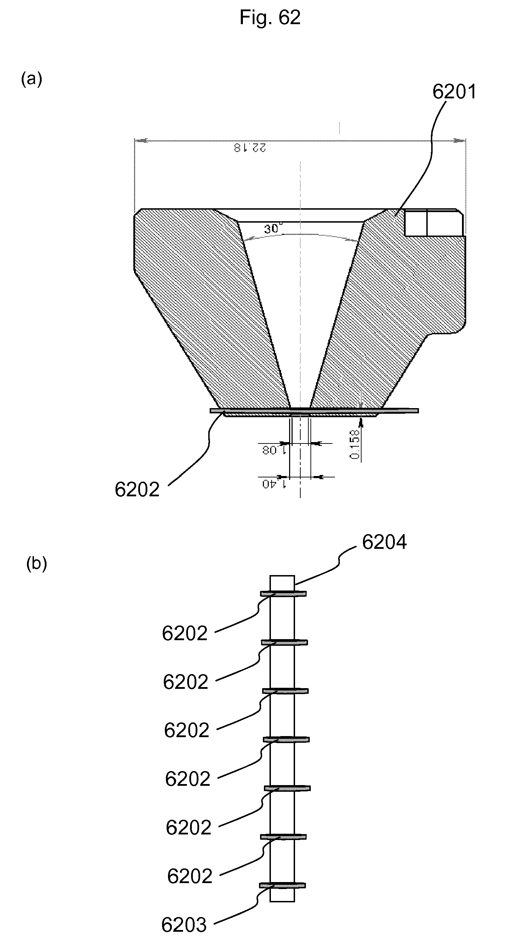

Figure 62 illustrates schematically an example of an embodiment in which metal micro wires are used as electrodes. In the example shown in FIG 62(a), small platinum electrodes 6202 of 10$\mu$m thickness is disposed on the bottom surface of container 6201 having the same shape as the sample loader shown in FIG 57, where the surface of the electrodes is modified with platinum black. If cardiomyocytes are dropped into the vessel 6201, platinum electrodes are incorporated into the cardiomyocyte network that has precipitated, and measurements of cardiomyocyte potential can be performed in the same way as when using the deposition electrode pattern placed on the substrate bottom surface. As shown in the top view of Figure 62(b), using the measurement micro-electrode wires 6202 which are regularly arranged, the extracellular potential of the myocardial cells which are arranged around the wires as well as the conduction between adjacent wires can also be measured. In particular, using one of the wires as a stimulation electrode wire 6203, measurements of the excitation conduction of the cells by forced stimulus are also possible. In this example, a platinum wire of 10$\mu$m in thickness is used. However, measurements with the same spatial resolution are possible as long as the thickness is 30$\mu$m or less. Further, the wire structure has also the effect of fixing the cells surrounding the wire so as to prevent the cardiomyocyte network from contraction as described above in relation to Figure 60.

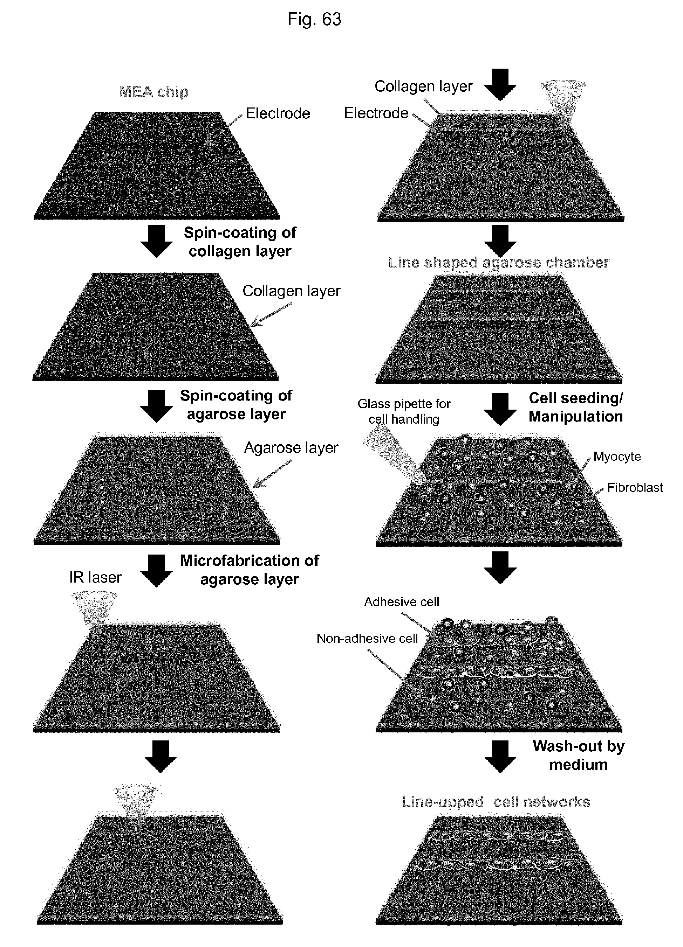

Figure 63 is a schematic illustration of an example of a procedure used for arranging cardiomyocytes. In this example, collagen is applied to a surface of a substrate on which electrodes are arranged. Then, agarose is applied to the surface and the agarose is removed to match with a desired arrangement pattern of a cell population by locally heating to solate the agarose gel using an infrared convergent light. Cells are placed on a spatial pattern thus constructed so as to arrange the cells in the desired pattern. Figure 63 shows an example illustrating that a chamber for housing cells in a space where agarose has been removed in a linear or a parallel fashion, and the cells adhered onto a surface of a substrate within the space. The cardiomyocyte population created in this way can be a population of exclusively cardiomyocytes only as ideal model. Alternatively, fibroblasts can be added up to about 40% to about 60% of the population as a model closely resembling a real heat organ. In particular, since it is known that the rate of fibroblasts in a human heart increases with aging, a standard model can be a population where about 40% of which is fibroblasts and an aging model can be a population where about 60% of which is fibroblasts. In both the standard and the aging models, a $\pm$10% error is allowed around each of the percentage values.

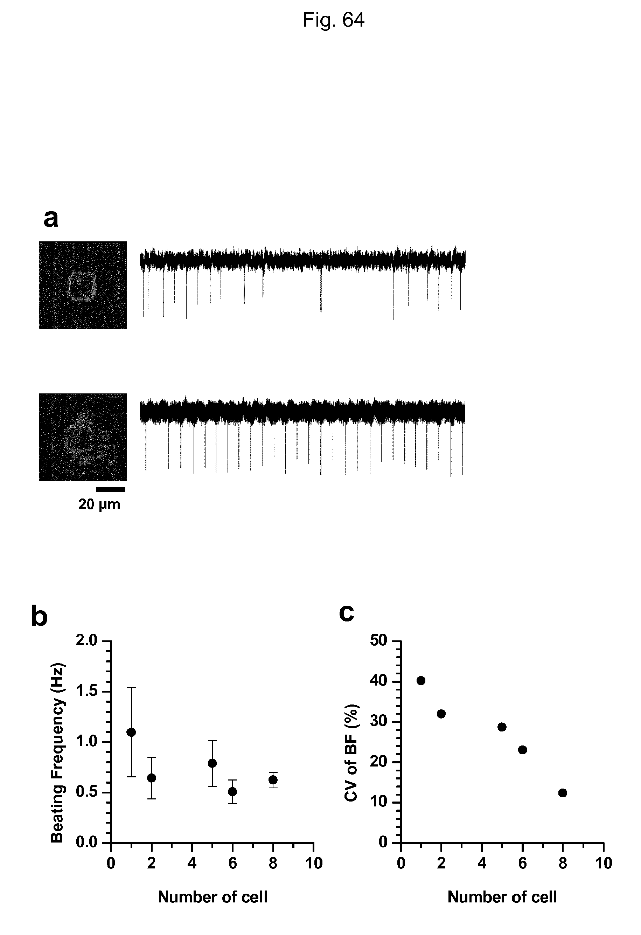

Figures 64a-c show an example of a result showing a population effect to result in pulsation stability of cardiomyocytes. The pulsation stability of the cardiomyocytes is important for obtaining a stable cellular potential waveform. In an isolated state such as a single cell, the pulsation does not stabilize and a temporal fluctuation can be up to 40% (see the upper column of Figure 64a and Figure 64c). With a population of 2 or more cardiomyocytes, the temporal fluctuation will become more stable (see the lower column of Figure 64a [number of cells = 6], Figures 64b, c). For example, in the example shown in Figure 64c, the population contains 8 cells, and the temporal fluctuation was stabilized to about 10%. This shows that it is desirable to use a population of at least two cardiomyocytes for pulsation stability of cardiomyocytes, and a population of at least three, four, five, six or seven cardiomyocytes is preferably used and a population of at least eight cardiomyocytes is further preferable.

In Figure 64b, a graph is shown for a distribution of a pulsation cycle of cardiomyocytes in relation to the number of cardiomyocytes of a cellular network. As can be seen from the graph, the pulsation cycle for a single isolated cardiomyocyte has a great dispersion and is unstable (see Figure 64a upper right column for the pulsation distribution versus elapsed time), while as the number of the cells in the cellular network increases, the pulsation cycle becomes stable. As shown in a graph in the lower column of Figure 64a, for example, the pulsation cycle is significantly stable with the cellular network of 6 cells.

Figure 64c is a graph of a distribution of the pulsation cycle shown in Figure 64b but re-presented in percentage. As can be seen from this graph, the ignition interval of an isolated single cardiomyocyte has an about 40% temporal fluctuation, while the ignition interval of a network of 8 cardiomyocytes settles down to about 10% which is a value of stability almost equal to that of a heart organ.

As shown in the Example above referring to Figures 64a-c, a risk of an administered drug is evaluated based on the level of fluctuation detected from temporal change in the pattern of the cellular-potential wave form of a single cell of interest or a certain population of cells, especially based on the level of fluctuation arisen from potassium ion channel area. With this procedure, it was found that the toxicity could be evaluated more highly accurately than a conventional procedure where an estimation of inhibition of ion channels is calculated based on a temporal average of a pattern of a cellular potential wave form. However, a problem is that one cannot accurately evaluate the toxicity with this procedure alone when evaluating toxicity of a drug such as terfenadine that requires direct measurements of an abnormal transmission between cardiomyocytes. For this problem, a solution is a measurement focusing on temporal fluctuation of excitation conduction transmitting through the cell population.

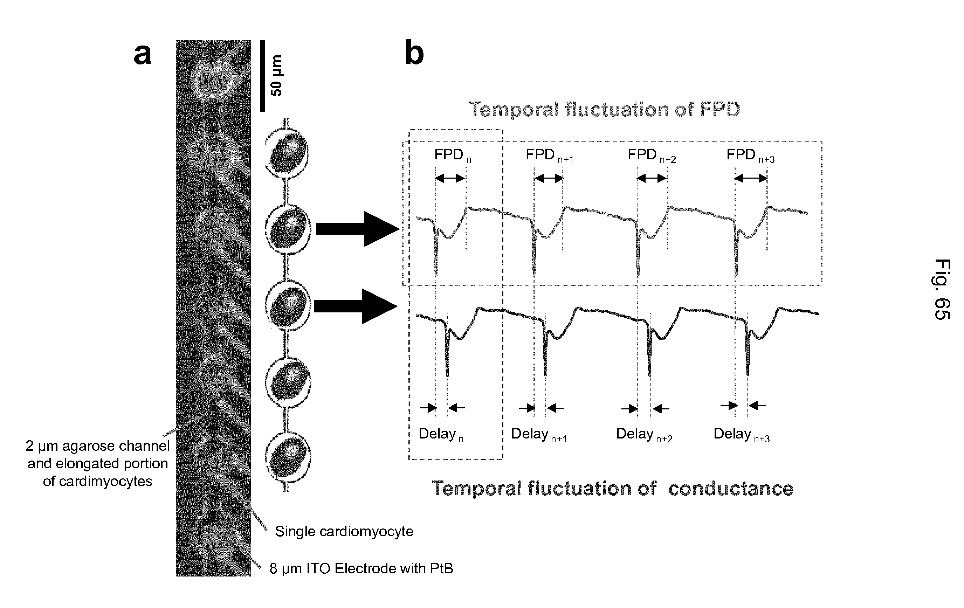

Figures 65a, b schematically show an example of a method of such measurement of excitation conduction transmitting through a cardiomyocyte network. Figure 65a shows a cellular potential measurement chip in which a plurality of cell housings, which were created by partially removing an agarose layer applied on a substrate by locally heating with a convergent infra-red ray, are connected together by communication channels (about 2 μm width), which were also created by partially removing the agarose layer, and arranged in a line. Arranged on a bottom surface of each cell housing on a substrate surface are respective electrodes (about 8 μm in diameter) for measurements of cellular potential, and from each of the electrodes, respective lead lines are extended. A single cardiomyocyte is received in each of the cell housings.

Specifically, in order to measure the temporal variation in the excitation conduction transmitting through the cellular population, an ignition peak of depolarization excited by an initial excitation conduction of each cell or each local point is measured, and at the same time, an ignition peak of an initial depolarization based on excitation conduction in another local area of adjacent another cell or adjacent another cellular population is measured; then a variation of a transmission rate or a transmission elapsed time is measured with respect to the distance between the two measurement points; and then the level of fluctuation of the transmission is quantitatively measured. Generally, the initial peak based on the excitation conduction is deemed to be a sodium spike. Thus, in view of this, the measurements of the initial ignition peak based on the excitation conduction are equivalent to the measurements of the sodium spikes. For example, where a drug with a big risk of proarrhlythmia is used, an increase in the fluctuation is utilized for the measurements. Here, the following are required: (1) a network of a plurality of cardiomyocytes is formed; (2) there is a means for accurately measuring the transmission delay time by measuring respective cellular potential wave form at two or more spatially different points within the network, and simultaneously accurately measuring the time of depolarization ignition spikes excited by the initial excitation conduction of the wave form; (3) in the case where a forced pulsation is provided, three or more electrodes are used to measure time required for transmission of the pulsation between respective electrodes at other measurement electrodes using the electrodes at both ends as stimulation electrodes, enabling measurements of temporal fluctuation of transmission rate or transmission time between the electrodes. As for the fluctuation, the STV as shown in Equation 3 in Figure 24(c), for example, can be used for quantification. Here, $Delay_n$ and $Delay_{n+1}$ (Figure 65) can substitute for $V_n$ and $V_{n+1}$. For transmission, when velocity is used, respective velocities can substitute for $Delay_n$ and $Delay_{n+1}$. To obtain statistic results, the larger the number of n is the better. Preferably n is at least 30 to evaluate the STV and obtain stable results. (4) Further, in the case where spontaneous pulsation of cardiomyocytes is utilized, the positon of a pace maker cell is important. For accurate measurements in the presence of a pacemaker cell for an overall cellular network between two electrodes for the measurements of spontaneous pulsation of cardiomyocytes, three or more measurement electrodes are necessary to be spatially arranged so as to enable 2 or more sets of the measurements between 2 different points. In particular, 3 or more measurement electrodes are preferably arranged in a line at equal intervals, while 2-dimensionally extended electrodes are desirable for measurements of irregular transmissions in a plane extending 2-dimensionally in a sheet-like fashion. In the case where a pacemaker cell is present between specific two electrodes, the distance from the pacemaker to the electrode is shorter than the distance between adjacent measurement electrodes. Thus, the transmission time will be measured as an extremely shorter transmission time than a transmission delay measured normally between adjacent electrodes. For this reason, the position where the pacemaker cell is present can be approximately predicted. In such a case, it is desirable not to use the data obtained from measurements at 2 measurement electrodes between which the pacemaker cell area is present. Further, the delay time for transmission can be used directly and the temporal fluctuation of the transmission time can be used as an indicator for the measurements of temporal fluctuation if the distance between two measurement electrodes of the plurality of electrodes is the same for all. However, if a combination of transmission time for different distances between two electrodes are used for the evaluation, the transmission rate, not the transmission time, is used as an indicator and the data is normalized to rate information that is independent of an inter-electrode distance, and the difference in the fluctuation time that is dependent on the inter-electrode distance need to be corrected.

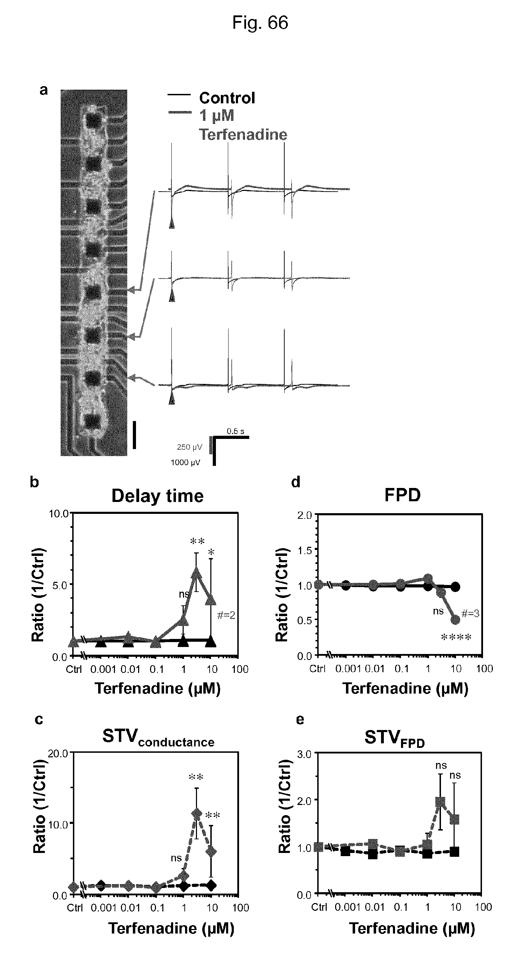

Figures 66a-e show an example of a result of a measurement of excitation conduction transmitting through a cardiomyocyte network. Figure 66a shows a microscopic photograph of a cardiomyocyte network in whch the cells are arranged in a line at a width actually used in experiments, and an example of information of the cellular potential wave forms obtained respectively from of electrodes adjacent to each other. Figure 66b shows an extent of delay in transmission time between adjacent electrodes due to an addition of terfenadine as a value relative to a standard value at which no drug is added. It can be seen that the transmission rate suddenly decreased at the point where the concentration of terfenadine is over 1 μM. From Figure 66c, it is apparent that the fluctuation of time required for transmission increases at the point where the terfenadine concentration is over 1 μM. On the other hand, FPD (Figure 66d) and $STV_{FPD}$ (Figure 66e), which is the fluctuation of FPD, of cardiomyocytes each of which are placed on each of the electrodes are obtained simultaneously by a direct analysis of a cellular potential wave form of each electrode. However, FPD which is one of the indicators of toxicity due to addition of terfenadine is not seen and the fluctuation of FPD is barely recognizable. From these results, it is understood that the fluctuation of the transmission rate is the most effective factor for prediction of toxicity of terfenadine.

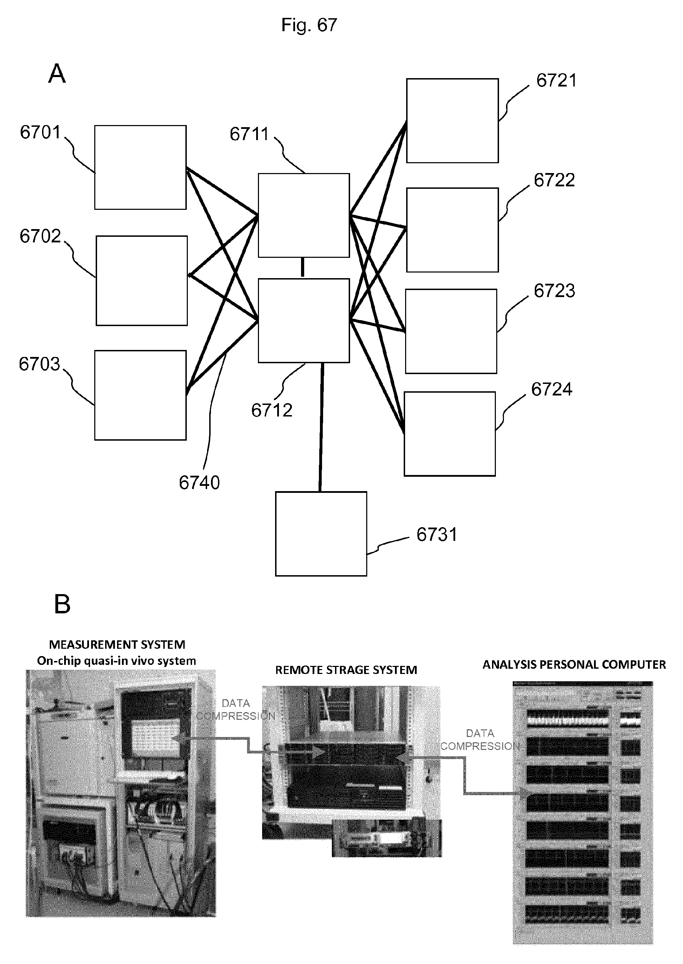

Figure 67A shows an example of a procedure of a measurement, a storing of results and an analysis of excitation conduction transmitting through a cardiomyocyte network. Using the measurement systems 6701, 6702 and 6703, continuous measurements of the excitation conduction transmitting through the cardiomyocyte network can be performed independently. As for an acute action (direct) protocol, for example, the evaluation starts after 5 minutes (assuming that it has reached an equilibrium state) from an addition of a drug at respective concentrations for evaluation. Data obtained between 5-10 minutes after the addition of the drug at respective concentrations are recorded for evaluation. Further, the concentrations for the effects of the drug are measured at 6 or more different conditions including a control condition.

The measured data are then transferred with a communication means 6740 (e.g. cables, wireless) and stored in the storing means 6711 such as a hard disk or SSD which stores measurement results in real time. Then, the data are analyzed by a analysis means 6721, 6722, 6723 and 6734 such as a personal computer for analysis of the stored data, and proarrhlythmia indices such as an extension of FPD and an increase in the fluctuation of transmission are calculated as numerical values, and then these analyzed results are stored in a recording device 6712 such as a hard disk or SSD, and at the same time, associated with the measured data in the storing means 6711. Here, although it is possible to use measurement means 6701, 6702 and 6703 at the same time for the real-time analysis, it is desirable to use the measurement means for measurements only because there is a risk that measurements with a high enough speed are not possible especially for measurements of transmission rates at a high speed due to a load on the measurement device, making the speed of the measurements slower. On the other hand, as for the analysis means, to prevent a prolonged analysis time required for analyzing input data obtained by the measurement means in real time, a dispersed analysis means such as a plurality personal computers for analyses can be used to access the data which is independently integrated and stored, and to divide the data and analyze the divided data to obtain analysis results in real time without delay with respect to the measurement intervals.

Next, the analysis data is displayed and reported to an operator on the evaluation means 6731 such as a personal computer for analyzing toxicity of a drug using the analyzed measurement results. As necessary, associated measurement data or numerical analysis results can be browsed through instructions of the operator. Further, a control means for feedback control of the measurement means to perform another measurement of the excitation conduction transmitting through the cardiomyocyte network depending on the evaluated results can also be provided in the case where the operator has pre-instructed to perform a retrial for confirmation, or the operator instructs to perform a retrial after browsing the results on a display of the computer.

Figure 67B shows a photographic representation of respective examples of the above measurement system, the data recording (storing) device, and the data analysis device.

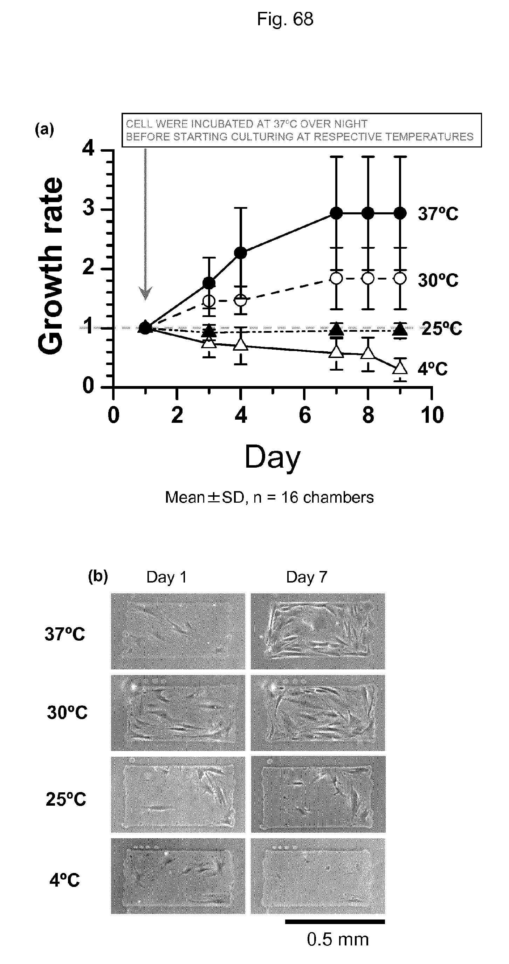

Figures 68(a) and (b) show a relationship between a growth rate of fibroblasts used in the cardiomyocyte network and a culturing temperature. It is desirable to add fibroblasts at a ratio corresponding to aging to the cardiomyocyte network in order to measure the conduction in a condition more similar to that of a living organism. For example, it is preferable to add 40% fibroblasts. However, there is a problem with the cardiomyocyte network chip containing fibroblasts in which the ratio of fibroblasts in the cardiomyocyte network changes as the cells undergo a repeated cell division during storage and/or transportation resulting in a shape change of the network. To prevent this from happening, it is desirable to use a means for preventing growth of fibroblasts during storage and/or transportation. Figure 68(a) shows a graph of growth curve as a function of change in the culturing temperature. Figure 68(b) shows a microscopic photograph of actual fibroblasts taken on the first day and 7th day of culturing at respective temperatures. As can be seen from Figure 68(a), fibroblasts stop growing at a culturing temperature of 25 °C or below and the number of fibroblasts decreases when the culturing temperature goes down to 4 °C. From these observations, it is recognizable that the growth of fibroblasts can be prevented at the temperature of 25 °C or lower. Ideally, the cells can be stored and transported at a temperature ranged from 20 °C to 25 °C with the growth of fibroblasts in the cardiomyocyte network being prevented and the cellular function being maintained.

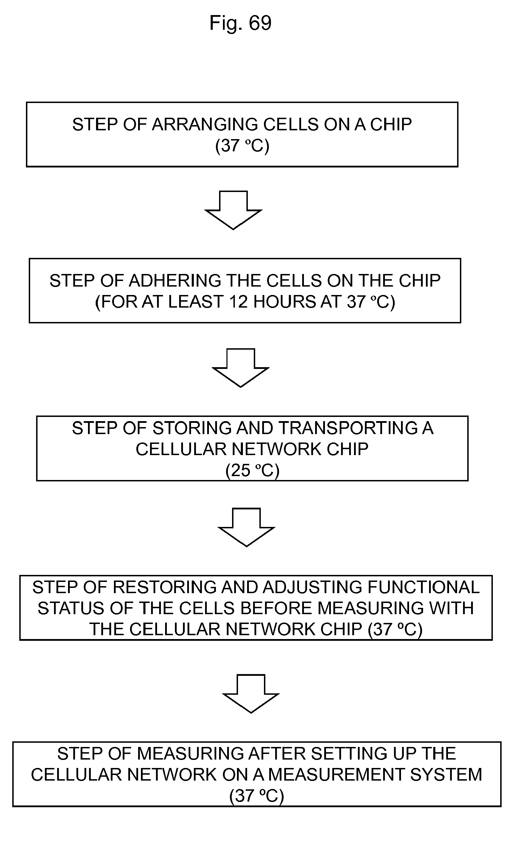

Figure 69 schematically shows an example of the procedure for creation, storage and use of a cardiomyocyte network in which fibroblasts are mixed. Here, the procedure is comprised of arranging cardiomyocytes and fibroblasts in a cardiomyocyte network chip used for a cardiomyocyte toxicity evaluation apparatus at a temperature of 37 °C; incubating the cells to allow adhesion to the chip at 37 °C for at least 12 hours; storing and transporting the cardiomyocyte network chip at a temperature of 25 °C or lower at which fibroblasts do not grow; restoring the function of the cells by re-culturing the cellular network chip at 37 °C before re-starting measurements; and setting up the chip on a measurement system for measurements.

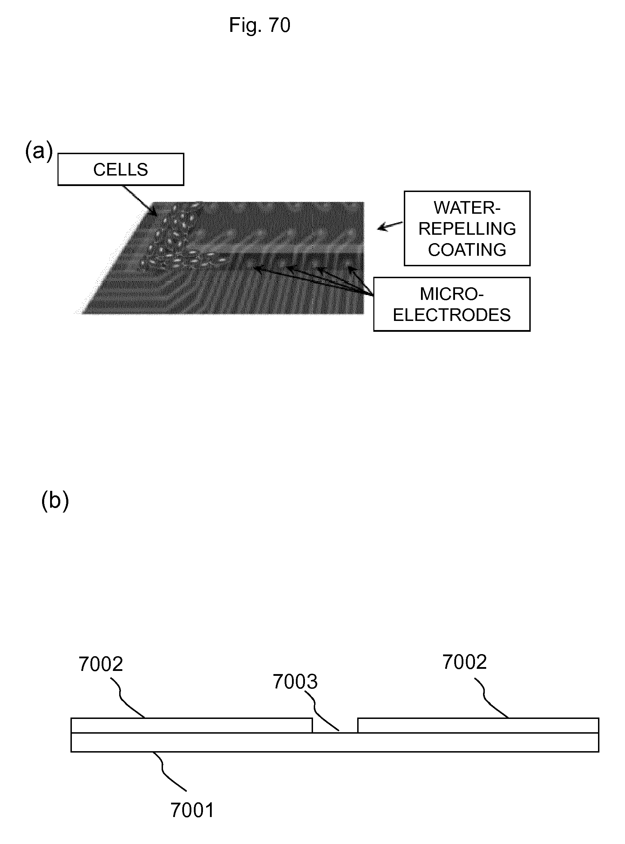

Figures 70(a) and (b) schematically show an example of a structure of a cardiomyocyte network chip on which water-repellent solids are arranged. For example, the water-repellent solids are arranged in areas where cells are not to be arranged in the cardiomyocyte network chip used in the cardiomyocyte toxicity evaluation apparatus so that the cells are arranged in areas only where the water-repellent solids are not arranged. In Figure 70(a), a collagen solution, which is dropped on areas where the cells, including microelectrodes, are to be arranged, is repelled by and cannot be applied on the water-repellent area and thus the cells are arranged only on the area where no water-repelling solids are arranged. Figure 70(b) further shows a cross-section of the chip. Water-repellent area 7002 is arranged on substrate 7001 and a cell-adhesive coating with collagen and the like is arranged on area 7003. For preparation of the water-repelling area, a polyimide solution containing dense Teflon™ micro particles, for example, are arranged on a chip using a means that enables arrangement at a resolution of a micrometer order such as an inkjet printer or micro printing technique, and the chip is baked at a temperature of 250 °C or higher to result in a cellular chip on which a rough surface of Teflon™ microparticles with a desired special pattern is arranged. Although a cardiomyocyte network is demonstrated by way of example in this example, the present procedure can be used widely for culturing nerve cells, hepatocytes and the like as well as cardiomyocytes in a particular cellular arrangement.

Preferably, the particle diameter is, but not limited to, at least 0.5 microns and not more than 20 microns. Further, although polyimides are used for immobilizing the Teflon™ particles on the surface of the substrate in the present example, thermosetting resins (e.g. phenol resin, epoxy resin, melamine resin, urea resin, unsaturated polyester resin, alkyd resin, polyurethane resin), commodity plastic (e.g. polyethylene, polypropylene, polyvinyl chloride, polystyrene, polyvinyl acetate, ABS resin, AS resin, acrylic resin), engineering plastic (e.g. polyamide, polyacetal, polycarbonate, modified polyphenylene ether, polyester), super engineering plastic (e.g. polyphenylene sulfide, polysulfine, polyethersulfon, polyether-ether-ketone, polyamide imide) and the like may be used. Here, in the case where the plastic is used, the plastic can be used directly as a material for a substrate and Teflon™ microparticles may be contained in the substrate. In such a case, if the specific gravity of the plastic material used is larger than that of the Teflon™ microparticle, a top surface of the plastic material can be used as a water-repelling surface, and the Teflon™ microparticles surface before the plastic material solidifies such that a hydrophobic surface can be formed. If the specific gravity of the plastic material is smaller than that of the Teflon™ microparticle, the Teflon™ microparticles sediment toward a bottom surface of the plastic material such that a hydrophobic surface is formed on the bottom surface.

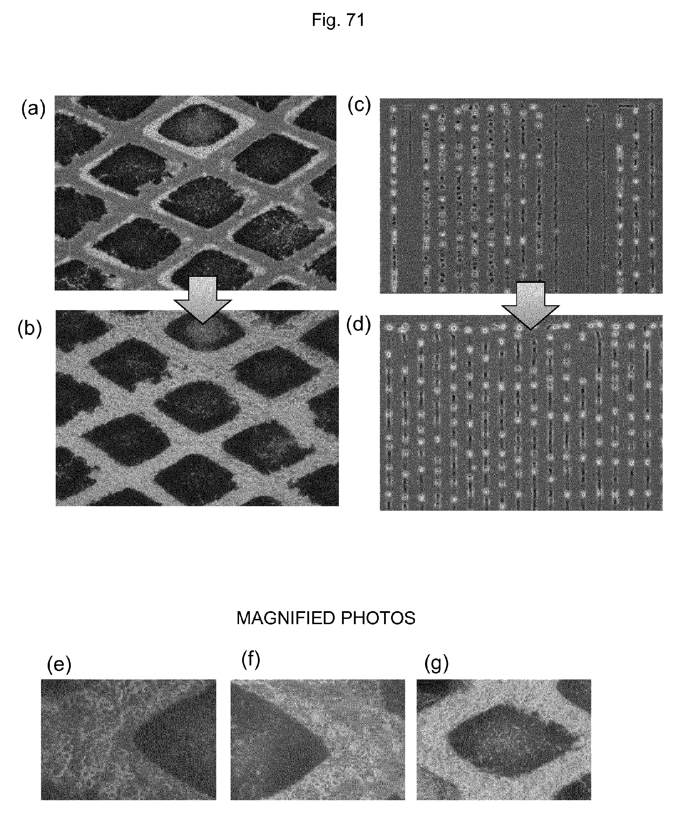

Figures 71(a)-(g) show microscopic photographs showing examples of culturing of cardiomyocytes on a chip on which Teflon™ microparticles are arranged in water-repelling areas. Figures 71 (a) and (c) show microscopic photographs immediately after starting of culturing of arranged cardiomyocytes. Figures 71(b) and (d) show microscopic photographs after culturing the cells which have adhered and formed a network. Particularly, Figures 71(e), (f) and (g) show enlarged photos of the photo of Figure 71 (b). As can be seen from these photos, the cells do not enter the Teflon™ treated area and the arrangement of the cells corresponds to the contour of the Teflon™ arrangement. In addition, especially, the latticed cellular network arrangement as shown in Figure 71 (a) is one of the arrangements that enable effective measurements of an occurrence of a micro-entry. The linear shape cellular network arrangement as shown in Figure 71(c) is also one of the arrangements that enable effective measurements of irregularity of the transmission.

In Figure 71, the width of the area where cell adhesive agents are applied for the cells to be arranged is preferably 10 microns or larger. The upper limit is preferably 100 microns or smaller. For these examples, even without using microelectrodes for measurements of cellular potentials or transmission rates, an optical means can be easily used to evaluate arrhythmogenicity by measuring irregularity of the transmission of contractile oscillation or a decrease in the contraction. More specifically, for example, the optical detection method as shown in Figures 51-55 can be used to detect asynchronized behavior of displacement between adjacent two points (i.e. between probe microparticles) to evaluate arrhythmogenicity. Further, in the case where the arrhythmogenicity is optically observed, since the frequency of a proarrhythmic behavior is around 3 Hertz, it is desirable to have optical measurability at a resolution capable of quantitative measurements of irregular oscillation at 3 Hertz. Specifically, preferably, the measurements can be made at a temporal resolution ranging from 1 millisecond to 10 milliseconds. It is also preferable to have a spatial resolution that enables quantitative measurements of, for example, a decrease in displacement or synchronization of respective micro regions. More specifically, the spatial resolution is preferably at least 1 micron to 5 microns.

INDUSTRIAL APPLICABILITY

According to the present invention, it is made possible to evaluate whether cardiomyocytes obtained through differentiation of stem cells, such as iPS cells, are healthy cardiomyocytes that can be used for agent screening or regenerative medicine for cardiomyocytes.

DESCRIPTION OF REFERENCE NUMERALS

1: transparent substrate, 2: microelectrode, 2c: reference electrode, 2': lead wire of microelectrode 2, $3_1$, $3_2$, $3_3$, $3_4$: a wall by agarose gel, $4_1$, $4_2$, $4_3$ and $4_4$: gap, 7: peripheral surrounding wall, $8_1$, $8_2$, $8_3$: pipe, PC: personal computer, Ms: operation signal of PC, $10, 10_1, 10_2, 10_3, ---, 10_n$: cardiomyocytes or fibroblasts, 15: transparent stage of an optical observation device, 16: X-Y drive unit, 18: Z drive unit, $CH_1$, $CH_2$, $CH_3$, $CH_n$: cell holding unit, CCC: cell communication channel,

$10_G$:cell population, $11_a$: barrier, $11_b$: opening, 19, 191,192,193: dichroic mirror, 20, 201: a band-pass filter, 21, 211: camera, 22: light source, 221: fluorescent light source, 23,231: band-pass filter, 24,241: shutter, 25: condenser lens, 26: objective lens, 27: movable electrode, 28: ground electrode, 29, 291: switching circuit, 30, 301: electrical signal measuring circuit, 31,311: electrical stimulation circuit, 32: cardiomyocytes, 33: fibroblasts, 34: pipette for cell placement, 35: N-th round transmission pathway, 36: (N +1)th round transmission pathway, 37: (N +2)th round transmission pathway, 38: measuring electrode, 39: reference electrode, 40: liquid sending system, 41: cell population arranged in a ring shape, 42: 96-well plate, 43: photo-sensitive element of a camera, 44: cell, 45: cell stimulation electrode, 100: myocardial toxicity testing apparatus, 4201: multi-electrode substrate, 4202: cell culture vessel, 4203: flow of a solution, 4204: liquid level, 4301: glass substrate, 4302: transparent electrode, 4303: metal layer, 4304: insulating film, 4401: stimulating electrode, 4402: measurement electrodes, 4403: reference electrode, 4404: cell culturing area, 5601: single well, 5602: electrode array, 5603: plate, 5701: sample loader, 5702: liquid containing cells, 6001: myocardial cell network, 6002: surface of the substrate, 6003: micro protrusions (pillar), 6101: stimulation electrode, 6102: measurement electrode, 6103: reference electrode, 6201: container, 6202: micro- electrode wire for measurement, 6203: stimulation electrode wire, 6204: groove, 6701, 6702, 6703: excitation conduction measurement system, 6711: measurement data recording (storing) device, 6712: analysis data recording (storing) device, 6721, 6722, 6723, 6724: measurement data analysis device (e.g. personal computer), 6731: drug toxicity determination means (e.g. personal computer), 6740: communication means, 7001: substrate, 7002: water-repellent area, 7003: cell adhesive coating.

**Claims**

1. A myocardial toxicity evaluation apparatus, comprising:

    a substrate;
    a plurality of stably pulsating subject cardiomyocytes or a cell population comprising the subject cardiomyocytes arranged on the substrate;
    a cell population holding area which is formed on the substrate and holds the cell population and a cell culture medium;
    at least two measurement electrodes on each of which a single cell or a local portion of the cell population is placed in a cardiomyocyte network consisting of the plurality of cardiomyocytes or the cell population comprising the cardiomyocytes;
    a potential measuring means configured to measure cellular potential of the cardiomyocytes that are placed on the measurement electrodes continuously over time using lead wires which are respectively connected to each of the measurement electrodes;
    a computing means configured to calculate a transmission time or a transmission rate of excitation conduction transmitting between the at least two measurement electrodes using data measured by the potential measuring means, wherein the transmission time or the transmission rate of the excitation conduction is calculated through a comparison of time for an occurrence of depolarization due to an initial excitation conduction of cellular potential between two adjacent measurement electrodes.

2. The myocardial toxicity evaluation apparatus according to claim 1, which is **characterized by** the use of a sodium spike as a measurement index for the depolarization excited by the initial excitation conduction of the cellular potential.

3. The myocardial toxicity evaluation apparatus according to claim 1 or 2, wherein the computing means performs a comparison computing of the transmission time or the transmission rate of the excitation conduction between adjacent two time points (Delay$_n$, Delay$_{n+1}$).

4. The myocardial toxicity evaluation apparatus according to any one of claims 1 to 3, wherein the computing means calculates fluctuation of the transmission time or the transmission rate of the excitation conduction measured between the adjacent at least two electrodes, wherein the apparatus is used for an evaluation of myocardial toxicity of a drug using the fluctuation as a means of the evaluation.

5. The myocardial toxicity evaluation apparatus according to any one of claims 1 to 4, wherein the number for continuous measurements of the cellular potential of the cardiomyocytes over time is at least 30.

6. The myocardial toxicity evaluation apparatus according to any one of claims 1 to 5, wherein said apparatus further comprises a stimulation electrode for enforced pulsation of the cardiomyocytes, wherein the stimulation electrode is arranged within the cell population holding area.

7. The myocardial toxicity evaluation apparatus according to any one of claims 1 to 6, wherein the at least two measurement electrodes for comparative measurements are arranged linearly, and the cardiomyocyte network is arranged in coordination with the arrangement of the electrodes.

8. The myocardial toxicity evaluation apparatus according to any one of claims 1 to 7, wherein said apparatus comprises a cell holding member that holds the cardiomyocyte or the cardiomyocyte population within the cell population holding area, wherein the cell holding member forms a culturing chamber whose shape is in coordination with the arrangement of the at least two measurement electrodes for the comparison measurements.

9. The myocardial toxicity evaluation apparatus according to claim 8, wherein the at least two measurement electrodes and the stimulation electrode are arranged linearly and the stimulation electrode is arranged at end nodes, and wherein the cardiomyocyte network is arranged in coordination with the arrangement of the electrodes.

10. The myocardial toxicity evaluation apparatus according to claim 1 to 9, wherein the cell population further comprises non-cardiomyocytes.

11. The myocardial toxicity evaluation apparatus according to claim 10, wherein the cell population forms a cardiomyocyte network comprising fibroblasts at a proportion corresponding to that of a human heart.

12. The myocardial toxicity evaluation apparatus according to claim 11, wherein the fibroblasts make up 40 $\pm$ 10% to 60 $\pm$ 10% of the cells in the cell population.

13. The myocardial toxicity evaluation apparatus according to any one of claims 1 to 12, wherein said apparatus comprises a plurality of cell housing members sized to hold a single cell, and channels connecting the plurality of the cell housing members in a fluid communicable fashion, wherein the measurement electrodes are respectively arranged in each of the cell housing members.

14. The myocardial toxicity evaluation apparatus according to any one of claims 1 to 13, wherein the plurality of cardiomyocytes comprise at least four or eight cardiomyocytes.

15. The myocardial toxicity evaluation apparatus according to any one of claims 1 to 14, wherein:

   (i) the apparatus comprises two areas in which an agarose gel is arranged and an agarose gel is not arranged, respectively, within the cell population holding area on the substrate, wherein the cardiomyocytes or the cardiomyocyte population are arranged in the area in which the agarose gel is not arranged; or
   (ii) the apparatus comprises two areas in which a water-repellent solid is arranged and a water-repellent solid is not arranged, respectively, within the cell population holding area on the substrate, wherein the cardiomyocytes or the cardiomyocyte population are arranged in the area in which the water-repellent solid is not arranged.

16. The myocardial toxicity evaluation apparatus according to claim 15, wherein the water-repellent solid is a solid comprising a Teflon™ microparticle.

17. The myocardial toxicity evaluation apparatus according to claim 15, wherein (i) the area in which the agarose gel is not arranged or (ii) the area in which the water-repellent solid is not arranged is arranged linearly, in parallel, or in a lattice pattern.

18. The myocardial toxicity evaluation apparatus according to any one of claims 15 to 17, wherein a cell adhesive material such as collagen is applied on a surface of the substrate in (i) the area in which the agarose gel is not arranged or (ii) the area in which the water-repellent solid is not arranged.

19. The myocardial toxicity evaluation apparatus according to any one of claims 1 to 18, wherein the apparatus comprising:

   at least two potential measurement means configured to independently and continuously measure the excitation conduction which transmits through the cardiomyocyte network, a measurement data storage means configured to store measured results of the excitation conduction transmitting through the cardiomyocyte network, wherein the measured results are measured by the potential measurement means, at least two analysis means configured to analyze the measured results, an analysis data storage means configured to store analysis results analyzed by the analysis means and associate the analysis results with the measurement results, and a determining means configured to determine toxicity of the drug based on the analysis of the measurement results.

20. The myocardial toxicity evaluation apparatus according to claim 19, wherein the determining means is configured to associate the results from the determination with the measurement results stored in the measurement data storage means.

21. The myocardial toxicity evaluation apparatus according to claim 19 or 20, wherein the determining means is further configured to direct instructions to repeat the measurement to the measurement means via a feedback system based on the results of the determination.

22. A cardiomyocyte network chip for use in the cardiomyocyte evaluation apparatus according to claim 11 or 12, wherein the cardiomyocytes and the fibroblasts are arranged on the chip and incubated for a given period of time for the cells to adhere onto the chip, and wherein the chip is stored at or below a temperature of about 25 °C before use, and the chip is cultured at about 37 °C to restore a function of the cells before use.

23. The cardiomyocyte network chip according to claim 22, wherein the given period of time is at least about 12 hours.

24. A method for preparing a cardiomyocyte network chip for use in evaluation of cardiomyocyte toxicity, comprising:

(A) preparing a cardiomyocyte network chip comprising the following (i) to (v):

(i) a substrate;
(ii) a cell population comprising a plurality of stably pulsating subject cardiomyocytes or a cell population comprising the cardiomyocytes arranged on the substrate, and further comprising fibroblasts at a proportion corresponding to that of a heart;
(iii) a cell population holding area formed on the substrate and configured to hold the cardiomyocytes or the cell population and a cell culture liquid;
(iv) at least two measurement electrodes on each of which a single cell or a local portion of the cell population of the cardiomyocyte network comprising the plurality of the cardiomyocytes or the cell population comprising the cardiomyocytes is placed; and
(v) lead wires connected respectively to each of the measurement electrodes,

(B) incubating the cells to adhere onto the substrate for a given period of time, and
(C) storing, or storing and transporting the cardiomyocyte network chip at a temperature of about 25 °C or below.

25. The method according to claim 24, wherein the given period of time is at least about 12 hours.

26. The method according to claim 24 or 25, comprising re-culturing the cardiomyocyte network chip, which have been stored, or stored and transported, at a temperature of about 37 °C to restore the function of the cells before starting measurements of cellular potential of the cells.

27. A method for manufacturing a cell culture chip comprising a culturing cell placed on a substrate, the method comprising arranging water-repelling materials in an area on the surface of the substrate other than the area on which the cells are to be arranged.

28. The method according to claim 27, wherein the water-repelling material comprises a Teflon™ microparticle.

29. A cell culturing chip for arranging culturing cells on a substrate, wherein an area of a surface of the substrate other than an area in which the cells are to be arranged has a water-repelling surface.

30. The cell culturing chip according to claim 29, wherein the water-repelling surface is formed on the substrate such that the area on which the cells are arranged on the surface of the substrate has a linear, a parallel or a lattice shape on the substrate.

31. The cell culturing chip according to claim 29 or 30, wherein the water-repelling surface is formed on the substrate by coating the surface of the substrate with water-repelling material.

32. The cell culturing chip according to claim 31, wherein the water-repelling material comprises a material comprising Teflon™ microparticles.

33. The cell culturing chip according to any one of claims 29 to 32, wherein probe micro-particles are arranged on the surface of the substrate to optically detect the cells.

Fig. 1

Fig. 2

Fig. 3

Fig. 4

(a) Pulse of cell population

(b) Pulse of target cell (normal state)

(c) Pulse of target cell (on-drug state)

## Fig. 5

(a) Changes in volume of cell due to pulsation of cell population

(b) Changes in volume of cell due to pulsation of target cell (normal state)

(c) Changes in volume of cell due to pulsation of target cell (on-drug state)

Fig. 6

(a) Changes in extracellular potentials of target cell (normal state)

(b) Changes in extracellular potentials of target cell (on-drug state)

Fig. 7

Fig. 8

Fig. 9

(a)

Convert FPD data
into Poincare plots

(b)

## Fig. 10

(a)

**Number and arrangement of cells under control**

Normal model

Disease model

34

32
32

Stimulation from
pacemaker

33

Attenuation
of conduction

Extinction due
to refractory
period

**Generation of re-entry**

(b)

100 μm

Fig. 11

（a）

Cell population having a certain
width that allows selection of
conduction pathways

37

32

33

35

36

（b）

450 μm

Fibroblasts  Cardiomyocyte

50 μm

Delay time (ms)    3.0 ms (±0.3 ms)

Velocity (mm / s)    153 mm/s

Chamber
    Length: 450 μm
    Width: about 50 μm

Total: 50 cells
    Myocyte: 32 cells
    Fibroblast: 18 cells

（c）

Blue: Nuclear
Green: Mitochondria (Cardiomyocyte)

Myocyte

Fibroblast

50 μm

6 Myocytes & 2
Fibroblasts in
yellow box

100 μm

## Fig. 12

(a)

φ1-3 mm
ring shape

(b)

Normal
conduction

Abnormal
conduction

Sampling rate: ～ 1 kHz

500 ms

## Fig. 13

(a)

2

Φ10, 20, 50 μm

Nuclear stain image. Bar: 20 μm

(b)

20 μm

ISI = 1.1 sec, SD = 0.7 sec, CV = 66 %

(c)

Cell number: 8

Cell to be measured

Cells adjacent to the cell to be measured

ISI = 1.1 sec, SD = 0.08 sec, CV = 7.2 %

Fig. 14

Local electrical stimulation system (tungsten electrode)

Tip diameter: 5 μm

43

CCD neuro-chip

Transfer data every 1/10,000 seconds

Cell    44

45

Cell    44

CCD Element    CCD Element

5μm

43

Transfer data every 1/10,000 seconds
Real-time calculation by FPGA for image processing

Image processing technique can be employed for data processing!

Feedback to stimulation electrode

## Fig. 15

Fig. 16

Fig. 17

Fig. 18

Fig. 19

(a)

(b)

$$STV = \frac{\sum_{n=1}^{k} |(FPD_{n+1} - FPD_{mean}) - (FPD_n - FPD_{mean})|}{k \times \sqrt{2}}$$

$$= \frac{\sum_{n=1}^{k} |FPD_{n+1} - FPD_n|}{k \times \sqrt{2}} \quad \cdots (\text{Equation 1})$$

Fig. 20

(a)

(b)

$$LTV = \frac{\sum_{n=1}^{k} | (FPD_{n+1} - FPD_{mean}) + (FPD_{n} - FPD_{mean}) |}{k \times \sqrt{2}}$$

$$= \frac{\sum_{n=1}^{k} | FPD_{n+1} + FPD_{n} - 2FPD_{mean} |}{k \times \sqrt{2}} \quad \cdots (\text{Equation 2})$$

Fig. 21

(a)

(b)

Fig. 22

## Fig. 23

## Fig. 24

(a)

(b)

| Before Addition of an Agent | Low Volume Addition | Medium Volume Addition |
|---|---|---|

A

Fibrillation/Stopped

B

Fibrillation/Stopped

C

D

(c)

$$STV = \frac{\sum_{n=1}^{k} |V_{n+1} - V_{n}|}{k \times \sqrt{2}} \quad \cdots \text{(Equation 3)}$$

Fig. 26

Data of
Comparison
with ECG

Super-
posing
Circuit

Accumulation/
Analysis Unit for
Waveform Data

Differe
ntial
Circuit

Data of
Comparison
with
Cell
Potential

Mechanism
for Direct
Prediction of
an
Occurrence
of
Extrasystole

FP Waveform Data
from Each of
the Electrodes

Estimation
of State
of Ion
Channels
from the
Prediction
of Cell
Potential

Fig. 27

(A)

(B)

(C)

1 s

1 mV

L8
L7
L6
L5
L4
L3
L2
L1
R1
(PM)
R2
R3
R4
R5
R6
R7
R8

S

200 ms

82 msec (S.D. = 1.2 ms, spike number = 33, CV = 1.4 %)

Fig. 28

(A)

(B)

(C)

Fig. 29

$y = 489.43x^{-0.424}$
$R^2 = 0.5158$

Fig. 30

Fig. 31

(a)

Measurement Electrode

Stimulation Electrode

200 μm

(b)

Measurement Electrode

Stimulation Electrode

500 um

Fig. 32

10

2

Feedback System
for Electrical
Current Recording

Power
Source

Feedback
Mechanism

Stimulation
Mechanism

Fig. 33

Fig. 34

(a)

0.4Hz
(ctrl)

FPD

0.5Hz

1.3Hz

1000 μV

1 s

(b)

900
800
700
600
500
400
300
200
100
0

Length of FPD (ms)

0      0.5      1      1.5      2

Forced Pulsation Stimulation Interval (Hz)

FPDc (^1/3) Fredericia
FPDc (^1/2.5)
FPDc (^1/2) Bazett

Fig. 35

Table. 1  Heart rate correction of beating frequency, field potential duration

| | Beating Frequency | | | | FPD | | | FPDc | | |
|---|---|---|---|---|---|---|---|---|---|---|
| | Average (Hz) | S.D. (Hz) | C.V. (%) | Spike No. | Average (ms) | S.D. (ms) | C.V. (%) | Average (ms) | S.D. (ms) | C.V. (%) |
| 0.4 Hz (ctrl) | 0.37 | 0.01 | 1.5 | 110 | 795.3 | 13.9 | 1.7 | 536.7 | 9.7 | 1.8 |
| 0.5 Hz | 0.50 | 0.00 | 0.0 | 94 | 675.6 | 6.6 | 1.0 | 513.7 | 5.0 | 1.0 |
| 0.6 Hz | 0.60 | 0.00 | 0.0 | 142 | 638.5 | 7.5 | 1.2 | 521.8 | 6.1 | 1.2 |
| 0.8 Hz | 0.75 | 0.00 | 0.0 | 189 | 613.7 | 8.6 | 1.4 | 547.8 | 7.7 | 1.4 |
| 1.3 Hz | 1.28 | 0.00 | 0.0 | 253 | 489.4 | 11.5 | 2.4 | 540.2 | 12.7 | 2.4 |
| 1.8 Hz | 1.78 | 0.00 | 0.0 | 306 | 431.2 | 9.1 | 2.1 | 542.8 | 11.4 | 2.1 |

Fig. 36(a)

(a)

2c 10 2

Difference Circuit

Fig. 36(b)

# Fig. 36(c)

Single end and differential noise spectra were compared in a measurement manner as depicted in Figure 4.

Examination was performed using two electrodes on the probes, connecting one electrode to (+) of an input amplifier and the other electrode to (-) of the input amplifier, and using the reference electrodes as a ground in the same way as the single end.

As shown in the data, a significant improvement effect on noise is observed. As for the currently used probes, the problem of the differentials is that several (2-4) electrodes out of the 64 electrodes for detecting signals have to be made (-) with respect to the differential, and thus the number of CH for signal detection decreases.

Fig-7 Differential

Fig-6 Single-ended

Fig. 37

(a)

X Axis: (Conventional) Average Values of FPD

FP waveforms of cardiomyocytes
obtained from microelectrodes

E-4031
(n=27)

Y Axis: Fluctuation of FPD between adjacent
waveforms (Phasic Evaluation)

(b)

Fig. 38

Fig. 39

1st layer

2nd layer

3rd layer

4th layer

20 mm

Laminated by Heat Seal under Pressure

8-well plate with 3D injection/ exhort path made of integral acryl plate.

Plate size : □125 mm    Electrode size : φ10 μm

100 μm

8-well (8 electrodes / 1-well) ITO electrodes

8-well Multi-Electrode Culture Plate with Liquid Reflux Function

8-Well Multi-Electrode Culture Plate with Liquid Reflux Function Integrated in a Culture Measurement System

Fig. 40

Fig. 41

Cell Observation Area

4304

4304

4303

4302

4301

Fig. 42

(a)

4402    4402    4404

4401
4403    4403

(b)

4404

4401
4403    4402    4403

(c)

4402    4404

4401
4403    4402    4403    4402

(d)    4401

4402    4403

(e)    4402    4401

4402    4403
4402

## Fig. 43

**An Example of a 64-channel micro multi-electrode pattern**
**10μm ITO-Pt Block Electrode × 64 channel**

**Reference electrode: Pt**

**Bar = 0.1 mm**

Illumination Light Source

**Micro Multi-Electrode Potential Measurement System**

64-Channel Micro Multi-Electrode Cellular Network Chip

Chip Mounter

Stimulation Device

A/D Converter

Amplifier

Microscope Stage

Environment Controlling Chamber

Measurement of Pulsation by Image Analysis

Image Capturing Camera

Microscope

Data Synchronization

Measurements of Potentials

PC for Cellular Potential Analysis/ Stimulation Control/Deta Recording

PC for Image Analysis

**Electrophysiological Properties of Cellular Response**

**Cell Stimulation**

A) Autonomic Pulsation Conduction
B) Forced Stimulation Conduction
C) Feed-Back Stimulation Conduction

**Measurements of Contractile (Morphological Change) Properties of Cellular Response**

EP 2 937 413 A1

Fig. 44

Cellular Potential Waveform from All Micro-Electrodes

Identified Probe Microparticle

Identified Probe Microparticle

Captured Image of Cardiomyocyte Population

Probe-Particle Displacement-Observation Window

Target Cellular Potential Waveform

Displacement Amount of Each Probe Microparticle in the Direction of Y-Axis

Displacement Amount of Each Probe Microparticle in the Direction of X-Axis

Condition Setting Member

Fig. 45

Temporal Change of Extra-Cellular Potential

FP recordings
Amplitude
(uV)

(SR: 20kHz)

Optical imaging

Temporal Change of
Cardiomyocyte Displacement
Displacement: r
(um)

(SR: 10Hz)

Temporal Change of Velocity
of Cardiomyocyte Contraction
Velocity: dr/dt
(um/s)

1 s

Fig. 46

Continuous Images Showing Temporal Change of
A Probe Microparticle Attached to A Cell

Fluctuation Analysis of Movement Direction

Fig. 47

(a)

100
um

(b)

(c)

(d)

(e)

| Day 1 | Day 3 | Day 5 | Day 7 | Day 10 | Day 12 |

Fig. 48

Atrial type

Ventricular / Purkinje type

SN/AV node type

Fig. 49

(A)

(A)

(B)

Fig. 50

(A)

Stimulation Electrode Array

(B)

Stimulation Electrode Array

# Fig. 51

Fig. 52

# Fig. 53

Sample: mECMs
Drug: Verapmail

A

B

C

Fig. 54

Fig. 55

Configuration of Multielectrode-Measurement Apparatus System

Cellular-Network Chip
with 64 Channel
Microelectrodes

Illumination Light Source

Chip Mounter

Stimulation Device

A/D Converter

Amplifier

Automatic X-Y Stage

Measurement
of Pulsation
by Image
Analysis

Microscope Optical
System

Image Capturing
Camera

Incubator

Data Synchronization

Measurement
of Potential

PC for Image Analysis

PC for Control/Data Recording

Fig. 56

5601

5602

5603

Fig. 57

A

5701

B

5701

60°

0.30

5601

C

0.30

2.40

E

16:00:18

L1
Length : 189.0 um

A1
Angle : 91.1 deg

D

5702

5701

60°

0.30

Fig. 58

Ring-Shaped Electrode Array    Multielectrode Early-Screening System

1 mm

Block-Type Multielectrode Well

Stimulation Device

A/D Converter

Amplifier

Switching Means

Incubator

Electric Measurement Module

Measurement
of Potential

PC for Control/Data Recoding

Fig. 59

Fig. 60

Fig. 61

(a)

6101

6102

6103

(b)

6102  6102

6101

6103

6103

6102

6102

## Fig. 62

(a)

6201

22.18

30°

6202

1.08

0.158

1.40

(b)

6204

6202

6202

6202

6202

6202

6202

6203

Fig. 63

Fig. 64

Fig. 65

Fig. 66

Fig. 67

A

6701

6702

6703

6711

6712

6740

6731

6721

6722

6723

6724

B

MEASUREMENT SYSTEM
On-chip quasi-in vivo system

DATA
COMPRESSION

REMOTE STRAGE SYSTEM

DATA
COMPRESSION

ANALYSIS PERSONAL COMPUTER

Fig. 68

(a)

CELL WERE INCUBATED AT 37°C OVER NIGHT
BEFORE STARTING CULTURING AT RESPECTIVE TEMPERATURES

Mean±SD, n = 16 chambers

(b)

0.5 mm

## Fig. 69

STEP OF ARRANGING CELLS ON A CHIP
(37 °C)

⬇

STEP OF ADHERING THE CELLS ON THE CHIP
(FOR AT LEAST 12 HOURS AT 37 °C)

⬇

STEP OF STORING AND TRANSPORTING A
CELLULAR NETWORK CHIP
(25 °C)

⬇

STEP OF RESTORING AND ADJUSTING FUNCTIONAL
STATUS OF THE CELLS BEFORE MEASURING WITH
THE CELLULAR NETWORK CHIP (37 °C)

⬇

STEP OF MEASURING AFTER SETTING UP THE
CELLULAR NETWORK ON A MEASUREMENT SYSTEM
(37 °C)

Fig. 70

(a)

CELLS

WATER-
REPELLING
COATING

MICRO-
ELECTRODES

(b)

7002          7003                    7002

7001

Fig. 71

(a)

(c)

(b)

(d)

MAGNIFIED PHOTOS

(e)

(f)

(g)

## INTERNATIONAL SEARCH REPORT

| International application No. |
|---|
| PCT/JP2013/084084 |

**A. CLASSIFICATION OF SUBJECT MATTER**
*C12M1/34(2006.01)i*

According to International Patent Classification (IPC) or to both national classification and IPC

**B. FIELDS SEARCHED**

Minimum documentation searched (classification system followed by classification symbols)
C12M1/34

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)
JSTPlus/JMEDPlus(JDreamIII), WPIDS(STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | Kenji YASUDA et al., "Hito iPS Nado no Kansaibo kara Bunka shita Shinkin Saibo o Mochiita Fuseimyaku Yosokuho no Kaihatsu", Shindenzu, 31 August 2011 (31.08.2011), vol.31, no.3, pages 318 to 324 | 1-23,27-33<br>24-26 |
| Y<br>A | KANEKO Tomoyuki, et al., On-chip constructive cell-network study (I): contribution of cardiac fibroblasts to cardiomyocyte beating synchronization and community effect., Journal of nanobiotechnology, 2011, Vol.9, pages 21 (1-13) | 1-23,27-33<br>24-26 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 14 January, 2014 (14.01.14) | 28 January, 2014 (28.01.14) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2013/084084

C (Continuation).    DOCUMENTS CONSIDERED TO BE RELEVANT

| Category* | Citation of document, with indication, where appropriate, of the relevant passages | Relevant to claim No. |
|---|---|---|
| Y<br>A | JP 2010-130966 A  (Tokyo Medical and Dental University),<br>17 June 2010 (17.06.2010),<br>entire text; claims; drawings<br>& WO 2010/064700 A1     & JP 2010-130966 A<br>& EP 2371941 A1          & US 2011/0262958 A1 | 1-23,27-33<br>24-26 |
| Y<br>A | JP 63-049067 A  (Advance Co., Ltd.),<br>01 March 1988 (01.03.1988),<br>entire text; page 2, upper left column<br>& JP 07-004217 B2 | 1-23,27-33<br>24-26 |
| Y<br>A | JP 63-049066 A  (Advance Co., Ltd.),<br>01 March 1988 (01.03.1988),<br>entire text; page 2, upper left column<br>& JP 07-040914 B2 | 1-23,27-33<br>24-26 |
| Y<br>A | JP 63-039583 A  (Bio-Polymers, Inc.),<br>20 February 1988 (20.02.1988),<br>entire text; page 5, upper left column<br>& EP 243818 A          & AU 8771885 A<br>& NO 8701665 A         & DK 8702052 A<br>& FI 8701728 A         & JP 63-039583 A<br>& US 5108923 A         & EP 243818 B1<br>& DE 3783643 G         & FI 91037 B<br>& NO 174675 B          & CA 1328237 C<br>& JP 07-079695 B2      & DK 171996 B | 1-23,27-33<br>24-26 |
| A | NOMURA Fumimasa, et al., On-chip constructive cell-network study (II): on-chip quasi-in vivo cardiac toxicity assay for ventricular tachycardia/fibrillation measurement using ring-shaped closed circuit microelectrode with lined-up cardiomyocyte cell network., Journal of nanobiotechnology, 2011, Vol.9, pages 39 (1-5) | 1-33 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006094703 A

**Non-patent literature cited in the description**

- **BRENNAN M ; PALANISWAMI M ; KAMEN P.** Do existing measures of Poincare plot geometry reflect non-linear features of heart rate variability?. *Biomedical Engineering, IEEE Transactions on, Proc. IEEE Transactions on Biomedical Engineering,* 2001, vol. 48, 1342-1347
- **KANTERS JK ; HOLSTEIN-RATHLOU NH ; AGNER E.** Lack of evidence for low-dimensional chaos in heart rate variability. *Journal of Cardiovascular Electrophysiology,* 1994, vol. 5 (7), 591-601
- **STORELLA RJ ; WOOD HW ; MILLS KM et al.** Approximate entropy and point correlation dimension of heart rate variability in healthy subjects. *Integrative Physiological & Behavioral Science,* 1994, vol. 33 (4), 315-20
- **PATRICK DAVEY.** How to correct the QT interval for the effects of heart rate in clinical studies. *Journal of Pharmacological and Toxicological Methods,* 2002, vol. 48, 3-9